# EUROPEAN PATENT APPLICATION

(11) **EP 4 837 906 A1**
(43) Date of publication of application: **11.11.2026**
(21) Application number: 25742313.7
(22) Date of filing: 16.01.2025
(51) Int. Cl.: C07D 403/14, A61K 31/4184, A61P 35/00, A61P 29/00, A61P 37/00, C07D 401/14, C07D 417/14, C07D 403/04, C07D 413/14, C07D 405/14

(54) **NOVEL COMPOUND USED AS CBP/PA300 INHIBITOR, AND PHARMACEUTICAL COMPOSITION FOR PREVENTING OR TREATING CANCER, INFLAMMATORY DISORDERS, OR AUTOIMMUNE DISEASES COMPRISING SAME AS ACTIVE INGREDIENT**

(30) Priority: 17.01.2024 KR 20240007503
(71) Applicant: LG Chem, Ltd., Seoul 07336 (KR)
(72) Inventor: PAK, Gyungah, Daejeon 34122 (KR); KIM, Rira, Daejeon 34122 (KR); KWAK, Young Shin, Daejeon 34122 (KR); LEE, Soyun, Daejeon 34122 (KR); CHO, Joongheui, Daejeon 34122 (KR); HWANG, Youson, Daejeon 34122 (KR); KIM, Byung Gyu, Daejeon 34122 (KR); KIM, Hak Joong, Daejeon 34122 (KR)
(74) Representative: Hoffmann Eitle
(86) International application number: PCT/KR2025/099053
(87) International publication number: WO 2025/155165

(57) **Abstract**

The present invention provides: a novel compound used as a CBP/p300 inhibitor; a preparation method thereof; an isomer, a pharmaceutically acceptable salt, a hydrate, or a solvate thereof; and a pharmaceutical composition for preventing or treating cancer, inflammatory disorders, or autoimmune diseases, the pharmaceutical composition comprising at least one of the foregoing as an active ingredient.

## Description

### Technical Field

This application claims the benefit of priority to Korean Patent Application No. 2024-0007503, filed on January 17, 2024, the entire disclosure of which is incorporated herein by reference. This disclosure relates to a novel compound used as a CBP/p300 inhibitor, and a pharmaceutical composition for preventing or treating cancer, inflammatory disorders, or autoimmune diseases containing the same as an active ingredient.

### Background Art

CBP [CREB (cyclic-AMP response element binding protein) binding protein] and the paralog thereof, p300, are transcription coactivators composed of multiple functional domains including a histone acetyl-transferase (HATs) domain and a bromodomain (BRD). CBP and p300 are major epigenetic modulators that recognize and induce histone lysine acetylation at specific loci such as enhancers and promoters on chromatin, thereby regulating the expression of various genes.

In consideration of high structural similarity and functional redundancy, overexpression or mutations of CBP and p300, often referred to as CBP/p300, have been associated with various diseases, especially malignant tumors. For example, CBP and p300 bind to chromatin through BRD and HAT and acetylate histones at enhancer sites of oncogenes, thereby relaxing a chromatin structure and allowing transcriptional functional proteins including RNA polymerase II to assemble. As a result, tumor cells increase the expression of oncogenes such as MYC and IRF4 in tumor cells, thereby forming and growing tumors. Inhibition of the HAT domain causes an overall decrease in acetylation, which is important for transcription, and acts in the same way not only on tumor cells but also on normal cells, especially stem cells. Accordingly, it has been reported that it is difficult to secure a therapeutic window, and toxicity may be induced, so it is expected that the concerns may be overcome through a bromodomain inhibitor strategy.

Accordingly, various studies have been conducted to develop inhibitors that selectively inhibit the bromodomain of CBP/p300 and reduce the transcription of oncogenes activated in tumor cells. For example, CCS1477, a CBP/p300 bromodomain inhibitor developed by CellCentric, is in phase 1b/2a clinical trials for the treatment of hematological malignancies and advanced drug-resistant prostate cancer, and various CBP/p300 activity modulators are also disclosed in Patent Documents 1 to 3 below.

However, the development of effective and selective therapeutic agents targeting CBP/p300 is still a difficult task. Therefore, the development of more chemotypes of CBP/p300 inhibitors is needed.
[Patent Document 1] WO2018073587
[Patent Document 2] WO2019191667
[Patent Document 3] WO2017205538

### Disclosure of Invention

### Technical Goals

The present disclosure has a purpose of providing a novel compound used as a CBP/p300 inhibitor.

The present disclosure also has another purpose of providing an isomer of the compound, a pharmaceutically acceptable salt thereof, a hydrate thereof, or a solvate thereof.

The present disclosure also has another purpose of providing a pharmaceutical composition for preventing or treating cancer, inflammatory disorders, or autoimmune diseases, which includes at least one of the compound, the isomer thereof, the pharmaceutically acceptable salt thereof, the hydrate thereof, and the solvate thereof, as an active ingredient.

### Technical solutions

To solve the task, according to an aspect of the present disclosure, there is provided a compound represented by the following Formula I:

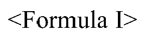

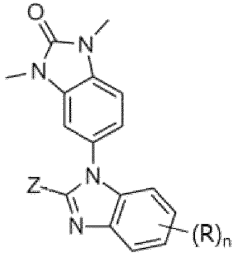

where
n is an integer from 0 to 3, where if n is an integer of 2 or more, Rs are the same or different, and Rs are each independently NR^{a}R^{b}, SO₂NHR^{c}, OR^{d}, CONR^{e}R^{f}, halogen, cyano, amidinyl, imine, C₁₋₈ alkyl, C₃₋₁₀ cyclolalkyl, C₆₋₁₀ aryl, 5- to 13-membered heteroaryl, 3- to 14-membered heterocycloalkyl, or 4- to 9-membered heterocycloalkenyl, and R^{a} to R^{f} are each independently selected from the group consisting of hydrogen, C₁₋₄ alkyl, C₃₋₁₀ cycloalkyl and C₆₋₁₀ aryl, and
Z is C₁₋₈ alkyl, C₃₋₁₀ cycloalkyl, C₁₋₅ haloalkyl, C₆₋₁₀ aryl, 5- to 13-membered heteroaryl or 3- to 14-membered heterocycloalkyl,
where R, R^{a} to R^{f} and Z are each substituted or unsubstituted with Substituent Group I.

Another aspect of the present disclosure relates to an isomer of the compound above, a pharmaceutically acceptable salt thereof, a hydrate thereof or a solvate thereof.

Another aspect of the present disclosure relates to a pharmaceutical composition for preventing or treating cancer, inflammatory disorders or autoimmune diseases, the composition including at least one among the compound above, an isomer thereof, a pharmaceutically acceptable salt thereof, a hydrate thereof and a solvate thereof, as an active ingredient.

### Effects

According to the present disclosure, it has been found that the compound represented by Formula I has excellent CBP/p300 inhibitory activity. Therefore, the compound represented by Formula I of the present disclosure; an isomer of the compound, a pharmaceutically acceptable salt thereof, a hydrate thereof or a solvate thereof; and a pharmaceutical composition including at least one of them as an active ingredient may be useful for inhibition, such as the prevention or treatment of cancer, inflammatory disorders or autoimmune diseases.

The effects of the present disclosure are not limited to the above-described effects, and are understood to include all effects that may be inferred from the configuration of the invention described in the description or claims of the present disclosure.

### Best Mode for Carrying Out the Invention

Hereinafter, in order to assist the understanding of the present disclosure, the present disclosure will be described in more detail. In this case, the terms or words used in this specification and claims should not be interpreted as limited to usual or dictionary meanings, and should be interpreted as meanings and concepts that conform to the technical idea of the present disclosure based on the principle that the inventor may appropriately define the concept of the term to explain his own invention in the best way.

The present disclosure provides a compound represented by the following Formula I:

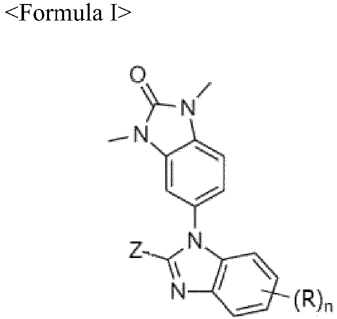

where
n may be an integer from 0 to 3, where if n is an integer of 2 or more, Rs may be the same or different, and Rs may be each independently NR^{a}R^{b}, SO₂NHR^{c}, OR^{d}, CONR^{e}R^{f}, halogen, cyano, amidinyl, imine, C₁₋₈ alkyl, C₃₋₁₀ cycloalkyl, C₆₋₁₀ aryl, 5- to 13-membered heteroaryl, 3- to 14-membered heterocycloalkyl, or 4- to 9-membered heterocycloalkenyl, and R^{a} to R^{f} may be each independently selected from the group consisting of hydrogen, C₁₋₄ alkyl, C₃₋₁₀ cycloalkyl and C₆₋₁₀ aryl, and Z may be C₁₋₈ alkyl, C₃₋₁₀ cycloalkyl, C₁₋₅ haloalkyl, C₆₋₁₀ aryl, 5- to 13-membered heteroaryl or 3- to 14-membered heterocycloalkyl, where R, R^{a} to R^{f} and Z may be each substituted or unsubstituted with Substituent Group I:
Substituent Group I: substituted or unsubstituted C₁₋₁₀ alkyl, substituted or unsubstituted C₂₋₁₀ alkenyl, substituted or unsubstituted C₃₋₂₀ carbocycle, substituted or unsubstituted C₃₋₂₀ cycloalkyl, substituted or unsubstituted C₆₋₃₀ aryl, substituted or unsubstituted 3- to 30-membered heterocycle, substituted or unsubstituted C₁₋₁₀ alkoxy, substituted or unsubstituted C₂₋₁₀ alkenyloxy, substituted or unsubstituted C₃₋₁₀ cycloalkyloxy, substituted or unsubstituted C₆₋₃₀ aryloxy, substituted or unsubstituted 3- to 30-membered heterocycleoxy, substituted or unsubstituted C₁₋₁₀ alkylketone, substituted or unsubstituted C₂₋₁₀ alkenylketone, substituted or unsubstituted C₃₋₁₀ cycloalkylketone, substituted or unsubstituted C₆₋₃₀ arylketone, substituted or unsubstituted 3- to 30-membered heterocycleketone, substituted or unsubstituted C₁₋₁₀ alkylthio, substituted or unsubstituted C₂₋₁₀ alkenylthio, substituted or unsubstituted C₃₋₁₀ cycloalkylthio, substituted or unsubstituted C₆₋₃₀ arylthio, substituted or unsubstituted 3- to 30-membered heterocyclethio, aldehyde, carboxy, halogen, substituted or unsubstituted C₁₋₁₀ haloalkyl, hydroxy, substituted or unsubstituted amino, imine, cyano, nitro, amide, thiol, sulfone and phosphoric acid,
where a C₁₋₁₀ alkyl moiety may be interposed by a heteroatom group selected from -O-, -S-, -SO-, -SO₂-, -NR^{g}- (R^{g} is hydrogen or substituted or unsubstituted C₁₋₁₀ alkyl), -N=, =N-, -POR^{g}- and -PO₄R^{g}-, and a heterocycle moiety may include at least one of S, N, P and O as a ring-forming atom.

In this specification, matters on R may be equally applied to R₁, 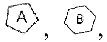 and

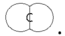

In defining the compound represented by Formula I throughout this specification, the defined concepts as follows are used. The definitions apply to terms used individually or as part of a larger group throughout this specification, unless otherwise specified. Terms and abbreviations used herein have their original meanings unless otherwise defined.

In this specification, the term "substituted" may mean that a hydrogen atom bonded to the compound or each substituent listed in the substituent group is replaced and bonded with an arbitrary substituent. In this case, the position to be substituted is not limited as long as the position is where a hydrogen atom bonded to the compound or each substituent listed in the substituent group exists, i.e., the position where the hydrogen atom may be substituted with a substituent. If two or more are substituted, the two or more substituents may be the same or different from each other. In contrast, "unsubstituted" may mean that no hydrogen atom bonded to a carbon atom is replaced with an arbitrary substituent.

Arbitrary substituent applicable to the "substitution" may be appropriately selected from the substituents listed in the substituent group as described in this specification, but the scope of the substituents available for the substitution is not limited to the substituent group.

If a substituent is not separately described or mentioned in the chemical formulas described in this specification, it may be considered that hydrogen is bonded, and in some cases, the substituent may be absent depending on the ring-forming elements of a heterocycle.

In this specification, the term "halogen" includes, but is not limited to, for example, fluoro, chloro, bromo, and iodo.

In this specification, the term "amino" is intended to encompass monovalent primary amine, monovalent secondary amine, and monovalent tertiary amine. That is, amino refers to both a monovalent group in which two hydrogen atoms are bonded to a nitrogen atom and a monovalent group in which at least one hydrogen atom is substituted with another substituent. In the above, the group in which two hydrogen atoms are bonded to a nitrogen atom may be an unsubstituted amino, and the group in which at least one hydrogen atom is substituted with another substituent may be a substituted amino. Meanwhile, for dialkylamino, alkyl moieties may be the same or different.

In this specification, the term "imine or imino" is intended to encompass monovalent primary ketimines (-C(=NH)Rⁱ⁻¹), secondary ketimines (-C(N=Rⁱ⁻²)Rⁱ⁻¹), primary aldimines (-C(=NH)H), and secondary aldimines (-C(=NRⁱ⁻¹)H), where Rⁱ⁻¹ and Rⁱ⁻² may be, but are not limited to, each independently, C₁₋₁₀ alkyl, C₂₋₁₀ alkenyl, C₃₋₁₀ carbocycle, C₃₋₁₀ cycloalkyl, C₆₋₃₀ aryl, 3- to 30-membered heterocycle, or C₁₋₁₀ haloalkyl, and each of which may be substituted or unsubstituted, but is not limited thereto.

In this specification, the term "alkyl" by itself or as a part of another substituent means a straight or branched monovalent hydrocarbon having an indicated number of carbon atoms (i.e., C₁₋₁₀ means 1 to 10 carbon atoms), unless otherwise referred to.

Examples of the alkyl include methyl, ethyl, n-propyl, isopropyl, n-butyl, isobutyl, tert-butyl, sec-butyl, 1-methyl-butyl, 1-ethyl-butyl, n-pentyl, isopentyl, neopentyl, tert-pentyl, n-hexyl, 1-methylpentyl, 2-methylpentyl, 4-methyl-2-pentyl, 3,3-dimethylbutyl, 2-ethylbutyl, n-heptyl, 1-methylhexyl, n-octyl, tert-octyl, 1-methylheptyl, 2-ethylhexyl, 2-propylpentyl, n-nonyl, 2,2-dimethylheptyl, 1-ethylpropyl, 1,1-dimethyl-propyl, isohexyl, 2-methylpentyl, 4-methylhexyl and 5-methylhexyl, but these are non-limiting examples and the scope of the present disclosure is not limited thereto.

In this specification, the term "cycloalkyl" means a monovalent aliphatic carbocycle in which one hydrogen atom is missing from an arbitrary position among the carbon atoms constituting the ring. Examples of cycloalkyl include, but are not limited to, cyclopropyl, cyclobutyl, cyclopentyl, 3-methylcyclopentyl, 2,3-dimethylcyclopentyl, cyclohexylene 3-methylcyclohexyl, 4-methylcyclohexyl, 2,3-dimethylcyclohexyl, 3,4,5-trimethylcyclohexyl, 4-tert-butylcyclohexyl, cycloheptyl, and cyclooctyl.

In this specification, the term "aryl" refers to a multiply unsaturated, typically aromatic hydrocarbon ring, where the aryl may be monocycle or polycycle. The case of polycycle includes a form in which only aromatic hydrocarbon rings are fused and a form in which an aliphatic hydrocarbon ring is fused with an aromatic hydrocarbon. Examples of the aryl include monocyclic aryls such as phenyl, biphenyl, terphenyl and quaterphenyl, and polycyclic aryls such as naphthyl, anthracenyl, phenanthrenyl, pyrenyl and benzopyrenyl.

In this specification, the term "heterocycle" refers to a ring including, together with carbon atoms, 1 to 5 heteroatoms other than carbon atoms, i.e., O, N, P and S, as ring-forming atoms. Here, the heterocycle may be aromatic, aliphatic, monocyclic or polycyclic and include, but is not limited to, thiophenyl, furanyl, pyrrolyl, imidazolyl, thiazolyl, oxazolyl, oxadiazolyl, pyridinyl, bipyridinyl, pyrimidinyl, triazinyl, triazolyl, pyridazinyl, pyrazinyl, pyrido pyrimidyl, pyrido pyrazinyl, pyrazino pyrazinyl, isoxazolyl and thiadiazolyl, but the scope of the present disclosure is not limited thereto.

In this specification, the term "heterocycloalkyl" means partially or fully saturated hydrocarbon containing one or more heteroatoms selected from O, N, P and S as a ring-forming atom and forming a monocycle or a fused ring. Preferably, the heteroatom may be 1, 1 to 2, or 1 to 3. For example, piperazinyl, piperazinedionyl, piperidinyl, pyrrolidinyl, morpholinyl, imidazolidinyl, imidazolidinonyl, lactamyl, tetrahydropyranyl, tetrahydrofuranyl, diazabicyclooctanyl and diazaspiroctanyl may be included, without limitation.

In this specification, the term "heterocycloalkenyl" means unsaturated hydrocarbon including one or more heteroatoms selected from O, N, P and S as ring-forming atoms and forming a monocycle or a fused ring. Preferably, the heteroatom may be 1, 1 to 2, or 1 to 3. For example, tetrahydropyridinyl may be included, without limitation.

In this specification, the term "heteroaryl" means an aromatic heterocycle and may be a monocycle or polycycle. The polycycle includes not only a form in which multiple aromatic rings are fused, but also a form in which an aromatic ring and an aliphatic ring are fused. Examples of the heteroaryl include pyrrolyl, pyrazolyl, imidazolyl, triazolyl, oxazolyl, isoxazolyl, thiazolyl, tetrazolyl, isothiazolyl, furyl, thienyl, pyridinyl, pyridyl N-oxide, pyrazinyl, pyrimidinyl, pyridazinyl, indolyl, benzothiazolyl, benzoxazolyl, benzothienyl, quinolinyl, quinazolinyl, quinoxalinyl, phthalazinyl, isoquinolinyl, thiophenyl, acridyl, indazolyl, carbazolyl, benzimidazolyl, benzocarbazolyl, benzofuranyl, benzothiophenyl, dibenzothiophenyl, pyrazolopyrimidinyl, or azaindolyl, but the scope of the present disclosure is not limited thereto.

In this specification, the term "alkenyl" may be a straight or branched chain. Alkenyl includes, for example, ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl or 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl, 1-ethyl-2-methyl-2-propenyl, 1-heptenyl, 2-heptenyl, 3-heptenyl, 1-octenyl, 2-octenyl, 3-octenyl, 4-octenyl, 1-nonenyl, 2-nonenyl, 3-nonenyl, 4-nonenyl, 1-decenyl, 2-decenyl, 3-decenyl, 4-decenyl, and 5-decenyl, but is not limited thereto.

In this specification, the terms "carbocycle" and "carbocyclic" mean a ring in which ring-forming atoms are composed of carbon. The carbocycle may be aliphatic or aromatic, saturated or unsaturated, and monocyclic or polycyclic. The polycyclic ring may be a fused, bridged, or spiro polycyclic ring. A monocyclic carbocycle may have 3 to 17 carbon atoms, specifically 3 to 14 carbon atoms, more specifically 3 to 10 carbon atoms, and particularly specifically 3 to 7 carbon atoms.

In this specification, the term "alkoxy" refers to a substituent having oxygen bonded to alkyl and may be a straight, branched, or cyclic chain. Examples of alkoxy include, but are not limited to, methoxy, ethoxy, n-propoxy, isopropoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, and monohalogenated and polyhalogenated variants thereof.

In this specification, the term "oxy" refers to a substituent which may be represented by "-OR^{oxy}", where R^{oxy} may be, but is not limited to, substituted or unsubstituted C₁₋₁₀ alkyl, substituted or unsubstituted C₂₋₁₀ alkenyl, substituted or unsubstituted C₃₋₁₀ carbocycle, substituted or unsubstituted C₃₋₁₀ cycloalkyl, substituted or unsubstituted C₆₋₃₀ aryl, substituted or unsubstituted 3- to 30-membered heterocycle, or substituted or unsubstituted C₁₋₁₀ haloalkyl.

In this specification, the term "carbonyl" is a substituent which may be represented by "-C(=O)R^{car}", where R^{car} may be, but is not limited to, substituted or unsubstituted C₁₋₁₀ alkyl, substituted or unsubstituted C₂₋₁₀ alkenyl, substituted or unsubstituted C₃₋₁₀ carbocycle, substituted or unsubstituted C₃₋₁₀ cycloalkyl, substituted or unsubstituted C₆₋₃₀ aryl, substituted or unsubstituted 3- to 30-membered heterocycle, or substituted or unsubstituted C₁₋₁₀ haloalkyl, each of which is defined as described above. "Carbonyl" may be referred to as a "ketone," and may be referred to as "alkylketone" depending on the substituent thereof and bonding site.

In this specification, the term "thio" is a substituent which may be represented by "-SR^{thio}", where R^{thio} may be, but is not limited to, substituted or unsubstituted C₁₋₁₀ alkyl, substituted or unsubstituted C₂₋₁₀ alkenyl, substituted or unsubstituted C₃₋₁₀ carbocycle, substituted or unsubstituted C₃₋₁₀ cycloalkyl, substituted or unsubstituted C₆₋₃₀ aryl, substituted or unsubstituted 3- to 30-membered heterocycle, or substituted or unsubstituted C₁₋₁₀ haloalkyl.

In this specification, the term "haloalkyl" is synonymous with "halogenated alkyl" and "halogen-substituted alkyl", and may mean alkyl in which an arbitrary hydrogen atom in the alkyl is substituted with an arbitrary halogen atom. For example, -CF₃, -CF₂H, -CH₂Cl, -CH₂CF₃, and -C(Cl₂)CF₃ are haloalkyl, but the scope of the present disclosure is not limited thereto.

In this specification, the term "amide" is a substituent which may be represented, for example, by R-C(=O)-NR'R", where R, R' and R" are each hydrogen, substituted or unsubstituted C₁₋₁₀ alkyl, substituted or unsubstituted C₃₋₁₀ carbocycle, substituted or unsubstituted C₃₋₁₀ cycloalkyl, substituted or unsubstituted C₆₋₃₀ aryl, substituted or unsubstituted 3- to 30-membered heterocycle or substituted or unsubstituted C₁₋₁₀ haloalkyl, but are not limited thereto.

In this specification, the term "sulfonyl" refers to a substituent which may be represented by "-S(=O)₂R^{s1}", where R^{s1} may be, but is not limited to, substituted or unsubstituted C₁₋₁₀ alkyl, substituted or unsubstituted C₂₋₁₀ alkenyl, substituted or unsubstituted C₃₋₁₀ carbocycle, substituted or unsubstituted C₃₋₁₀ cycloalkyl, substituted or unsubstituted C₆₋₃₀ aryl, substituted or unsubstituted 3- to 30-membered heterocycle, or substituted or unsubstituted C₁₋₁₀ haloalkyl.

In this specification, the term "thioamide" refers to a substituent which may be represented by "-C(=S)NR^{ta}₂", "-C(=S)NHR^{ta}" and "-C(=S)NH₂", where R^{ta}s are independent from each other and each may be, but are not limited to, substituted or unsubstituted C₁₋₁₀ alkyl, substituted or unsubstituted C₂₋₁₀ alkenyl, substituted or unsubstituted C₃₋₁₀ carbocycle, substituted or unsubstituted C₃₋₁₀ cycloalkyl, substituted or unsubstituted C₆₋₃₀ aryl, substituted or unsubstituted 3- to 30-membered heterocycle, or substituted or unsubstituted C₁₋₁₀ haloalkyl.

In this specification, the term "sulfinyl" refers to a substituent which may be represented by "-S(=O)R^{s2}", where R^{s2} may be, but is not limited to, substituted or unsubstituted C₁₋₁₀ alkyl, substituted or unsubstituted C₂₋₁₀ alkenyl, substituted or unsubstituted C₃₋₁₀ carbocycle, substituted or unsubstituted C₃₋₁₀ cycloalkyl, substituted or unsubstituted C₆₋₃₀ aryl, substituted or unsubstituted 3- to 30-membered heterocycle, or substituted or unsubstituted C₁₋₁₀ haloalkyl, and each of which is defined as described above. Sulfinyl may also be used interchangeably with "thiocarbonyl", and in a narrower sense may also be referred to as alkylthiocarbonyl in a case where R^{s2} is alkyl.

In this specification, the term "phosphoric acid" refers to a substituent which may be represented by "-OP(=O)(OH)₂" or "-OP(=O)(OH)(OR^{P})", where R^{P} may be, but is not limited to, substituted or unsubstituted C₁₋₁₀ alkyl, substituted or unsubstituted C₂₋₁₀ alkenyl, substituted or unsubstituted C₃₋₁₀ carbocycle, substituted or unsubstituted C₃₋₁₀ cycloalkyl, substituted or unsubstituted C₆₋₃₀ aryl, substituted or unsubstituted 3- to 30-membered heterocycle, or substituted or unsubstituted C₁₋₁₀ haloalkyl.

In this specification, the term "ester" is a substituent which may be represented by "-OC(=O)R^{ester}", where R^{ester} may be, but is not limited to, substituted or unsubstituted C₁₋₁₀ alkyl, substituted or unsubstituted C₃₋₁₀ carbocycle, substituted or unsubstituted C₃₋₁₀ cycloalkyl, substituted or unsubstituted C₆₋₃₀ aryl, substituted or unsubstituted 3- to 30-membered heterocycle, or substituted or unsubstituted C₁₋₁₀ haloalkyl.

In an embodiment, R, R^{a} to R^{f}, A and Z each may be substituted or unsubstituted with Substituent Group I-1.

Substituent Group I-1: substituted or unsubstituted C₁₋₈ alkyl, substituted or unsubstituted C₃₋₈ cycloalkyl, hydroxy, substituted or unsubstituted C₁₋₃ alkoxy, substituted or unsubstituted C₁₋₄ haloalkyl, substituted or unsubstituted C₆₋₈ aryl, substituted or unsubstituted 3- to 8-membered heteroaryl, substituted or unsubstituted cyano, substituted or unsubstituted amino, substituted or unsubstituted amide, aldehyde, halogen, CO₂R^{p1}, NHCOR^{p2}, NHCO₂R^{p3} or NHSO₂R^{p4}, where R^{p1} to R^{p4} are each independently hydrogen, substituted or unsubstituted methyl, substituted or unsubstituted ethyl, substituted or unsubstituted butyl or substituted or unsubstituted phenyl.

In an embodiment, the compound represented by Formula I may be represented by the following Formula I-1:

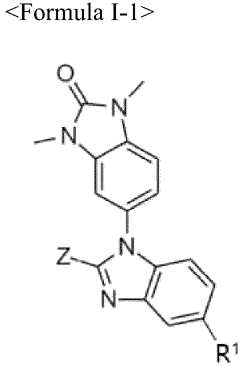

where
R¹ may be NR^{a}R^{b}, SO₂NHR^{c}, OR^{d}, CONR^{e}R^{f}, halogen, cyano or imine, and may be substituted or unsubstituted with Substituent Group I, and R^{a} to R^{f} and Z may be the same as defined above.

In this specification, unless otherwise specifically stated, matters relating to R may be equally applied to R¹.

In an embodiment, R¹ may be NR^{a}R^{b}, SO₂NHR^{c}, OR^{d}, CONR^{e}R^{f}, bromo, cyano or amidinyl, where R^{a} to R^{f} may be each independently hydrogen, substituted or unsubstituted methyl, substituted or unsubstituted ethyl, substituted or unsubstituted propyl, substituted or unsubstituted cyclopropyl, substituted or unsubstituted cyclobutyl or substituted or unsubstituted phenyl, and R¹ may be substituted or unsubstituted with Substituent Group I-1-1:

Substituent Group I-1-1: substituted or unsubstituted C₁₋₃ alkyl, substituted or unsubstituted C₃₋₅ cycloalkyl, and hydroxy.

In an embodiment, the compound of Formula I may be represented by the following Formula I-2:

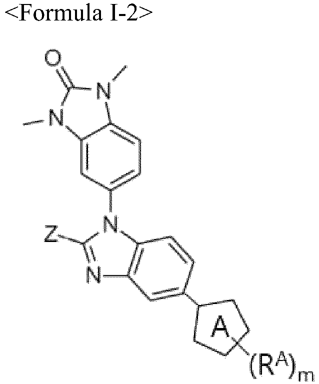

where 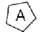 may be C₅ cycloalkyl, 5-membered heteroaryl or 5-membered heterocycloalkyl, m may be an integer from 0 to 3, where if m is an integer of 2 or more, R^{A}s may be the same or different, and R^{A} may be substituted or unsubstituted C₁₋₆ alkyl, substituted or unsubstituted C₁₋₄ haloalkyl, substituted or unsubstituted cyano, substituted or unsubstituted amide, substituted or unsubstituted amino, or aldehyde.

In this specification, unless otherwise specifically stated, matters relating to R may be equally applied to

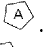

In an embodiment, 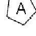 may be furanyl, isoxazolyl, oxadiazolonyl, pyrazolyl, thiophenyl, triazolyl, thiazolyl or thiazolinyl.

In an embodiment, Z may be the same as described above.

In an embodiment, the compound of Formula I may be represented by the following Formula I-3:

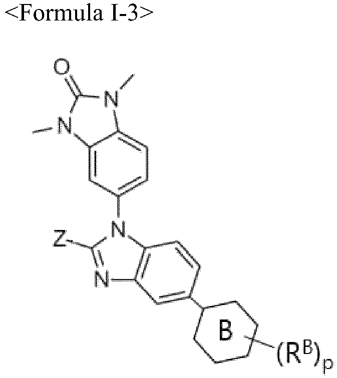

where 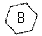 may be C₅ cycloalkyl, C₆ aryl, 6-membered heteroaryl, 6-membered heterocycloalkyl or 6-membered heterocycloalkenyl, p may be an integer from 0 to 3, where if p is an integer of 2 or more, R^{B}s may be the same or different, and R^{B} may be substituted or unsubstituted C₁₋₅ alkyl, substituted or unsubstituted C₁₋₃ alkoxy, substituted or unsubstituted amide, substituted or unsubstituted amino, halogen, cyano, hydroxy, aldehyde, CO₂R^{h}, NHCORⁱ, NHCO₂R^{j} or NHSO₂R^{k}, where R^{h} to R^{k} may be each independently hydrogen, substituted or unsubstituted methyl, substituted or unsubstituted butyl or substituted or unsubstituted phenyl.

In this specification, unless otherwise specifically stated, matters relating to R may be equally applied to

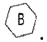

In an embodiment, 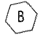 may be phenyl, pyridonyl, pyridinyl, pyrimidinyl, piperidinyl or tetrahydropyridinyl.

In an embodiment, Z may be the same as described above.

In an embodiment, the compound of Formula I may be represented by the following Formula I-4:

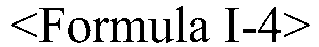

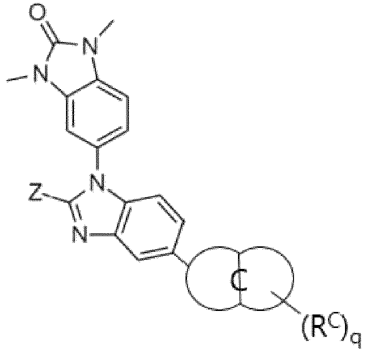

where
q may be an integer from 0 to 3, where if q is an integer of 2 or more, R^{C}s may be the same or different, 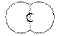 may be C₉ cycloalkyl, C₁₀ cycloalkyl, C₉ aryl, C₁₀ aryl, 9-membered heteroaryl, 10-membered heteroaryl, 9-membered heterocycloalkyl or 10-membered heterocycloalkyl, and R^{C} may be substituted or unsubstituted C₁₋₈ alkyl, substituted or unsubstituted C₃₋₇ cycloalkyl, halogen or cyano.

In this specification, unless otherwise specifically stated, matters relating to R may be equally applied to

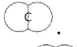

In an embodiment, 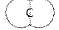 may be benzimidazolyl, benzodioxolyl, benzodioxanyl or dihydrobenzofuranyl.

In an embodiment, Z may be the same as described above.

In an embodiment, q may be 0.

In an embodiment, Z may be methyl, ethyl, propyl, butyl, cyclopropyl, cyclohexyl, trifluoromethyl, phenyl, furanyl, imidazolyl, isoxazolyl, oxazolyl, pyrazolyl, pyrrolyl, thiazolyl, thiophenyl, pyridinyl, pyrimidinyl, azaindolyl, indolyl, piperidinyl, tetrahydropyranyl, lactamyl or tetralinyl, and Z may be substituted or unsubstituted with Substituent Group I-1-2:
Substituent Group I-1-2: substituted or unsubstituted C₁₋₃ alkyl, substituted or unsubstituted C₃₋₈ cycloalkyl, substituted or unsubstituted C₁₋₃ haloalkyl, halogen, hydroxy, substituted or unsubstituted C₁₋₃ alkoxy, substituted or unsubstituted C₆₋₈ aryl, substituted or unsubstituted 3- to 8-membered heteroaryl, substituted or unsubstituted cyano, substituted or unsubstituted amino, substituted or unsubstituted amide, CO₂R^{l}, or NHCO₂R^{m}, where R^{l} and R^{m} are each independently hydrogen, substituted or unsubstituted methyl, substituted or unsubstituted ethyl, substituted or unsubstituted butyl, or substituted or unsubstituted phenyl.

In an embodiment, the compound represented by Formula I may be any one selected from Table 1 below.

In an embodiment, the compound represented by Formula I may be used as a CBP/p300 inhibitor. In this specification, CBP/p300 may mean CBP and/or p300. The CBP refers to CREB (cyclic-AMP response element binding protein) binding protein, and the p300 refers to a paralog of CBP.

The CBP/p300 induces histone H3 lysine 27 acetylation (H3K27ac) at target gene promoters, enhancers and super enhancers, and at the same time, recognizes acetylated histones via BRD to activate gene transcription. According to the overexpression of the CBP/p300 in cancer cells and drug-resistant cancer cells, oncogene transcription is activated and cancer cell proliferation, survival, tumor formation, metastasis, immune evasion or drug resistance is induced. Therefore, it is necessary to inhibit the overexpression of CBP/p300 in cancer cells, or the like. The use of the compound represented by Formula I as a CBP/p300 inhibitor in this specification may mean that the compound has the inhibition activity of the overexpression of CBP/p300 as described above. The compound represented by Formula I of the present disclosure may, for example, reduce the H3K27ac and down-regulate oncogene transcription, thereby inducing cancer cell growth inhibition and apoptosis, activating immune responses, overcoming drug resistance, and inhibiting tumor progression in vivo. The compound represented by Formula I of the present disclosure may also, for example, enhance the anticancer efficacy of chemotherapy, radiotherapy, and epigenetic anticancer agents by inhibiting BRD.

The present disclosure also provides an isomer of the compound represented by Formula I, a pharmaceutically acceptable salt thereof, a hydrate thereof, or a solvate thereof.

In this specification, the term "isomer" may include an enantiomer, a diastereomer thereof, a tautomer thereof, and a geometric isomer thereof. In this specification, enantiomers refer to two stereoisomers of a compound that are non-superimposable mirror images of each other.

In this specification, diastereomers refer to stereoisomers having two or more chiral centers and whose molecules are not mirror images of each other. Diastereomers have different physical properties such as melting points, boiling points, spectral characteristics, and reactivities. Mixtures of diastereomers may be separated under high-resolution analytical procedures such as electrophoresis and chromatography.

In this specification, geometric isomers refer to cis forms, in which the same type of atoms or groups of atoms are on the same side of a double bond, and trans forms, in which the same type of atoms or groups of atoms are on opposite sides of a double bond.

In this specification, tautomers or tautomeric forms refer to structural isomers with different energies that are compatible through a low energy barrier. For example, proton tautomers (also known as protonic tautomers) include interconversions via the transfer of a proton, including keto-enol and imine-enamine isomerization. Atomic tautomers include interconversions by retransfer of some bonding electrons.

In this specification, an arbitrary atom present in the compound of the present disclosure may be present in any of naturally occurring isotopic forms. For example, isotopes of hydrogen, carbon, nitrogen, oxygen, phosphorus, sulfur, fluorine, chlorine and iodine may be ²H ("D"), ³H, ¹¹C, ¹²C, ¹³C, ¹⁴C, ¹³N, ¹⁵N, ¹⁵O, ¹⁷O, ¹⁸O, ³²P, ³³P, ³⁵S, ¹⁸F, ³⁶Cl, ¹²³I and ¹²⁵I.

In this specification, the term "acceptable salt" means a salt of an active compound prepared with an acid or a base, which depends on the particular substituent based on the compound described in this specification. If the compound according to the present disclosure contains a relatively acidic functional group, a base addition salt may be obtained by contacting such a compound in a neutral state with a sufficient amount of a desired base in the absence of a solvent or in the presence of a suitable inert solvent.

Acceptable salts include, for example, alkali metal salts, for example, sodium or potassium salt; alkaline earth metal salts, for example, calcium or magnesium salt; ammonium salt; aliphatic amine salts, for example, trimethylamine, triethylamine, dicyclohexylamine, ethanolamine, diethanolamine, triethanolamine or procaine salt; aralkyl amine salts, for example, N,N-dibenzylethylenediamine salt; heterocyclic aromatic amine salts, for example, pyridine salt, picolino salt, quinoline salt or isoquinoline salt; quaternary ammonium salts, for example, tetramethylammonium salt, tetraethylammonium salt, benzyltrimethylammonium salt, benzyltriethylammonium salt, benzyltributylammonium salt, methyltrioctylammonium salt or tetrabutylammonium salt; and basic amino acid salts, for example, arginine salt or lysine salt. The acid salts may be, for example, inorganic acid salts, for example, hydrochloride, sulfate salt, nitrate salt, phosphate salt, carbonate salt, hydrogencarbonate or perchlorate; organic acid salts, for example, acetate, propionate, lactate, malate, fumarate, tartrate, malate, fumarate, citrate, ascorbate, or formic acid; sulfonates, for example, methanesulfonate, isothionate, benzenesulfonate or p-toluenesulfonate; and acidic amino acid salts, for example, aspartate or glutamate.

The term "hydrate" in this specification refers to a compound in which water (H₂O) is bonded to another compound.

In this specification, the term "solvate" refers to an aggregate or composite of one or more solvent molecules with the compound of the present disclosure. Examples of the solvent forming a solvate include, but are not limited to, water, isopropanol, ethanol, methanol, dimethyl sulfoxide, ethyl acetate, acetic acid, and ethanolamine.

The present disclosure also provides a pharmaceutical composition for preventing or treating cancer, inflammatory disorders, or autoimmune diseases, including at least one of the compound represented by Formula I, an isomer thereof, a pharmaceutically acceptable salt thereof, a hydrate thereof, and a solvate thereof, as an active ingredient.

In an embodiment, the cancer may be cancer dependent on oncogene MYC, tumor harboring a loss of function mutation of CBP or p300, or cancer disease with activation of CBP and/or p300 function.

In an embodiment, the cancer disease may be one or more selected from the group consisting of blood cancer, prostate cancer, breast cancer, lung cancer, pancreatic cancer, colorectal cancer, and melanoma.

In an embodiment, the cancer disease may be one or more selected from the group consisting of acoustic neuroma, acute leukemia, acute lymphoblastic leukemia, acute myeloid leukemia, acute T-cell leukemia, basal cell carcinoma, cholangiocarcinoma, bladder cancer, brain cancer, breast cancer, bronchial carcinoma, cervical cancer, chondrosarcoma, chordoma, choriocarcinoma, chronic leukemia, chronic lymphocytic leukemia, chronic myeloid leukemia, chronic myeloid leukemia, colon cancer, colon cancer, craniopharyngioma, cystadenocarcinoma, diffuse large B-cell lymphoma, dysproliferative changes, embryonal carcinoma, endometrial cancer, endotheliosarcoma, ependymoma, epithelial carcinoma, erythroleukemia, esophageal cancer, estrogen receptor-positive breast cancer, essential thrombocythemia, Ewing's tumor, fibrosarcoma, follicular lymphoma, germ cell testicular cancer, glioma, glioblastoma, pedunculated tumor, heavy chain disease head and neck cancer, hemangioblastoma, hepatoma, hepatocellular carcinoma, hormone-insensitive prostate cancer, leiomyosarcoma, leukemia, liposarcoma, lung cancer, lymphoendotheliosarcoma, lymphangiosarcoma, lymphocytic leukemia, lymphoma, lymphoid malignancy of T-cell or B-cell origin, medullary carcinoma, medulloblastoma, melanoma, meningioma, mesothelioma, multiple myeloma, myeloid leukemia, myeloma, myxosarcoma, neuroblastoma, NUT midline carcinoma (NMC), non-small cell lung cancer, oligodendroglioma, oral cancer, osteosarcoma, ovarian cancer, pancreatic cancer, papillary adenocarcinoma, papillary carcinoma, pinealoma, polycythemia vera, prostate cancer, rectal cancer, renal cell carcinoma, retinoblastoma, rhabdomyosarcoma, sarcoma, sebaceous carcinoma, seminoma, skin cancer, small cell lung carcinoma, solid tumors (carcinomas and sarcomas), small cell lung cancer, stomach cancer, squamous cell carcinoma, synovioma, sweat gland carcinoma, thyroid cancer, Waldenstrom macroglobulinemia, testicular tumor, uterine cancer and Wilms tumor.

In an embodiment, the inflammatory disorder or autoimmune disease may be one or more selected from the group consisting of Addison's disease, acute gout, ankylosing spondylitis, asthma, atherosclerosis, Behcet's disease, bullous skin disease, chronic obstructive pulmonary disease, Crohn's disease, dermatitis, eczema, giant cell arteritis, fibrosis, glomerulonephritis, hepatic vascular occlusion, hepatitis, hypophysitis, immunodeficiency syndrome, inflammatory bowel disease, Kawasaki disease, lupus nephritis, multiple sclerosis, myocarditis, myositis, nephritis, organ transplant rejection, osteoarthritis, pancreatitis, pericarditis, polyarteritis nodosa pneumonia, primary biliary cirrhosis, psoriasis, psoriatic arthritis, rheumatoid arthritis, scleritis, sclerosing cholangitis, sepsis, systemic lupus erythematosus, Takayasu's arteritis, toxic shock, thyroiditis, type 1 diabetes, ulcerative colitis, uveitis, vitiligo, vasculitis, and Wegener's granulomatosis.

In an embodiment, the pharmaceutical composition may further include a pharmaceutically acceptable diluent or carrier. Specifically, the pharmaceutical composition of the present disclosure may include a pharmaceutically acceptable carrier, and may be formulated in the form of oral dosage forms such as powders, granules, tablets, capsules, suspensions, emulsions, syrups and aerosols, external preparations, suppositories and sterile injectable solutions, respectively, according to common methods. The pharmaceutically acceptable carrier may include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinyl pyrrolidone, water, methyl hydroxybenzoate, propyl hydroxybenzoate, talc, magnesium stearates and mineral oil. In addition, the carrier may include diluents or excipients, such as fillers, weighting agents, binders, wetting agents, disintegrating agents and surfactants. An oral solid preparation may include tablets, pills, powders, granules and capsules, and the solid preparation may include one or more excipients, for example, starch, calcium carbonate, sucrose or lactose, and gelatin, and may include a lubricant such as magnesium stearate and talc. An oral liquid preparation may include suspensions, solutions, emulsions and syrups, and may include diluents such as water and liquid paraffin, wetting agents, sweeteners, flavoring agents and preservatives. A parenteral preparation may include sterile aqueous solutions, non-aqueous solvents, suspensions, emulsions, lyophilized preparations and suppositories, and the non-aqueous solvent and suspension may include propylene glycol, polyethylene glycol, vegetable oils such as olive oil, and injectable esters such as ethyl oleate. As a suppository base, witepsol, macrogol, tween 61, cacao butter, laurin butter, glycerogelatin, or the like may be used.

In an embodiment, the dosage of the compound represented by Formula I, the isomer thereof, the pharmaceutically acceptable salt thereof, the hydrate thereof or the solvate thereof, contained in the pharmaceutical composition of the present disclosure varies depending on the patient's condition, weight, the degree of the disease, the drug form, the administration route and period, but may be appropriately selected by a person skilled in the art. For example, the compound represented by Formula I, or the pharmaceutically acceptable salt thereof may be administered at a dosage of 0.0001 to 1000 mg/kg, preferably 0.01 to 1000 mg/kg per day, and the administration may be performed once a day or in several divided doses. In addition, the pharmaceutical composition of the present disclosure may contain the compound represented by Formula I, or the pharmaceutically acceptable salt thereof, in a weight percentage of 0.001 to 90% based on the total weight of the composition.

In an embodiment, the pharmaceutical composition of the present disclosure may be administered to mammals such as rats, mice, livestock, and humans by various routes, for example, orally, intraperitoneally, rectally, or by intravenous, intramuscular, subcutaneous, intrauterine or intracerebroventricular injection.

Hereinafter, the present disclosure will be described in more detail through preparation examples, examples, and experimental examples. However, the preparation examples, examples and experimental examples below are intended to illustrate the present disclosure, and the scope of the present disclosure is not limited thereto.

The compound having the structure of Formula I may be synthesized by the method below.

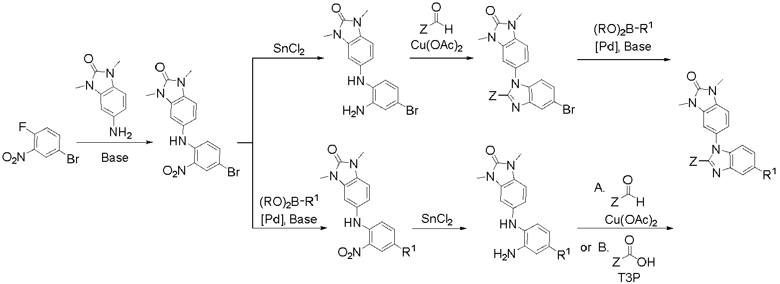

The analyses of the compounds prepared in the Preparation Examples and Examples were performed as follows: Nuclear magnetic resonance (NMR) spectroscopy was performed on a Bruker 500 MHz or Jeol 400 MHz spectrometer. Mass spectrometry was performed using LC/MS (Agilent 1260 infinity II/Agilent infinity lab LC/MSD, API-ES mode, UV detection at 220 nm/254 nm). (Column: ZORBAX RRHT StableBond C18, 2.1 x 50 mm, 1.8 µm, 80 Å, mobile phase: water/acetonitrile/formic acid (30/70/0.1%), isocratic elution for 3 min.)

In addition, medium pressure liquid chromatography (MPLC, CombiFlash Nextgen 300+) or prepHPLC (Thermo Ultimate 3000 equipped with a diode array detector, UV detection: 215 nm, 254 nm, 280 nm) was used for the purification of the compounds. The prepHPLC conditions were as follows. (Column: Phenomenex Luna, 10 µm, C18(2), 100 Å, 250 x 21.20 mm), mobile phase A: water/0.1% trifluoroacetic acid, mobile phase B: acetonitrile/0.1% trifluoroacetic acid, gradient elution for 30 min, A:B = 75:25 - 65:35)

Each starting material is a known compound and was synthesized according to the literature or purchased commercially.

The analyses of the compounds prepared in the Preparation Examples and Examples were performed as follows: Nuclear magnetic resonance (NMR) spectroscopy was performed on a Bruker 500 MHz or Jeol 400 MHz spectrometer. Mass spectrometry was performed using LC/MS (Agilent 1260 infinity II/Agilent infinity lab LC/MSD, API-ES mode, UV detection at 220 nm/254 nm). (Column: ZORBAX RRHT StableBond C18, 2.1 x 50 mm, 1.8 µm, 80 Å, mobile phase: water/acetonitrile/formic acid (30/70/0.1%), isocratic elution for 3 min.)

In addition, medium pressure liquid chromatography (MPLC, CombiFlash Nextgen 300+) or prepHPLC (Thermo Ultimate 3000 equipped with a diode array detector, UV detection: 215 nm, 254 nm, 280 nm) was used for the purification of the compounds. The prepHPLC conditions were as follows. (Column: Phenomenex Luna, 10 µm, C18(2), 100 Å, 250 x 21.20 mm), mobile phase A: water/0.1% trifluoroacetic acid, mobile phase B: acetonitrile/0.1% trifluoroacetic acid, gradient elution for 30 min, A:B = 75:25 - 65:35)

Each starting material is a known compound and was synthesized according to the literature or purchased commercially.

### Intermediate Synthetic Method

### Intermediate I-1: Preparation of 5-amino-1,3-dimethyl-1,3-dihydro-2H-benzo[d]imidazol-2-one

### Step 1) Preparation of 5-nitro-1,3-dihydro-2H-benzo[d]imidazol-2-one

4-Nitrobenzene-1,2-diamine (10 g, 65.3 mmol) was dissolved in tetrahydrofuran (365 mL), and cooled to 0°C, and then 1,1-carboimidazole (21 g, 130.6 mmol) was added. The reaction solution was stirred at room temperature for 2 hours. The reaction solution was concentrated under reduced pressure, and the obtained solid was washed with diethyl ether, 1 N hydrochloric acid and water. The solid was dried under vacuum to obtain the title compound. (9.59 g, 53.5 mmol, 82% yield)

¹H NMR (500 MHz, DMSO-d₆) δ 11.43 (s, 1H), 11.20 (s, 1H), 7.94 (dd, J = 8.6, 2.3 Hz, 1H), 7.71 (d, J = 2.2 Hz, 1H), 7.10 (d, J = 8.6 Hz, 1H)

### Step 2) Preparation of 1,3-dimethyl-5-nitro-1,3-dihydro-2H-benzo[d]imidazol-2-one

5-Nitro-1,3-dihydro-2H-benzo[d]imidazol-2-one (4.9 g, 27.5 mmol) was dissolved in dimethylformamide (70 mL) and cooled to 0°C. Sodium hydride (60% dispersed in mineral oil, 4.4 g, 110 mmol) was added little by little, and methyl iodide (11.7 g, 5.13 mL, 83.0 mmol) was added. The reaction solution was stirred at 60°C for 2 hours and then water was added to terminate the reaction. The reaction solution was diluted with ethyl acetate and washed with 0.1 N hydrochloric acid and brine. An organic layer was distilled under reduced pressure, and the obtained solid was washed with hexane and dried under vacuum to obtain the title compound. (4.95 g, 23.4 mmol, 87% yield).

¹H NMR (500 MHz, CHLOROFORM-d) δ 8.12 (dd, J = 8.6, 2.2 Hz, 1H), 7.89 (d, J = 2.1 Hz, 1H), 7.02 (d, J = 8.6 Hz, 1H), 3.50 (s, 3H), 3.49 (s, 3H)

### Step 3) Preparation of 5-amino-1,3-dimethyl-1,3-dihydro-2H-benzo[d]imidazol-2-one

1,3-Dimethyl-5-nitro-1,3-dihydro-2H-benzo[d]imidazol-2-one (3.5 g, 16.9 mmol) was dissolved in ethyl acetate/methanol (50 mL/50 mL) and palladium/carbon (10 wt%, 0.7 g) was added. After the reaction system was replaced with hydrogen using a hydrogen balloon, the reaction solution was stirred under hydrogen conditions for 3 hours. After the reaction was completed, the reaction solution was filtered through a celite pad to remove a catalyst. The filtrate was concentrated under reduced pressure to obtain the title compound, which was used in a subsequent reaction without further purification. (2.79 g, 15.7 mmol, 93% yield).

¹H NMR (500 MHz, DMSO-d₆) δ 6.78 (d, J = 8.2 Hz, 1H), 6.35 (d, J = 2.0 Hz, 1H), 6.31 (dd, J = 8.2, 2.1 Hz, 1H), 4.77 (s, 2H), 3.22 (s, 3H), 3.21 (s, 3H)

### Intermediate I-2: Preparation of 4-fluoro-N-methyl-3-nitrobenzenesulfonamide

4-Fluoro-3-nitrobenzenesulfonyl chloride (1.076 g, 4.49 mmol) was dissolved in dichloromethane (8.98 mL), and methylamine (2 M in tetrahydrofuran, 5.6 mL, 11.23 mmol) was slowly added dropwisely at 0°C. The reaction solution was stirred at room temperature for 3 hours. After the reaction was completed, the reaction solution was filtered and washed with ethyl acetate. The filtrate was concentrated under reduced pressure, and the residue was purified by MPLC to obtain the title compound. (533 mg, 2.276 mmol, 50.7% yield).

¹H-NMR (400 MHz, CHLOROFORM-d) δ 8.55 (dd, J = 6.9, 2.3 Hz, 1H), 8.16-8.13 (m, 1H), 7.51-7.47 (m, 1H), 5.10-5.07 (m, 1H), 2.71 (d, J = 5.0 Hz, 3H)

### Intermediate I-3: Preparation of 4-fluoro-N-methyl-3-nitrobenzamide

4-Fluoro-3-nitrobenzoic acid (2 g, 10.8 mmol) and 1-ethyl-3-(3-dimethylaminopropyl)carbodiimide hydrochloride (2.69 g, 14.1 mmol) were dissolved in dichloromethane (30 mL), and methylamine (2 M in tetrahydrofuran, 5.4 mL, 10.8 mmol) was slowly added dropwisely at 0°C. The reaction solution was stirred at room temperature for 2 hours. After the reaction was completed, the solution was cooled to 0°C, water was added, and an organic layer was extracted with dichloromethane. The organic layer was washed with 1 N sodium hydroxide aqueous solution and brine, and concentrated under reduced pressure. The resultant was used in a subsequent reaction without further purification. (1.8 g, 9.08 mmol, 84% yield)

¹H-NMR (500 MHz, CHLOROFORM-d) δ 8.47 (dd, J = 6.9, 2.3 Hz, 1H), 8.14 (qd, J = 4.3, 2.4 Hz, 1H), 7.41 (dd, J = 10.1, 8.8 Hz, 1H), 6.21 (s, 1H), 3.08 (d, J = 4.9 Hz, 3H)

### Intermediate I-4 (Synthetic Method I-A): Preparation of 3-amino-4-((1,3-dimethyl-2-oxo-2,3-dihydro-1H-benzo[d]imidazole-5-yl)-N-methylbenzenesulfonamide

### Step 1) Preparation of 4-((1,3-dimethyl-2-oxo-2,3-dihydro-1H-benzo[d]imidazol-5-yl)amino)-N-methyl-3-nitrobenzenesulfonamide

4-Fluoro-N-methyl-3-nitrobenzenesulfonamide (Intermediate I-2, 533 mg, 2.276 mmol) was dissolved in 1,4-dioxane (5.5 mL) and then 5-amino-1,3-dimethyl-1,3-dihydro-2H-benzo[d]imidazol-2-one (Intermediate I-1, 403 mg, 2.276 mmol), and N,N-diisopropylethylamine (994 µl, 5.69 mmol) were added. The reaction solution was stirred at 110°C for 18 hours. After the reaction was completed, the solvent was removed by concentration under reduced pressure, and the produced solid was washed with dichloromethane and methanol to obtain the title compound without further purification. (846 mg, 2.161 mmol, 95% yield).

¹H-NMR (400 MHz, CHLOROFORM-d) δ 9.80 (s, 1H), 8.74 (s, 1H), 7.72 (d, J = 8.7 Hz, 1H), 7.07-7.03 (m, 3H), 6.90 (s, 1H), 4.40-4.39 (m, 1H), 3.47 (s, 3H), 3.43 (s, 3H), 2.70 (d, J = 5.0 Hz, 3H)

### Step 2) Preparation of 3-amino-4-((1,3-dimethyl-2-oxo-2,3-dihydro-1H-benzo[d]imidazol-5-yl)amino)-N-methylbenzenesulfonamide

The compound of 4-((1,3-dimethyl-2-oxo-2,3-dihydro-1H-benzo[d]imidazol-5-yl)amino)-N-methyl-3-nitrobenzenesulfonamide (846 mg, 2.161 mmol) obtained above was dissolved in ethanol (10 mL), and tin(II) chloride dihydrate (1.463 g, 6.48 mmol) was added. The reaction solution was stirred at 90°C for 18 hours. After the reaction was completed, 1 N sodium hydroxide aqueous solution was added, and an organic layer was extracted with dichloromethane. The organic layer was washed with water and brine, dried over anhydrous magnesium sulfate, and concentrated under reduced pressure, and the residue was purified by MPLC to obtain the title compound. (610 mg, 1.688 mmol, 78% yield)

¹H-NMR (400 MHz, DMSO-d₆) δ 7.34 (brs, 1H), 7.15 (s, 1H), 7.09-7.04 (m, 2H), 7.00-6.94 (m, 2H), 6.88 (d, J = 1.8 Hz, 1H), 6.80 (dd, J = 8.2, 1.8 Hz, 1H), 3.31 (s, 3H), 3.28 (s, 3H), 2.38 (d, J = 3.7 Hz, 3H)

### Intermediate I-5 (Synthetic Method I-B): Preparation of 5-bromo-1',3'-dimethyl-2-(1-methyl-1H-imidazol-5-yl)-1',3'-dihydro-2'H-[1,5'-bibenzo[d]imidazol]-2'-one

### Step 1) Preparation of 5-((4-bromo-2-nitrophenyl)amino)-1,3-dimethyl-1,3-dihydro-2H-benzo[d]imidazol-2-one

The title compound was obtained in a similar manner to Step 1 of Synthetic Method I-A, using 5-amino-1,3-dimethyl-1,3-dihydro-2H-benzo[d]imidazol-2-one (Intermediate I-1, 2.85 g, 16.07 mmol) and 4-bromo-1-fluoro-2-nitrobenzene (2 mL, 16.07 mmol). (3 g, 7.95 mmol, 49.5% yield)

¹H-NMR (500 MHz, DMSO-d₆) δ 9.51 (s, 1H), 8.22 (d, J = 2.4 Hz, 1H), 7.59-7.56 (m, 1H), 7.22-7.19 (m, 2H), 7.04-7.02 (m, 1H), 6.94 (d, J = 9.2 Hz, 1H), 3.35 (s, 3H), 3.31 (s, 3H)

### Step 2) Preparation of 5-((2-amino-4-bromophenyl)amino)-1,3-dimethyl-1,3-dihydro-2H-benzo[d]imidazol-2-one

The title compound was obtained in a similar manner to Step 2 of Synthetic Method I-A using the compound 5-((4-bromo-2-nitrophenyl)amino)-1,3-dimethyl-1,3-dihydro-2H-benzo[d]imidazol-2-one (3 g, 7.95 mmol) obtained above. (2.4 g, 6.91 mmol, 87% yield)

¹H-NMR (400 MHz, DMSO-d₆) δ 6.96 (d, J = 8.2 Hz, 1H), 6.92 (s, 1H), 6.87-6.85 (m, 2H), 6.62-6.59 (m, 2H), 6.56 (dd, J = 8.2, 1.8 Hz, 1H), 5.04 (s, 2H), 3.27 (s, 3H), 3.23 (s, 3H), LC/MS: 347 349 (M+H)

### Step 3) Preparation of 5-bromo-1',3'-dimethyl-2-(1-methyl-1H-imidazol-5-yl)-1',3'-dihydro-2'H-[1,5'-bibenzo[d]imidazol]-2'-one

The compound 5-((2-amino-4-bromophenyl)amino)-1,3-dimethyl-1,3-dihydro-2H-benzo[d]imidazol-2-one (450 mg, 1.296 mmol) obtained above and 1-methyl-1H-imidazole-5-carbaldehyde (143 mg, 1.296 mmol) were dissolved in acetic acid (1 mL), and then copper acetate (118 mg, 0.648 mmol) was added. The reaction solution was stirred at 80°C for 30 minutes. After the reaction was completed, water and 1 N sodium hydroxide aqueous solution were added, and an organic layer was extracted with dichloromethane. The organic layer was washed with water and brine, dried over anhydrous magnesium sulfate and concentrated under reduced pressure, and the residue was purified by MPLC to obtain the title compound. (337 mg, 0.771 mmol, 59.5% yield)

¹H-NMR (500 MHz, CHLOROFORM-d) δ 7.95 (s, 1H), 7.61 (s, 1H), 7.33 (d, J = 8.5 Hz, 1H), 7.12-7.06 (m, 2H), 6.94-6.91 (m, 2H), 6.57 (s, 1H), 4.14 (s, 3H), 3.51 (s, 3H), 3.39 (s, 3H)

### Intermediate I-6 (Synthetic Method I-C): Preparation of 3'-amino-4'-((1,3-dimethyl-2-oxo-2,3-dihydro-1H-benzo[d]imidazol-5-yl)amino)-3-fluoro-N-methyl-[1,1'-biphenyl]-4-carboxamide

### Step 1) Preparation of 4'-((1,3-dimethyl-2-oxo-2,3-dihydro-1H-benzo[d]imidazol-5-yl)amino)-3-fluoro-N-methyl-3'-nitro-[1,1'-biphenyl]-4-carboxamide

5-((4-Bromo-2-nitrophenyl)amino)-1,3-dimethyl-1,3-dihydro-2H-benzo[d]imidazol-2-one (Compound of Step 1 of Synthetic Method I-B, 353 mg, 0.936 mmol), (3-fluoro-4-(methylcarbamoyl)phenyl)boronic acid (203 mg, 1.029 mmol), and tripotassium phosphate (397 mg, 1.872 mmol) were dissolved in 1,4-dioxane (4 mL) and water (1 mL), and then 1,1'-bis(diphenylphosphino)ferrocene]palladium(II) dichloride dichloromethane adduct (76 mg, 0.094 mmol) was added. The reaction solution was stirred under nitrogen conditions at 100°C for 3 hours. After the reaction was completed, water was added, and an organic layer was extracted with dichloromethane. The organic layer was washed with water and brine, dried over anhydrous magnesium sulfate and concentrated under reduced pressure. The produced solid was washed with ethanol and used in a subsequent reaction without further purification. (438 mg, 0.565 mmol, 60.4% yield)

¹H-NMR (500 MHz, DMSO-d₆) δ 9.60 (s, 1H), 8.46 (s, 1H), 8.22 (m, 1H), 7.90 (d, J = 8.9 Hz, 1H), 7.72-7.59 (m, 3H), 7.24-7.22 (m, 2H), 7.09 (s, 1H), 7.07 (s, 1H), 3.36 (s, 3H), 3.32 (s, 3H), 2.79 (d, J = 4.3 Hz, 3H)

### Step 2) Preparation of 3'-amino-4'-((1,3-dimethyl-2-oxo-2,3-dihydro-1H-benzo[d]imidazol-5-yl)amino)-3-fluoro-N-methyl-[1,1'-biphenyl]-4-carboxamide

The title compound was obtained in a similar manner to Step 2 of Synthetic Method I-A using the compound 4'-((1,3-dimethyl-2-oxo-2,3-dihydro-1H-benzo[d]imidazol-5-yl)amino)-3-fluoro-N-methyl-3'-nitro-[1,1'-biphenyl]-4-carboxamide (254 mg, 0.565 mmol) obtained above. (228 mg, 0.446 mmol, 79% yield)

¹H-NMR (500 MHz, DMSO-d₆) δ 8.17-8.16 (m, 1H), 7.68 (t, J = 7.9 Hz, 1H), 7.47-7.41 (m, 2H), 7.10 (s, 1H), 7.06 (s, 1H), 7.04-6.99 (m, 2H), 6.92 (d, J = 8.2 Hz, 1H), 6.74 (s, 1H), 6.68 (d, J = 8.2 Hz, 1H), 4.90 (s, 2H), 3.29 (s, 3H), 3.26 (s, 3H), 2.79 (d, J = 4.6 Hz, 3H), LC/MS: 420 (M+H)

### Intermediate 1-7: Preparation of ((4-fluoro-3-nitrophenyl)ethynyl)trimethylsilane

4-Bromo-1-fluoro-2-nitrobenzene (4.4 g, 20 mmol), bis(triphenylphosphine)palladium(II) dichloride (1.4 g, 2 mmol), and copper iodide (0.570 g, 3 mmol) were dissolved in toluene (66 mL) and water (33 mL), and then trimethylsilylacetylene (4.2 mL, 30 mmol) was added. The reaction solution was stirred at room temperature for 2 hours. After the reaction was completed, the reaction solution was concentrated under reduced pressure to remove the solvent, and then water and ethyl acetate were added to extract an organic layer. The organic layer was washed with water and brine, dried over anhydrous magnesium sulfate and concentrated under reduced pressure, and the residue was purified by MPLC to obtain the title compound. (4.13 g, 17.4 mmol, 87% yield)

¹H NMR (500 MHz, CHLOROFORM-d) δ 8.14 (dd, J = 7.1, 2.1 Hz, 1H), 7.68 (ddd, J = 8.6, 4.2, 2.2 Hz, 1H), 7.23 (dd, J = 10.5, 8.6 Hz, 1H), 0.26 (s, 9H)

### Example Synthetic Method

### Example 1 (Synthetic Method A): Preparation of 1-(1,3-dimethyl-2-oxo-benzimidazol-5-yl)-N-methyl-2-(3-methylimidazol-4-yl)benzimidazol-5-sulfonamide

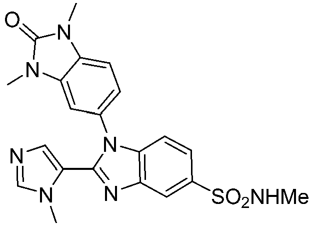

3-Amino-4-((1,3-dimethyl-2-oxo-2,3-dihydro-1H-benzo[d]imidazol-5-yl)amino)-N-methylbenzenesulfonamide (Intermediate I-4, 42.3 mg, 0.117 mmol) and 1-methyl-1H-imidazole-5-carbaldehyde (12.9 mg, 0.117 mmol) were dissolved in acetic acid (5.3 mL), and then copper acetate (21.28 mg, 0.117 mmol) was added. The reaction solution was stirred at 85°C for 1 hour. After the reaction was completed, water and 1 N sodium hydroxide aqueous solution were added, and an organic layer was extracted with dichloromethane. The organic layer was washed with water and brine, dried over anhydrous magnesium sulfate and concentrated under reduced pressure, and the residue was purified by MPLC and PTLC to obtain the title compound. (21 mg, 0.047 mmol, 39.7% yield)

¹H-NMR (400 MHz, Methanol-d₄) δ 8.25 (d, J = 1.4 Hz, 1H), 7.82 (brs, 1H), 7.74 (dd, J = 8.5, 1.6 Hz, 1H), 7.38-7.35 (m, 2H), 7.31 (d, J = 8.2 Hz, 1H), 7.22 (dd, J = 8.2, 1.8 Hz, 1H), 6.56 (brs, 1H), 4.10 (s, 3H), 3.50 (s, 3H), 3.40 (s, 3H), 2.53 (s, 3H)

### Example 3 (Synthetic Method B): Preparation of 1-(1,3-dimethyl-2-oxo-benzimidazol-5-yl)-N-methyl-2-(3-phenylimidazol-4-yl)benzimidazole-5-sulfonamide

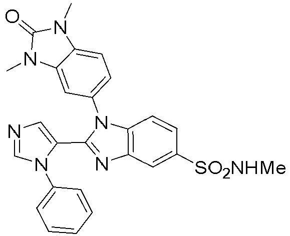

3-Amino-4-((1,3-dimethyl-2-oxo-2,3-dihydro-1H-benzo[d]imidazol-5-yl)amino)-N-methylbenzenesulfonamide (Intermediate I-4, 50 mg, 0.138 mmol), 1-phenyl-1H-imidazole-5-carboxylic acid (26.0 mg, 0.138 mmol), and N,N-diisopropylethylamine (0.031 mL, 0.180 mmol) were dissolved in ethyl acetate (3.2 mL), and then 2,4,6-tripropyl-1,3,5,2,4,6-trioxatriphosphinane 2,4,6-trioxide (50% wt in ethyl acetate, 0.107 mL, 0.180 mmol) and acetic acid (78 µl, 1.38 mmol) were added thereto, followed by stirring at 90°C for 48 hours. After the reaction was completed, 1 N sodium hydroxide aqueous solution was added, and an organic layer was extracted with ethyl acetate. The organic layer was washed with water and brine, dried over anhydrous magnesium sulfate and concentrated under reduced pressure. The residue was purified by MPLC and PTLC to obtain the title compound. (10 mg, 0.019 mmol, 14.07% yield)

¹H-NMR (500 MHz, CHLOROFORM-d) δ 7.85 (d, J = 8.5 Hz, 1H), 7.76 (dd, J = 8.2, 1.5 Hz, 1H), 7.68 (s, 1H), 7.63 (s, 1H), 7.23 (d, J = 7.6 Hz, 1H), 7.21 (s, 1H), 7.15-7.12 (m, 1H), 7.00 (d, J = 8.2 Hz, 1H), 6.89-6.83 (m, 3H), 6.79 (dd, J = 8.2, 1.8 Hz, 1H), 6.62 (d, J = 1.8 Hz, 1H), 3.51 (s, 3H), 3.37 (s, 3H), 2.62 (d, J = 5.2 Hz, 3H)

### Example 7 (Synthetic Method C): Preparation of 1,3-dimethyl-5-[2-(3-methylimidazol-4-yl)-5-phenyl-benzimidazol-1-yl]benzimidazol-2-one

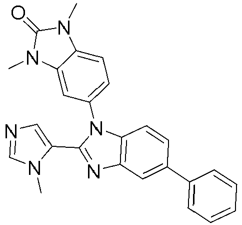

5-Bromo-1',3'-dimethyl-2-(1-methyl-1H-imidazol-5-yl)-1',3'-dihydro-2'H-[1,5'-bibenzo[d]imidazol]-2'-one (Intermediate I-5, 37.9 mg, 0.087 mmol), phenylboronic acid (12.68 mg, 0.104 mmol), and tripotassium phosphate (36.8 mg, 0.173 mmol) were dissolved in 1,4-dioxane (0.8 mL) and water (0.2 mL). 1,1'-Bis(diphenylphosphino)ferrocene]palladium(II) dichloride dichloromethane adduct (7.08 mg, 8.67 µmol) was added. The reaction solution was stirred under nitrogen conditions at 100°C for 4 hours. After the reaction was completed, water was added, and an organic layer was extracted with dichloromethane. The organic layer was washed with water and brine, dried over anhydrous magnesium sulfate and concentrated under reduced pressure. The residue was purified by MPLC and PTLC to obtain the title compound. (30 mg, 0.069 mmol, 80% yield)

¹H-NMR (500 MHz, CHLOROFORM-d) δ 8.05 (s, 1H), 7.68-7.66 (m, 2H), 7.51-7.44 (m, 4H), 7.34 (t, J = 7.3 Hz, 1H), 7.13 (d, J = 8.2 Hz, 1H), 7.12 (s, 2H), 6.96 (s, 1H), 6.47 (s, 1H), 4.17 (s, 3H), 3.51 (s, 3H), 3.40 (s, 3H), LC/MS: 434 (M+H)

### Example 8 and Example 13 (Synthetic Method D): Preparation of 5-[5-bromo-2-(6-methyl-1H-indol-3-yl)benzimidazol-1-yl]-1,3-dimethyl-benzimidazol-2-one and tert-butyl 4-[1-(1,3-dimethyl-2-oxo-benzimidazol-5-yl)-2-(6-methyl-1H-indol-3-yl)benzimidazol-5-yl]piperidine-1-carboxylate

### Step 1) Preparation of 5-[5-bromo-2-(6-methyl-1H-indol-3-yl)benzimidazol-1-yl]-1,3-dimethyl-benzimidazol-2-one (Example 8)

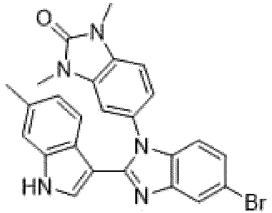

The title compound was obtained in a similar manner to Step 3 of Synthetic Method I-B using 5-((2-amino-4-bromophenyl)amino)-1,3-dimethyl-1,3-dihydro-2H-benzo[d]imidazol-2-one (compound of Step 2 of Synthetic Method I-B, 250 mg, 0.720 mmol) and 6-methyl-1H-indole-3-carbaldehyde (114.6 mg, 0.720 mmol). (280 mg, 0.576 mmol, 80.0% yield)

¹H-NMR (400MHz, DMSO-d₆) δ 11.19 (s, 1H), 8.47 (d, 1H), 7.93 (s, 1H), 7.47 (m, 2H), 7.42 (d, 1H), 7.22 (m, 2H), 7.05 (d, 1H), 6.96 (m, 1H), 6.51 (d, 1H), 3.46 (s, 3H), 3.34 (s, 3H), 2.42 (s, 3H), LC/MS: 486, 488 (M+H)

### Step 2) Preparation of tert-butyl 4-(1',3'-dimethyl-2-(6-methyl-1H-indol-3-yl)-2'-oxo-2',3'-dihydro-1'H-[1,5'-bibenzo[d]imidazol]-5-yl)-3,6-dihydropyridine-1(2H)-carboxylate

The title compound was obtained in the same manner as described in Synthetic Method C using the compound 5-[5-bromo-2-(6-methyl-1H-indol-3-yl)benzimidazol-1-yl]-1,3-dimethyl-benzimidazol-2-one (Example 8, 300 mg, 0.617 mmol) obtained above and tert-butyl 4-(4,4,5,5-tetramethyl-1,3,2-dioxaborolan-2-yl)-3,6-dihydropyridine-1(2H)-carboxylate (191 mg, 0.617 mmol). (267 mg, 0.454 mmol, 73.5% yield)

### Step 3) Preparation of tert-butyl 4-[1-(1,3-dimethyl-2-oxo-benzimidazol-5-yl)-2-(6-methyl-1H-indol-3-yl)benzimidazol-5-yl]piperidine-1-carboxylate (Example 13)

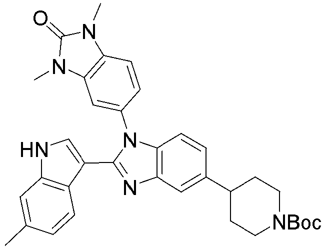

The compound tert-butyl 4-(1',3'-dimethyl-2-(6-methyl-1H-indol-3-yl)-2'-oxo-2',3'-dihydro-1'H-[1,5'-bibenzo[d]imidazol]-5-yl)-3,6-dihydropyridine-1(2H)-carboxylate (200 mg, 0.340 mmol) obtained above was dissolved in methanol/tetrahydrofuran (2:1), and then palladium/carbon (36 mg, 0.340 mmol) was added. The reaction solution was stirred at room temperature for 2 hours under hydrogen conditions. After the reaction was completed, the reaction solution was passed through a celite filter, and the filtrate was concentrated and purified by MPLC to obtain the title compound. (175 mg, 0.296 mmol, 87.0% yield)

¹H-NMR (400MHz, DMSO-d₆) δ 11.10 (s, 1H), 8.48 (d, 1H), 7.58 (s, 1H), 7.46 (m, 2H), 7.19 (m, 2H), 7.03 (m, 2H), 6.92 (d, 1H), 6.49 (s, 1H), 4.12 (brs, 2H), 3.45 (s, 3H), 3.32 (s, 3H), 2.84 (m, 3H), 2.42 (s, 3H), 1.85 (m, 2H), 1.61 (m, 2H), 1.44 (s, 9H), LC/MS: 591 (M+H)

### Example 14 (Synthetic Method E): Preparation of 1,3-dimethyl-5-[2-(6-methyl-1H-indol-3-yl)-5-(4-piperidyl)benzimidazol-1-yl]benzimidazol-2-one

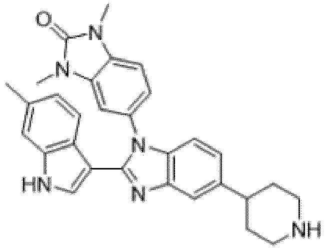

Tert-butyl 4-[1-(1,3-dimethyl-2-oxo-benzidazol-5-yl)-2-(6-methyl-1H-indol-3-yl)benzimidazol-5-yl]piperidine-1-carboxylate (Example 13, 170 mg, 0.288 mmol) was dissolved in dichloromethane, and then trifluoroacetic acid (2 mL) was added. The reaction solution was stirred at room temperature for 1 hour. The reaction solution was concentrated under reduced pressure and then neutralized with a saturated sodium bicarbonate solution. An organic layer was extracted with ethyl acetate, washed with water and brine, dried over anhydrous magnesium sulfate and concentrated under reduced pressure. The residue was purified by MPLC to obtain the title compound. (105 mg, 0.214 mmol, 74.4% yield)

¹H-NMR (400MHz, DMSO-d₆) δ 11.11 (d, 1H), 8.48 (d, 1H), 7.57 (s, 1H), 7.42 (m, 2H), 7.20 (m, 2H), 7.05 (m, 2H), 6.93 (d, 1H), 6.50 (d, 1H), 3.45 (s, 3H), 3.34 (s, 3H), 3.19 (m, 3H), 2.80 (m, 3H), 2.42 (s, 3H), 1.86 (m, 2H), 1.72 (m, 2H), 1.44 (s, 9H), LC/MS 491 (M+H)

### Example 15: Preparation of 5-(1-ethylpiperidin-4-yl)-1',3'-dimethyl-2-(6-methyl-1H-indol-3-yl)-1',3'-dihydro-2'H-[1,5'-bibenzo[d]imidazol]-2'-one formate

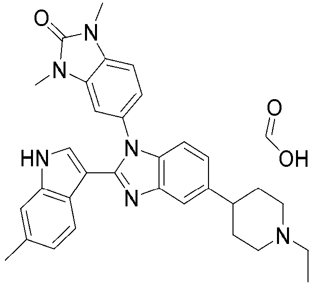

1,3-Dimethyl-5-[2-(6-methyl-1H-indol-3-yl)-5-(4-piperidyl)benzimidazol-1-yl]benzimidazol-2-one (Example 14, 30 mg, 0.061 mmol) was dissolved in acetonitrile, and then bromoethane (8 mg, 0.073 mmol) and potassium carbonate (17 mg, 0.122 mmol) were added. The reaction solution was stirred at room temperature for 1 hour. The reaction solution was concentrated under reduced pressure, and the residue was purified by prepHPLC to obtain the title compound. (16 mg, 0.031 mmol, 50.4% yield)

¹H-NMR (400MHz, DMSO-d₆) δ 11.11 (d, 1H), 8.48 (d, 1H), 8.20 (s, 1H), 7.59 (s, 1H), 7.42 (m, 2H), 7.20 (m, 2H), 7.07 (m, 2H), 6.92 (d, 1H), 6.49 (d, 1H), 3.45 (s, 3H), 3.36 (s, 3H), 3.14 (m, 4H), 2.68 (m, 1H), 2.42 (s, 3H), 2.24 (m 2H), 1.88 (m, 4H), 1.11 (t, 3H) ; LC/MS: 519 (M+H)

### Example 20 (Synthetic Method F): Preparation of 1,3-dimethyl-5-[2-(3-methylimidazol-4-yl)-5-(1-piperidyl)benzimidazol-1-yl]benzimidazol-2-one

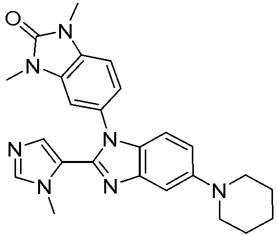

5-Bromo-1'3'-dimethyl-2-(1-methyl-1H-imidazol-5-yl)-1',3'-dihydro-2'H-[1,5'-bibenzo[d]imidazol]-2'-one (Intermediate I-5, 58.2 mg, 0.133 mmol), piperidine (131 µl, 1.331 mmol), and potassium tert-butoxide (59.7 mg, 0.532 mmol) were dissolved in toluene (0.9 mL), and then di-tert-butyl(2',4',6'-triisopropyl-[1,1'-biphenyl]-2-yl)phosphane (5.65 mg, 0.013 mmol) and tris(dibenzylideneacetone)dipalladium(0) (6.09 mg, 6.65 µmol) were added. The reaction solution was stirred under nitrogen conditions at 110°C for 18 hours. After the reaction was completed, the reaction solution was passed through a celite filter, and the filtrate was concentrated and purified by PTLC to obtain the title compound. (1.3 mg, 0.003 mmol, 2.2% yield)

1H-NMR (500 MHz, Methanol-d4) δ 7.76 (s, 1H), 7.35 (s, 1H), 7.32 (d, J = 8.2 Hz, 1H), 7.27 (s, 1H), 7.17-7.09 (m, 3H), 6.61 (s, 1H), 3.97 (s, 3H), 3.50 (s, 3H), 3.40 (s, 3H), 3.17-3.15 (m, 4H), 1.82-1.77 (m, 4H), 1.64-1.59 (m, 3H).

### Example 30 (Synthetic Method G): Preparation of 5-[1-(1,3-dimethyl-2-oxo-benzimidazol-5-yl)-2-(3-methylimidazol-4-yl)benzimidazol-5-yl]-N-methylpyridine-2-carboxamide

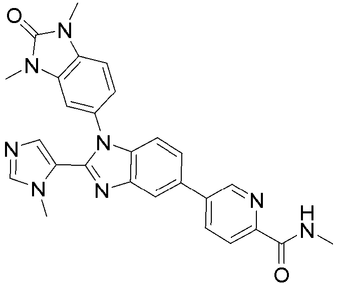

A compound obtained in the manner of Synthetic Method H of 5-(1',3'-dimethyl-2-(1-methyl-1H-imidazol-5-yl)-2'-oxo-2',3'-dihydro-1'H-[1,5'-bibenzo[d]imidazole]-5-yl)picolinic acid (45 mg, 0.094 mmol), 1-ethyl-3-(3-dimethylaminopropyl)carbodiimide hydrochloride (27.0 mg, 0.141 mmol), and 1-hydroxybenzotriazole hydrate (21.56 mg, 0.141 mmol) were dissolved in dimethylformamide (1 mL), and methylamine (2 M in tetrahydrofuran, 117 µl, 0.235 mmol) was slowly added. The reaction solution was stirred at room temperature for 18 hours. After the reaction was completed, water was added, and an organic layer was extracted with dichloromethane. The organic layer was washed with water and brine, dried over anhydrous magnesium sulfate and concentrated under reduced pressure, and the residue was purified by PTLC to obtain the title compound. (5 mg, 10.15 µmol, 10.82% yield)

¹H-NMR (500 MHz, CHLOROFORM-d) δ 8.84 (d, J = 1.8 Hz, 1H), 8.28 (d, J = 8.2 Hz, 1H), 8.10-8.07 (m, 2H), 8.04-8.01 (m, 1H), 7.54 (s, 1H), 7.50 (dd, J = 8.5, 1.5 Hz, 1H), 7.19 (d, J = 8.5 Hz, 1H), 7.16-7.09 (m, 2H), 6.97 (s, 1H), 6.47 (s, 1H), 4.19 (s, 3H), 3.53 (s, 3H), 3.42 (s, 3H), 3.07 (d, J = 5.2 Hz, 3H)

### Example 44 (Synthetic Method H): Preparation of 4-[1-(1,3-dimethyl-2-oxo-benzimidazol-5-yl)-2-(3-methylimidazol-4-yl)benzimidazol-5-yl]benzoic acid

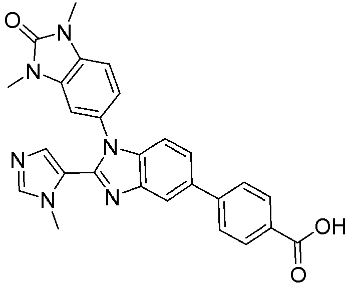

Methyl 4-[1-(1,3-dimethyl-2-oxo-benzimidazol-5-yl)-2-(3-methylimidazol-4-yl)benzimidazol-5-yl]benzoate (Example 43, 22 mg, 0.045 mmol) was dissolved in methanol (0.2 mL) and water (0.1 mL), and then lithium hydroxide hydrate (18.74 mg, 0.447 mmol) was added. The reaction solution was stirred at 50°C for 5 hours. After the reaction was completed, the reaction solution was neutralized with 1 N hydrochloric acid aqueous solution. Ethyl acetate was added and an organic layer was extracted. The organic layer was washed with water and brine, dried over anhydrous magnesium sulfate and concentrated under reduced pressure, and the residue was purified by PTLC to obtain the title compound. (5.6 mg, 0.012 mmol, 26.2% yield)

LC/MS: 479 (M+H)

### Example 58: Preparation of 5-[5-[4-(hydroxymethyl)phenyl]-2-(3-methylimidazol-4-yl)benzimidazol-1-yl]-1,3-dimethyl-benzimidazol-2-one

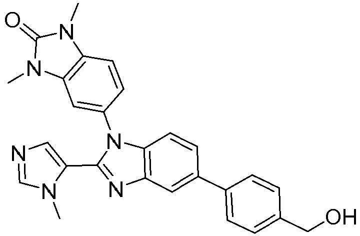

Methyl 4-[1-(1,3-dimethyl-2-oxo-benzimidazol-5-yl)-2-(3-methylimidazol-4-yl)benzimidazol-5-yl]benzoate (Example 43, 22 mg, 0.045 mmol) was dissolved in tetrahydrofuran (0.4 mL), and lithium aluminum hydride (1 M in tetrahydrofuran, 0.089 mL, 0.089 mmol) was slowly added at 0°C. The reaction solution was stirred at room temperature for 4 hours. Water was slowly added at 0°C to terminate the reaction, and the reaction solution was filtered. Water and ethyl acetate were added to the filtrate, and an organic layer was extracted. The organic layer was washed with water and brine, dried over anhydrous magnesium sulfate and concentrated under reduced pressure, and the residue was purified by MPLC to obtain the title compound. (10.5 mg, 0.023 mmol, 50.6% yield)

¹H-NMR (500 MHz, CHLOROFORM-d) δ 8.03 (s, 1H), 7.65 (d, J = 8.2 Hz, 2H), 7.53 (s, 1H), 7.46 (d, J = 8.2 Hz, 2H), 7.12-7.10 (m, 3H), 6.95 (s, 1H), 6.46 (s, 1H), 4.75 (s, 2H), 4.17 (s, 3H), 3.52 (s, 3H), 3.40 (s, 3H)

### Example 71: Preparation of 1,3-dimethyl-5-[2-(3-methylimidazol-4-yl)-5-phenoxy-benzimidazol-1-yl]benzimidazol-2-one

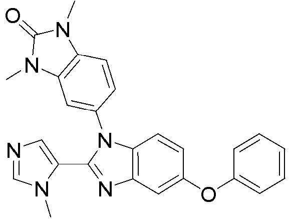

5-Bromo-1',3'-dimethyl-2-(1-methyl-1H-imidazol-5-yl)1',3'-dihydro-2'H-[1,5'-bibenzo[d]imidazol]-2'-one (Intermediate I-5, 19.8 mg, 0.045 mmol) was dissolved in 1,4-dioxane (1.4 mL), and then phenol (12.78 mg, 0.136 mmol), cesium carbonate (88.6 mg, 0.272 mmol), dimethylglycine (8.74 µl, 0.09 mmol), and copper iodide (7.77 mg, 0.042 mmol) were added. The reaction solution was stirred at 110°C for 18 hours. After the reaction was completed, the reaction solution was passed through a celite filter, and the filtrate was concentrated and purified by PTLC to obtain the title compound. (5.3 mg, 0.012 mmol, 26.0% yield)

¹H-NMR (500 MHz, CHLOROFORM-d) δ 7.51 (s, 1H), 7.46 (s, 1H), 7.34-7.31 (m, 2H), 7.12 (s, 2H), 7.08 (t, J = 7.5 Hz, 1H), 7.04-7.02 (m, 4H), 6.96 (s, 1H), 6.45 (s, 1H), 4.14 (s, 3H), 3.52 (s, 3H), 3. 41 (s, 3H), LC/MS: 451 (M+H)

### Example 84 (Synthetic Method I): Preparation of 4-[2-cyclopropyl-1-(1,3-dimethyl-2-oxo-benzimidazol-5-yl)benzimidazol-5-yl]-2-fluoro-N-methyl-benzamide

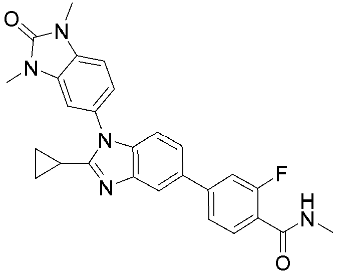

3'-Amino-4'-((1,3-dimethyl-2-oxo-2,3-dihydro-1H-benzo[d]imidazol-5-yl)amino)-3-fluoro-N-methyl-[1,1'-biphenyl]-4-carboxamide (Intermediate I-6, 30 mg, 0.072 mmol) and cyclopropanecarbaldehyde (5.01 mg, 0.072 mmol) were dissolved in ethanol (1 mL) and acetic acid (0.1 mL), and then copper acetate (6.50 mg, 0.036 mmol) was added. The reaction solution was stirred at 80°C for 30 minutes. After the reaction was completed, water and 1 N sodium hydroxide aqueous solution were added, and an organic layer was extracted with dichloromethane. The organic layer was washed with water and brine, dried over anhydrous magnesium sulfate and concentrated under reduced pressure, and the residue was purified by MPLC and PTLC to obtain the title compound. (22.7 mg, 0.048 mmol, 67.6% yield)

¹H-NMR (500 MHz, CHLOROFORM-d) δ 8.15 (t, J = 8.2 Hz, 1H), 7.92 (s, 1H), 7.51 (d, J = 8.2 Hz, 1H), 7.40 (d, J = 8.2 Hz, 1H), 7.35 (d, J = 13.7 Hz, 1H), 7.21 (d, J = 8.2 Hz, 1H), 7.15-7.13 (m, 2H), 7.08 (s, 1H), 6.81-6.77 (m, 1H), 3.51 (s, 3H), 3.46 (s, 3H), 3.04 (d, J = 4.9 Hz, 3H), 1.86-1.81 (m, 1H), 1.36-1.33 (m, 2H), 1.06-1.02 (m, 2H), LC/MS: 470 (M+H)

### Example 87 (Synthetic Method J): Preparation of 4-[1-(1,3-dimethyl-2-oxo-benzimidazol-5-yl)-2-isoxazol-5-yl-benzimidazol-5-yl]-2-fluoro-N-methyl-benzamide

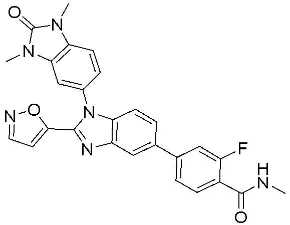

3'-Amino-4'-((1,3-dimethyl-2-oxo-2,3-dihydro-1H-benzo[d]imidazol-5-yl)amino)-3-fluoro-N-methyl-[1,1'-biphenyl]-4-carboxamide (Intermediate I-6, 27.8 mg, 0.066 mmol), isoxazole-5-carboxylic acid (8.24 mg, 0.073 mmol), and N,N-diisopropylethylamine (0.013 mL, 0.073 mmol) were dissolved in ethyl acetate (0.8 mL), and 2,4,6-tripropyl-1,3,5,2,4,6-triphosphinane 2,4,6-trioxide (50% wt in ethyl acetate, 0.197 mL, 0.331 mmol) was added. The reaction solution was stirred at 110°C for 30 minutes using a microwave. After the reaction was completed, the reaction solution was diluted with water, 1 N sodium hydroxide aqueous solution was added, and an organic layer was extracted with ethyl acetate. The organic layer was washed with water and brine, dried over anhydrous magnesium sulfate and concentrated under reduced pressure, and the residue was purified by MPLC and PTLC to obtain the title compound. (18.9 mg, 0.038 mmol, 57.4% yield)

¹H-NMR (500 MHz, CHLOROFORM-d) δ 8.24 (s, 1H), 8.18 (t, J = 8.2 Hz, 1H), 8.09 (s, 1H), 7.57-7.52 (m, 2H), 7.39-7.36 (m, 1H), 7.23-7.21 (m, 1H), 7.18-7.14 (m, 2H), 7.04 (s, 1H), 6.82-6.79 (m, 1H), 6.46 (s, 1H), 3.53 (s, 3H), 3.43 (s, 3H), 3.05 (d, J = 4.9 Hz, 3H), LC/MS 497 (M+H)

### Example 100 (Synthetic Method K): Preparation of 4-[1-(1,3-dimethyl-2-oxo-benzimidazol-5-yl)-2-(2-thienyl)benzimidazol-5-yl]-2-fluoro-N-methyl-benzamide

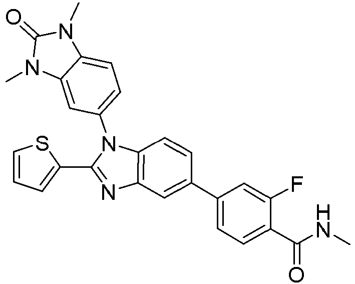

4'-((1,3-Dimethyl-2-oxo-2,3-dihydro-1H-benzo[d]imidazol-5-yl)amino)-3-fluoro-N-methyl-3'-nitro-[1,1'-biphenyl]-4-carboxamide (compound of Step 1 of Synthetic Method I-C, 20.3 mg, 0.045 mmol) was dissolved in dimethyl sulfoxide (0.5 mL), and then thiophene-2-carbaldehyde (5.07 mg, 0.045 mmol) and sodium hyposulfite (23.59 mg, 0.136 mmol) were added. The reaction solution was stirred at 90°C for 18 hours. After the reaction was completed, water and ammonium hydroxide aqueous solution were added, and an organic layer was extracted with ethyl acetate. The organic layer was washed with water and brine, dried over anhydrous magnesium sulfate and concentrated under reduced pressure, and the residue was purified by MPLC and PTLC to obtain the title compound. (13.6 mg, 0.027 mmol, 58.9% yield)

¹H-NMR (500 MHz, CHLOROFORM-d) δ 8.18 (t, J = 8.2 Hz, 1H), 8.06 (s, 1H), 7.54 (d, J = 8.2 Hz, 1H), 7.46 (d, J = 8.2 Hz, 1H), 7.39-7.36 (m, 2H), 7.20-7.16 (m, 2H), 7.10 (d, J = 8.5 Hz, 1H), 7.03 (s, 1H), 6.98 (d, J = 3.7 Hz, 1H), 6.94-6.93 (m, 1H), 6.81-6.78 (m, 1H), 3.54 (s, 3H), 3.43 (s, 3H), 3.06 (d, J = 4.6 Hz, 3H), LC/MS 512 (M+H)

### Example 124: Preparation of 4-[1-(1,3-dimethyl-2-oxo-benzimidazol-5-yl)-2-isopropyl-benzimidazol-5-yl]-2-fluoro-N-methyl-benzamide

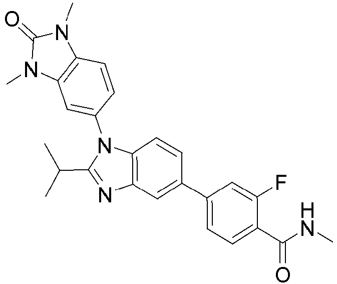

Methyl 2-[1-(1,3-dimethyl-2-oxo-benzimidazol-5-yl)-5-[3-fluoro-4-(methylcarbamoyl)phenyl]benzimidazol-2-yl]-2-methyl-propanoate (Example 123, 33 mg, 0.062 mmol) was dissolved in methanol (0.24 mL) and water (0.8 mL), and then lithium hydroxide hydrate (13.07 mg, 0.312 mmol) was added. The reaction solution was stirred at 45°C for 6 hours. After the reaction was completed, the reaction solution was neutralized with 1 N hydrochloric acid aqueous solution. Dichloromethane was added and an organic layer was extracted. The organic layer was washed with water and brine, dried over anhydrous magnesium sulfate and concentrated under reduced pressure, and the residue was purified by PTLC to obtain the title compound. (3.1 mg, 6.01 µmol, 9.65% yield)

¹H-NMR (500 MHz, CHLOROFORM-d) δ 8.18 (t, J = 8.4 Hz, 1H), 8.04 (s, 1H), 7.54 (d, J = 7.9 Hz, 1H), 7.44 (d, J = 8.2 Hz, 1H), 7.37 (d, J = 14.0 Hz, 1H), 7.16-7.09 (m, 3H), 6.97 (s, 1H), 6.80-6.76 (m, 1H), 3.53 (s, 3H), 3.46 (s, 3H), 3.12-3.08 (m, 1H), 3.06 (d, J = 4.6 Hz, 3H), 1.38 (d, J = 6.7 Hz, 6H), LC/MS: 472 (M+H)

### Example 128 (Synthetic Method L): Preparation of 4-[1-(1,3-dimethyl-2-oxo-benzimidazol-5-yl)-2-[2-methyl-3-(1-methylpyrazol-4-yl)phenyl]benzimidazol-5-yl]-2-fluoro-N-methyl-benzamide

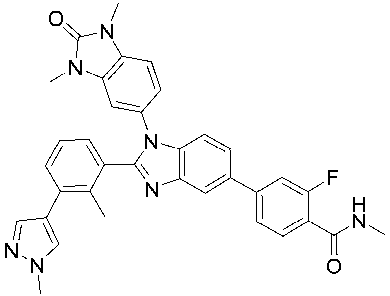

4-[2-(3-Bromo-2-methyl-phenyl)-1-(1,3-dimethyl-2-oxo-benzimidazol-5-yl)benzimidazol-5-yl]-2-fluoro-N-methyl-benzamide (Example 127, 30 mg, 0.050 mmol), 1-methyl-4-(4,4,5,5-tetramethyl-1,3,2-dioxaborolan-2-yl)-1H-pyrazole (12.52 mg, 0.060 mmol), and tripotassium phosphate (21.28 mg, 0.100 mmol) were dissolved in 1,4-dioxane (1 mL) and water (0.250 mL), and 1,1'-bis(diphenylphosphino)ferrocene]palladium(II) dichloride dichloromethane adduct (4.09 mg, 5.01 µmol) was added. The reaction solution was stirred under nitrogen conditions at 100°C for 18 hours. After the reaction was completed, water was added, and an organic layer was extracted with ethyl acetate. The organic layer was washed with water and brine, dried over anhydrous magnesium sulfate and concentrated under reduced pressure, and the residue was purified by MPLC and PTLC to obtain the title compound. (24 mg, 0.040 mmol, 80% yield)

¹H-NMR (500 MHz, CHLOROFORM-d) δ 8.17 (t, J = 8.2 Hz, 1H), 8.09 (s, 1H), 7.57-7.53 (m, 2H), 7.50 (s, 1H), 7.42 (s, 1H), 7.39 (s, 1H), 7.38 (s, 1H), 7.33 (d, J = 7.3 Hz, 1H), 7.25 (d, J = 8.2 Hz, 1H), 7.17-7.14 (m, 1H), 7.00-6.95 (m, 2H), 6.88-6.84 (m, 1H), 6.83 (s, 1H), 3.93 (s, 3H), 3.42 (s, 3H), 3.31 (s, 3H), 3.04 (d, J = 4.3 Hz, 3H), 2.22 (s, 3H), LC/MS: 600 (M+H)

### Example 132: Preparation of 4-[2-[3-[2-(dimethylamino)ethylamino]-2-methyl-phenyl]-1-(1,3-dimethyl-2-oxo-benzimidazol-5-yl)benzimidazol-5-yl]-2-fluoro-N-methyl-benzamide

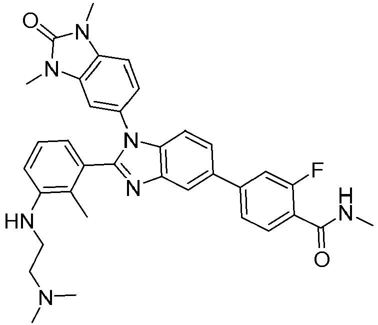

4-[2-(3-Bromo-2-methyl-phenyl)-1-(1,3-dimethyl-2-oxo-benzimidazol-5-yl)benzimidazol-5-yl]-2-fluoro-N-methyl-benzamide (Example 127, 52.3 mg, 0.044 mmol), N1,N1-dimethylethane-1,2-diamine (5.17 µl, 0.048 mmol), rac-[1,1'-binaphthalene]-2,2'-diylbis(diphenylphosphine) (27.2 mg, 0.044 mmol) and potassium tert-butoxide (9.81 mg, 0.087 mmol) were dissolved in toluene (0.8 mL), and tris(dibenzylideneacetone)dipalladium(0) (4.00 mg, 4.37 µmol) was added. The reaction solution was stirred under nitrogen conditions at 100°C for 48 hours. After the reaction was completed, water was added, and an organic layer was extracted with ethyl acetate. The organic layer was washed with water and brine, dried over anhydrous magnesium sulfate and concentrated under reduced pressure, and the residue was purified by MPLC and PTLC to obtain the title compound. (9.2 mg, 0.015 mmol, 34.8% yield)

¹H-NMR (500 MHz, CHLOROFORM-d) δ 8.19 (t, J = 8.2 Hz, 1H), 8.09 (s, 1H), 7.58 (d, J = 8.2 Hz, 1H), 7.53 (d, J = 8.2 Hz, 1H), 7.43-7.38 (m, 2H), 7.09-7.06 (m, 1H), 6.99 (d, J = 8.2 Hz, 1H), 6.94 (d, J = 8.2 Hz, 1H), 6.84-6.80 (m, 2H), 6.73 (d, J = 7.6 Hz, 1H), 6.62 (d, J = 8.2 Hz, 1H), 3.47 (s, 3H), 3.42 (s, 3H), 3.31 (s, 3H), 3.15 (t, J = 5.6 Hz, 2H), 3.06 (d, J = 4.3 Hz, 3H), 2.57 (t, J = 5.5 Hz, 2H), 2.22 (s, 6H), LC/MS: 606 (M+H)

### Example 142: Preparation of 4-[2-(3,5-dimethylimidazol-4-yl)-1-(1,3-dimethyl-2-oxo-benzimidazol-5-yl)benzimidazol-5-yl]-2-fluoro-N-methyl-benzamide

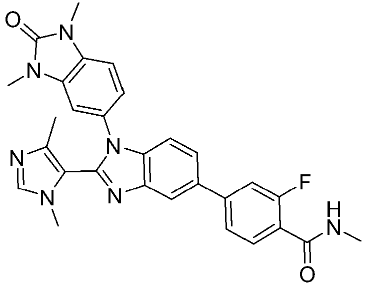

4-[1-(1,3-Dimethyl-2-oxo-benzimidazol-5-yl)-2-(4-fluorophenyl)benzimidazol-5-yl]-2-fluoro-N-methyl-benzamide (Example 141, 10 mg, 0.020 mmol) was dissolved in tetrahydrofuran (0.5 mL), and then 1,4-diazabicyclo[5,4,0]undec-7-ene (5.92 µl, 0.040 mmol) and methyl iodide (2.454 µl, 0.040 mmol) were added. The reaction solution was stirred at 50°C for 4 hours. After the reaction was completed, water was added and an organic layer was extracted with ethyl acetate. The organic layer was washed with a saturated ammonium chloride aqueous solution and brine, dried over anhydrous magnesium sulfate and concentrated under reduced pressure, and the residue was purified by PTLC to obtain the title compound. (1.4 mg, 2.67 µmol, 13.62% yield)

¹H-NMR (500 MHz, CHLOROFORM-d) δ 8.22 (t, J = 7.9 Hz, 1H), 8.12 (s, 1H), 7.59 (s, 1H), 7.57 (s, 1H), 7.48-7.42 (m, 3H), 7.05-7.02 (m, 1H), 6.97 (d, J = 8.2 Hz, 1H), 6.82 (s, 1H), 6.79-6.77 (m, 1H), 3.65 (s, 3H), 3.49 (s, 3H), 3.47 (s, 3H), 3.38 (s, 3H), 3.07 (s, 3H), LC/MS: 524 (M+H)

### Example 145 (Synthetic Method M): Preparation of 4-[1-(1,3-dimethyl-2-oxo-benzimidazol-5-yl)-2-(trifluoromethyl)benzimidazol-5-yl]-2-fluoro-N-methyl-benzamide

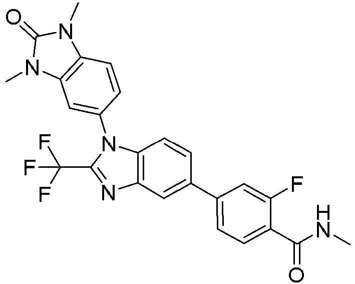

3'-Amino-4'-((1,3-dimethyl-2-oxo-2,3-dihydro-1H-benzo[d]imidazol-5-yl)amino)-3-fluoro-N-methyl-[1,1'-biphenyl]-4-carboxamide (Intermediate I-6, 60 mg, 0.054 mmol) was dissolved in trifluoroacetic acid (1 mL), and the reaction solution was stirred at 90°C for 18 hours. The reaction solution was stirred at 100°C for 3 hours. After the reaction was completed, the reaction solution was diluted with water, 1N sodium hydroxide aqueous solution was added at 0°C, and an organic layer was extracted with ethyl acetate. The organic layer was washed with water and brine, dried over anhydrous magnesium sulfate and concentrated under reduced pressure, and the residue was purified by MPLC to obtain the title compound. (19.2 mg, 0.039 mmol, 71.0% yield)

¹H-NMR (500 MHz, CHLOROFORM-d) δ 8.23-8.20 (m, 1H), 8.15 (s, 1H), 7.63 (d, J = 8.5 Hz, 1H), 7.54 (d, J = 8.5 Hz, 1H), 7.38 (d, J = 13.4 Hz, 1H), 7.20-7.13 (m, 3H), 7.04 (s, 1H), 6.80-6.77 (m, 1H), 3.53 (s, 3H), 3.46 (s, 3H), 3.07 (d, J = 3.0Hz, 3H), LC/MS: 498 (M+H)

### Example 152: Preparation of 4-[2-(3-cyano-2-methyl-phenyl)-1-(1,3-dimethyl-2-oxo-benzimidazol-5-yl)benzimidazol-5-yl]-2-fluoro-N-methyl-benzamide

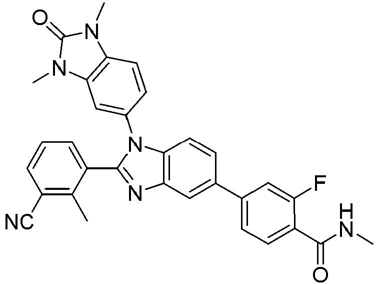

4-[2-(3-Bromo-2-methyl-phenyl)-1-(1,3-dimethyl-2-oxo-benzimidazol-5-yl)benzimidazol-5-yl]-2-fluoro-N-methyl-benzamide (Example 127, 12 mg, 0.020 mmol) was dissolved in anhydrous dimethylformamide (0.8 mL), and then dicyanozinc (23.54 mg, 0.20 mmol) and tetrakis(triphenylphosphine)palladium(0) (11.59 mg, 0.01 mmol) were added. The reaction solution was stirred at 130°C for 10 hours using a microwave. After the reaction was completed, the reaction solution was diluted with water, and an organic layer was extracted with ethyl acetate. The organic layer was washed with water and brine, dried over anhydrous magnesium sulfate and concentrated under reduced pressure, and the residue was purified by MPLC and PTLC to obtain the title compound. (4.8 mg, 8.81 µmol, 44.0% yield)

¹H-NMR (500 MHz, CHLOROFORM-d) δ 8.22 (t, J = 8.0 Hz, 1H), 8.12 (s, 1H), 7.67 (d, J = 17.7 Hz, 1H), 7.60-7.57 (m, 3H), 7.45-7.40 (m, 2H), 7.30 (d, J = 8.5 Hz, 1H), 6.98-6.97 (m, 1H), 6.92-6.90 (m, 1H), 6.81-6.78 (m, 2H), 3.46 (s, 3H), 3.36 (s, 3H), 3.07 (d, J = 5.5 Hz, 3H), 2.46 (s, 3H), LC/MS: 545 (M+H)

### Example 156: Preparation of 4-[1-(1,3-dimethyl-2-oxo-benzimidazol-5-yl)-2-(1-methoxy-1-methyl-ethyl)benzimidazol-5-yl]-2-fluoro-N-methyl-benzamide

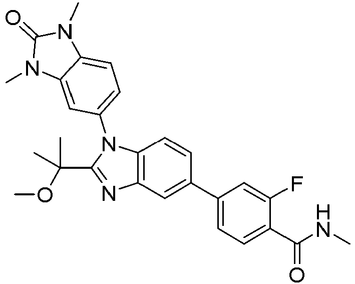

2-Methoxy-2-methylpropan-1-ol (26.6 mg, 0.256 mmol) was dissolved in ethyl acetate (1 mL) and dimethyl sulfoxide (0.5 mL), and then 2,4,6-tripropyl-1,3,5,2,4,6-trioxatriphosphinane 2,4,6-trioxide (50% wt in ethyl acetate, 0.277 mL, 0.465 mmol) was added at 0°C. The reaction solution was stirred at room temperature for 2 hours. After the oxidation of alcohol was completed, 3'-amino-4'-((1,3-dimethyl-2-oxo-2,3-dihydro-1H-benzo[d]imidazol-5-yl)amino)-3-fluoro-N-methyl-[1,1'-biphenyl]-4-carboxamide (Intermediate I-6, 195 mg, 0.232 mmol) was added and stirred for an additional 3 hours. After the reaction was completed, water and a saturated aqueous sodium bicarbonate solution were added, and an organic layer was extracted with ethyl acetate. The organic layer was washed with water and brine, dried over anhydrous magnesium sulfate and concentrated under reduced pressure, and the residue was purified by MPLC and PTLC to obtain the title compound. (4.8 mg, 9.57 µmol, 4.12% yield)

¹H-NMR (500 MHz, CHLOROFORM-d) δ 8.18 (t, J = 8.0 Hz, 1H), 8.07 (s, 1H), 7.54 (d, J = 8.0 Hz, 1H), 7.46 (d, J = 8.9 Hz, 1H), 7.37 (d, J = 13.7 Hz, 1H), 7.16-7.14 (m, 1H), 7.11-7.09 (m, 1H), 7.01 (m, 2H), 6.80-6.77 (m, 1H), 3.51 (s, 3H), 3.44 (s, 3H), 3.16 (s, 3H), 3.06 (d, J = 3.0 Hz, 3H), 1.65 (d, J = 21.1 Hz, 6H), LC/MS: 502 (M+H)

### Example 158: Preparation of N'-hydroxy-1',3'-dimethyl-2-(1-methyl-1H-imidazol-5-yl)-2'-oxo-2',3'-dihydro-1'H-[1,5'-bibenzo[d]imidazole]-5-carboximidamide

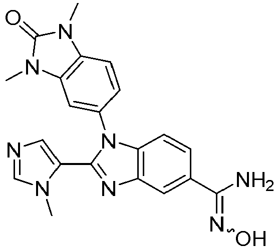

1',3'-Dimethyl-2-(1-methyl-1H-imidazol-5-yl)-2'-oxo-2',3'-dihydro-1'H-[1,5'-bibenzo[d]imidazole]-5-carbonitrile (Example 157, 100.0 mg, 0.2608 mmol) was dissolved in dimethylformamide (1 mL), and then 3 N hydroxylamine solution (1 mL) was added. The reaction solution was stirred at 50°C for 18 hours. After the reaction was completed, the reaction solution was concentrated under reduced pressure to remove the solvent, and the title compound was obtained without further purification. (97.0 mg, 0.2329 mmol, 89% yield)

1H NMR (500 MHz, DMSO-d6) δ 10.43 (s, 1H), 8.89 (s, 2H), 8.10 (s, 1H), 7.82 (s, 1H), 7.80 (s, 2H), 7.64 (dd, J = 8.7, 1.3 Hz, 1H), 7.43 (d, J = 1.9 Hz, 1H), 7.36 (d, J = 8.2 Hz, 1H), 7.18 (dd, J = 8.2, 2.0 Hz, 1H), 7.06 (d, J = 8.4 Hz, 1H), 6.36 (s, 1H), 4.05 (d, J = 3.6 Hz, 3H), 3.43 (s, 3H), 3.33 (s, 3H)

### Example 159: Preparation of 3-(1',3'-dimethyl-2-(1-methyl-1H-imidazol-5-yl)-2'-oxo-2',3'-dihydro-1'H-[1,5'-bibenzo[d]imidazol]-5-yl)-1,2,4-oxadiazol-5(2H)-one

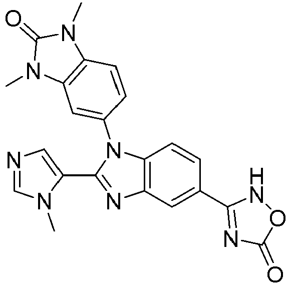

N'-Hydroxy-1',3'-dimethyl-2-(1-methyl-1H-imidazol-5-yl)-2'-oxo-2',3'-dihydro-1'H-[1,5'-bibenzo[d]imidazole]-5-carboximidamide (Example 158, 20.0 mg, 0.048 mmol) and 1,8-diamazaicyclo[5.4.0]undec-7-ene (0.022 mL, 0.1441 mmol) were dissolved in dimethylformamide (0.5 mL), and 1,1'-carbonyldiimidazole (16.0 mg, 0.096 mmol) was added. The reaction solution was stirred at 80°C for 18 hours. After the reaction was completed, the reaction solution was concentrated under reduced pressure to remove the solvent, and the residue was purified by prepHPLC to obtain the title compound. (2.5 mg, 5.651 µmol, 12% yield)

¹H NMR (500 MHz, DMSO-d₆) δ 12.99 (s, 1H), 8.25 (d, J = 1.0 Hz, 1H), 8.14 (s, 1H), 7.76 (dd, J = 8.5, 1.4 Hz, 1H), 7.48 (d, J = 1.7 Hz, 1H), 7.39 (d, J = 8.2 Hz, 1H), 7.28 (d, J = 8.6 Hz, 1H), 7.23 (dd, J = 8.2, 1.7 Hz, 1H), 6.55 (s, 1H), 4.10 (s, 3H), 3.43 (s, 3H), 3.33 (s, 3H)

### Example 163: Preparation of 5-(4,5-dihydrothiazol-2-yl)-1',3'-dimethyl-2-(1-methyl-1H-imidazol-5-yl)-1',3'-dihydro-2'H-[1,5'-bibenzo[d]imidazol]-2'-one

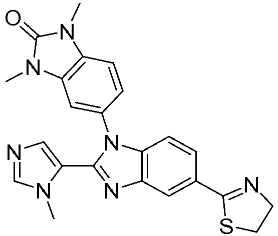

1',3'-Dimethyl-2-(1-methyl-1H-imidazol-5-yl)-2'-oxo-2',3'-dihydro-1'H-[1,5'-bibenzo[d]imidazole]-5-carbonitrile (Example 157, 38.0 mg, 0.09911 mmol) was dissolved in methanol (1 mL), and then 2-aminoethane-1-thiol (38.0 mg, 0.4956 mmol) was added. The reaction solution was stirred at 80°C for 18 hours. After the reaction was completed, the reaction solution was concentrated under reduced pressure to remove the solvent, and the residue was purified by MPLC to obtain the title compound. (0.0135 g, 0.03044 mmol, 31% yield)

¹H NMR (500 MHz, CHLOROFORM-d) δ 8.26 (d, J = 0.8 Hz, 1H), 7.86 (dd, J = 8.5, 1.4 Hz, 1H), 7.52 (s, 1H), 7.15 - 7.10 (m, 2H), 7.07 (d, J = 8.5 Hz, 1H), 6.95 (s, 1H), 6.44 (s, 1H), 4.49 (t, J = 8.2 Hz, 2H), 4.18 (s, 3H), 3.52 (s, 3H), 3.45 (t, J = 8.2 Hz, 2H), 3.41 (s, 3H)

### Example 164 (Synthetic Method O): Preparation of ethyl 2-(4-(1',3'-dimethyl-2-(1-methyl-1H-imidazol-5-yl)-2'-oxo-2',3'-dihydro-1'H-[1,5'-bibenzo[d]imidazol]-5-yl)-1H-1,2,3-triazol-1-yl)acetate

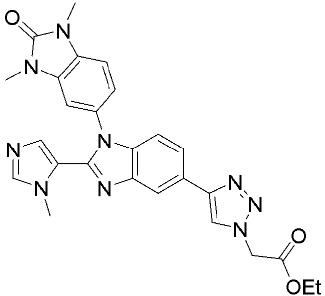

### Step 1) Preparation of 5-ethynyl-1',3'-dimethyl-2-(1-methyl-1H-imidazol-5-yl)-1',3'-dihydro-2'H-[1,5'-bibenzo[d]imidazol]-2'-one

The title compound was obtained in a similar manner to Synthetic Method I-B using ((4-fluoro-3-nitrophenyl)ethynyl)trimethylsilane (Intermediate I-7, 4.1 g, 17.4 mmol) and 5-amino-1,3-dimethyl-1,3-dihydro-2H-benzo[d]imidazol-2-one (Intermediate I-1, 3.08 g, 17.4 mmol). (13.5% yield over 3 steps)

¹H NMR (500 MHz, CHLOROFORM-d) δ 7.97 (s, 1H), 7.60 (s, 1H), 7.37 (dd, J = 8.3, 1.3 Hz, 1H), 7.12 (d, J = 8.2 Hz, 1H), 7.08 (dd, J = 8.1, 1.1 Hz, 1H), 7.00 (d, J = 8.3 Hz, 1H), 6.93 (s, 1H), 6.48 (s, 1H), 4.15 (s, 3H), 3.52 (s, 3H), 3.40 (s, 3H), 3.07 (s, 1H)

### Step 2) Preparation of ethyl 2-(4-(1',3'-dimethyl-2-(1-methyl-1H-imidazol-5-yl)-2'-oxo-2',3'-dihydro-1'H-[1,5'-bibenzo[d]imidazole]-5-yl)-1H-1,2,3-triazol-1-yl)acetate (Example 164)

The compound 5-ethynyl-1',3'-dimethyl-2-(1-methyl-1H-imidazol-5-yl)-1',3'-dihydro-2'H-[1,5'-bibenzo[d]imidazole]-2'-one (1 g, 2.6 mmol) obtained above, sodium ascorbate (0.051 g, 0.26 mmol) and copper iodide (0.098 g, 0.52 mmol) were dissolved in methanol (18 mL) and water (9 mL), and then ethyl azidoacetate (0.670 g, 5.2 mmol) was added. The reaction solution was stirred at room temperature for 18 hours. After the reaction was completed, the reaction solution was concentrated under reduced pressure and diluted with ethyl acetate. An organic layer was washed with water and brine, dried over anhydrous magnesium sulfate and concentrated under reduced pressure, and the residue was purified by MPLC to obtain the title compound. (0.5140 g, 1.005 mmol, 39% yield)

¹H NMR (500 MHz, CHLOROFORM-d) δ 8.23 (d, J = 0.9 Hz, 1H), 7.96 (s, 1H), 7.86 (dd, J = 8.4, 1.5 Hz, 1H), 7.57 (s, 1H), 7.14 (s, 1H), 7.13 (s, 3H), 6.97 (s, 1H), 6.49 (s, 1H), 5.24 (s, 2H), 4.31 (q, J = 7.1 Hz, 2H), 4.18 (s, 3H), 3.53 (s, 3H), 3.42 (s, 3H), 1.33 (t, J = 7.1 Hz, 3H)

### Example 165: Preparation of 2-(4-(1',3'-dimethyl-2-(1-methyl-1H-imidazol-5-yl)-2'-oxo-2',3'-dihydro-1'H-[1,5'-bibenzo[d]imidazol]-5-yl)-1H-1,2,3-triazol-1-yl)acetic acid

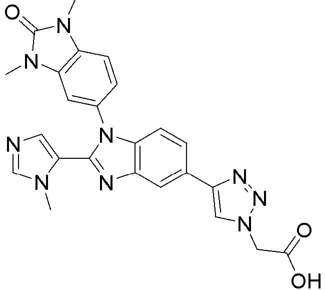

After dissolving ethyl 2-(4-(1',3'-dimethyl-2-(1-methyl-1H-imidazol-5-yl)-2'-oxo-2',3'-dihydro-1'H-[1,5'-bibenzo[d]imidazol]-5-yl)-1H-1,2,3-triazol-1-yl)acetate (Example 164, 0.510 g, 0.9970 mmol) in methanol/tetrahydrofuran (2:3), 1 M lithium hydroxide aqueous solution (2.991 mL, 2.991 mmol) was added. The reaction solution was stirred at room temperature for 5 hours. After the reaction was completed, the reaction solution was concentrated under reduced pressure, water was added, and an organic layer was extracted with dichloromethane. The organic layer was washed with water and brine, dried over anhydrous magnesium sulfate and concentrated under reduced pressure, and the title compound was obtained without further purification. (0.4387 g, 0.9073 mmol, 91% yield)

¹H NMR (500 MHz, Methanol-d₄) δ 8.33 (s, 1H), 8.26 (s, 1H), 7.81 (d, J = 8.4 Hz, 2H), 7.37 (d, J = 8.2 Hz, 1H), 7.35 (s, 1H), 7.27 - 7.19 (m, 2H), 6.61 (s, 1H), 5.04 (s, 2H), 4.06 (s, 3H), 3.52 (s, 3H), 3.42 (s, 3H)

### Example 167 (Synthetic Method P): Preparation of 2-(4-(1',3'-dimethyl-2-(1-methyl-1H-imidazol-5-yl)-2'-oxo-2',3'-dihydro-1'H-[1,5'-bibenzo[d]imidazol]-5-yl)-1H-1,2,3-triazol-1-yl)-N-methylacetamide

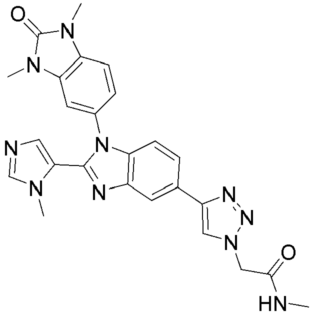

2-(4-(1',3'-Dimethyl-2-(1-methyl-1H-imidazol-5-yl)-2'-oxo-2',3'-dihydro-1'H-[1,5'-bibenzo[d]imidazole]-5-yl)-1H-1,2,3-triazol-1-yl)acetic acid (Example 165, 43.5 mg, 0.09 mmol) and methylamine (2 M in tetrahydrofuran, 0.0585 mL, 0.117 mmol) were dissolved in dimethylformamide (0.45 mL), and 1-ethyl-3-(3-dimethylaminopropyl)carbodiimide (0.0419 g, 0.27 mmol), 1-hydroxybenzotriazole hydrate (0.0365 g, 0.27 mmol), and N,N-diisopropylethylamine (0.047 mL, 0.27 mmol) were added. The reaction solution was stirred at room temperature for 3 hours. After the reaction was completed, the reaction solution was concentrated under reduced pressure, water was added, and an organic layer was extracted with dichloromethane. The organic layer was washed with water and brine, dried over anhydrous magnesium sulfate and concentrated under reduced pressure, and the residue was purified by MPLC to obtain the title compound. (5.8 mg, 0.0117 mmol, 13% yield)

¹H NMR (500 MHz, CHLOROFORM-d) δ 8.23 (d, J = 1.0 Hz, 1H), 7.94 (s, 1H), 7.82 (dd, J = 8.4, 1.5 Hz, 2H), 7.57 (s, 1H), 7.13 (dd, J = 12.6, 5.3 Hz, 2H), 6.95 (d, J = 1.7 Hz, 1H), 6.46 (d, J = 0.8 Hz, 1H), 6.14 (s, 1H), 5.12 (s, 2H), 4.18 (s, 3H), 3.54 (s, 3H), 3.41 (s, 3H), 2.85 (d, J = 4.9 Hz, 3H)

### Examples 2, 4-6, 9-12, 16-19, 21-29, 31-43, 45-57, 59-70, 72-83, 85, 86, 88-99, 101-123, 125-127, 129-131, 133-141, 143, 144, 146-151, 153-155, 157, 160-162, 166 and 168-176

Syntheses were performed using the same synthetic methods of the representative examples above.

Specifically, Examples 2, 4, 6, 10 and 11 were similarly prepared by Synthetic Method A, Examples 5, 12 and 16 were similarly prepared by Synthetic Method B, Examples 9, 17 to 19, 21 to 29, 31 to 43, 45, 47 to 57, 59 to 69, 73 to 83, 86, 160 to 162 and 172 to 176 were similarly prepared by Synthetic Method C, Example 157 was similarly prepared by Synthetic Method D, Examples 16, 93 and 154 were similarly prepared by Synthetic Method E, Example 70 was similarly prepared by Synthetic Method F, Example 72 was similarly prepared by Synthetic Method G, Example 46 was similarly prepared by Synthetic Method H, and Examples 85, 89 to 91, 94, 96, 97, 99, 101 to 103, 106 to 109, 112 and 113 were similarly prepared by Synthetic Method I, Examples 88, 92, 95, 98, 110, 111 and 129 were similarly prepared by Synthetic Method J, Examples 104, 105, 114 to 123, 125 to 127, 130, 133 to 141, 143, 144, 147 to 151, 153 and 155 were similarly prepared by Synthetic Method K, Example 131 was similarly prepared by Synthetic Method L, Example 146 was similarly prepared by Synthetic Method M, Example 166 was similarly prepared by Synthetic Method O, and Examples 168 to 171 were similarly prepared by Synthetic Method P.

The structure and name, NMR, LC/MS, and synthetic method of each of the compounds synthesized from the examples are described in Table 1 below.

**[Table 1]**

| **Example** | **Compound structure** | **Compound name** | **NMR / MS** |
|---|---|---|---|
| 1 | 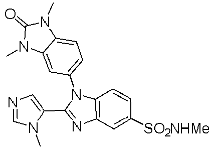 | 1-(1,3-dimethyl-2-oxo-benzimidazol-5-yl)-N-methyl-2-(3-methylimidazol-4-yl)benzimidazole-5-sulfonamide | ¹H-NMR (400 MHz, Methanol-d₄) δ 8.25 (d, J = 1.4 Hz, 1H), 7.82 (brs, 1H), 7.74 (dd, J = 8.5, 1.6 Hz, 1H), 7.38-7.35 (m, 2H), 7.31 (d, J = 8.2 Hz, 1H), 7.22 (dd, J = 8.2, 1.8 Hz, 1H), 6.56 (brs, 1H), 4.10 (s, 3H), 3.50 (s, 3H), 3.40 (s, 3H), 2.53 (s, 3H); m/z N.D. |
| 2 | 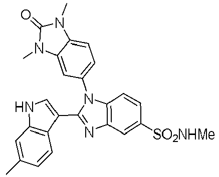 | 1-(1,3-dimethyl-2-oxo-benzimidazol-5-yl)-N-methyl-2-(6-methyl-1H-indol-3-yl)benzimidazole-5-sulfonamide | ¹H-NMR (500 MHz, Methanol-d₄) δ 8.24 (d, J = 1.5 Hz, 1H), 8.17 (d, J = 8.2 Hz, 1H), 7.71 (dd, J = 8.5, 1.5 Hz, 1H), 7.39 (d, J = 8.2 Hz, 1H), 7.35 (d, J = 2.1 Hz, 1H), 7.27-7.25 (m, 2H), 7.20 (s, 1H), 7.04 (d, J = 8.9 Hz, 1H), 6.77 (s, 1H), 3.53 (s, 3H), 3.40 (s, 3H), 2.56 (s, 3H), 2.45 (s, 3H); m/z N.D. |
| 3 | 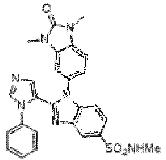 | 1-(1,3-dimethyl-2-oxo-benzimidazol-5-yl)-N-methyl-2-(3-phenylimidazol-4-yl)benzimidazole-5-sulfonamide | ¹H-NMR (500 MHz, CHLOROFORM-d) δ 7.85 (d, J = 8.5 Hz, 1H), 7.76 (dd, J = 8.2, 1.5 Hz, 1H), 7.68 (s, 1H), 7.63 (s, 1H), 7.23 (d, J = 7.6 Hz, 1H), 7.21 (s, 1H), 7.15-7.12 (m, 1H), 7.00 (d, J = 8.2 Hz, 1H), 6.89-6.83 (m, 3H), 6.79 (dd, J = 8.2, 1.8 Hz, 1H), 6.62 (d, J = 1.8 Hz, 1H), 4.38-4.35 (m, 1H), 3.51 (s, 3H), 3.37 (s, 3H), 2.62 (d, J = 5.2 Hz, 3H); m/z N.D. |
| 4 | 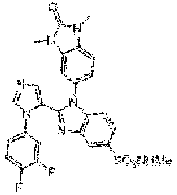 | 2-[3-(3,4-difluorophenyl)imida zol-4-yl]-1-(1,3-dimethyl-2-oxo-benzimidazol-5-yl)-N-methyl-benzimidazole-5-sulfonamide | ¹H -NMR (500 MHz, CHLOROFORM-d) δ 8.28 (s, 1H), 7.76 (d, J = 8.5 Hz, 1H), 7.64 (s, 1H), 7.22 (d, J = 8.2 Hz, 1H), 7.17-7.14 (m, 2H), 7.02 (d, J = 7.9 Hz, 1H), 6.87 (q, J = 7.6 Hz, 3H), 6.81 (d, J = 8.2 Hz, 1H), 6.63 (s, 1H), 4.39-4.36 (m, 1H), 3.51 (s, 3H), 3.37 (s, 3H), 2.68 (d, J = 5.5 Hz, 3H); m/z N.D. |
| 5 | 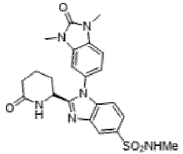 | 1-(1,3-dimethyl-2-oxo-benzimidazol-5-yl)-N-methyl-2-(6-oxo-2-piperidyl)benzimidaz ole-5-sulfonamide | ¹H -NMR (400 MHz, CHLOROFORM-d) δ 8.34 (s, 1H), 7.71 (d, J = 8.2 Hz, 1H), 7.17-7.10 (m, 3H), 6.97 (d, J = 8.7 Hz, 1H), 4.80 (s, 1H), 3.52 (s, 3H), 3.46 (s, 3H), 2.62 (s, 5H), 2.42 (m, 2H), 2.03 (m, 2H), 1.62-1.80 (m, 1H); m/z N.D. |
| 6 | 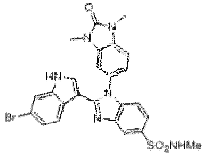 | 2-(6-bromo-1H-indol-3-yl)-1-(1,3-dimethyl-2-oxo-benzimidazol-5-yl)-N-methyl-benzimidazole-5-sulfonamide | ¹H -NMR (400 MHz, CHLOROFORM-d) δ 8.56 (d, J = 8.2 Hz, 1H), 8.37 (s, 1H), 8.34 (d, J = 0.9 Hz, 1H), 7.65 (dd, J = 8.5, 1.6 Hz, 1H), 7.51 (d, J = 1.4 Hz, 1H), 7.40 (dd, J = 8.5, 1.6 Hz, 1H), 7.21-7.16 (m, 2H), 7.07 (d, J = 8.2 Hz, 1H), 7.05 (d, J = 1.4 Hz, 1H), 6.51 (d, J = 2.7 Hz, 1H), 4.38 (q, J = 5.2 Hz, 1H), 3.55 (s, 3H), 3.43 (s, 3H), 2.69 (d, J = 5.0 Hz, 3H); m/z N.D. |
| 7 | 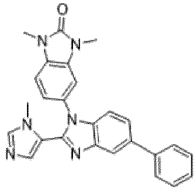 | 1,3-dimethyl-5-[2-(3-methylimidazol-4-yl)-5-phenyl-benzimidazol-1-yl]benzimidazol-2-one | ¹H-NMR (500 MHz, CHLOROFORM-d) δ 8.05 (s, 1H), 7.68-7.66 (m, 2H), 7.51-7.44 (m, 4H), 7.34 (t, J = 7.3 Hz, 1H), 7.13 (d, J = 8.2 Hz, 1H), 7.12 (s, 2H), 6.96 (s, 1H), 6.47 (s, 1H), 4.17 (s, 3H), 3.51 (s, 3H), 3.40 (s, 3H); m/z 434 |
| 8 | 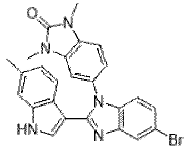 | 5-[5-bromo-2-(6-methyl-1H-indol-3-yl)benzimidazol-1-yl]-1,3-dimethyl-benzimidazol-2-one | ¹H-NMR (500 MHz, DMSO-d₆) δ 11.19 (s, 1H), 8.47 (d, 1H), 7.93 (s, 1H), 7.47 (m, 2H), 7.42 (d, 1H), 7.22 (m, 2H), 7.05 (d, 1H), 6.96 (m, 1H), 6.51 (d, 1H), 3.46 (s, 3H), 3.34 (s, 3H), 2.42 (s, 3H); m/z 486, 488 |
| 9 | 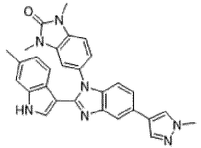 | 1,3-dimethyl-5-[2-(6-methyl-1H-indol-3-yl)-5-(1-methylpyrazol-4-yl)benzimidazol-1-yl]benzimidazol-2-one | ¹H-NMR (500 MHz, DMSO-d₆) δ 11.54 (s, 1H), 8.93 (d, 1H), 8.56 (s, 1H), 8.37 (d, 1H), 8.32 (s, 1H), 7.88 (m, 2H), 7.84 (d, 1H), 7.64 (t, 2H), 7.47 (m, 1H), 7.39 (d, 1H), 6.92 (d, 1H), 4.30 (s, 3H), 3.88 (s, 3H), 2.85 (s, 3H); m/z 488 |
| 10 | 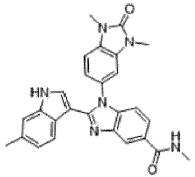 | 1-(1,3-dimethyl-2-oxo-benzimidazol-5-yl)-N-methyl-2-(6-methyl-1H-indol-3-yl)benzimidazole-5-carboxamide | ¹H-NMR (500 MHz, CHLOROFORM-d) δ 8.43 (d, J = 7.6 Hz, 2H), 8.23 (s, 1H), 7.76 (d, J = 8.5 Hz, 1H), 7.23-7.04 (m, 5H), 7.00 (s, 1H), 6.56 (s, 1H), 6.36 (d, J = 3.7 Hz, 1H), 3.53 (s, 3H), 3.39 (s, 3H), 3.08 (d, J = 4.9 Hz, 3H), 2.48 (s, 3H); m/z 465 |
| 11 | 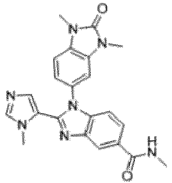 | 1-(1,3-dimethyl-2-oxo-benzimidazol-5-yl)-N-methyl-2-(3-methylimidazol-4-yl)benzimidazole-5-carboxamide | ¹H-NMR (500 MHz, CHLOROFORM-d) δ 8.23 (s, 1H), 7.76 (d, J = 8.5 Hz, 1H), 7.08-7.14 (m, 4H), 6.93 (s, 1H), 6.64 (s, 1H), 4.16 (s, 3H) 3.52 (s, 3H), 3.41 (s, 3H), 3.04 (d, J = 4.9 Hz, 3H); m/z 416 |
| 12 | 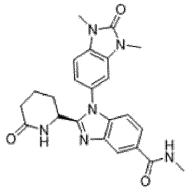 | 1-(1,3-dimethyl-2-oxo-benzimidazol-5-yl)-N-methyl-2-(6-oxo-2-piperidyl)benzimidaz ole-5-carboxamide | ¹H-NMR (500 MHz, CHLOROFORM-d) δ 8.18 (s, 1H), 7.86-7.75 (1H), 7.48-7.32 (1H), 7.16-6.98 (m, 5H), 6.77 (s, 1H), 4.89-4.70 (1H), 3.60-3.51 (s, 3H), 3.47 (s, 3H), 3.04 (d, J = 4.6 Hz, 3H), 2.36 (m, 2H), 2.02 (m, 3H), 1.74-1.56 (m, 1H); m/z 433 |
| 13 | 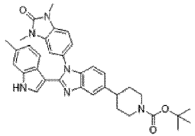 | tert-butyl 4-[1-(1,3-dimethyl-2-oxo-benzimidazol-5-yl)-2-(6-methyl-1H-indol-3-yl)benzimidazol-5-yl]piperidine-1-carboxylate | ¹H-NMR (500 MHz, DMSO-d₆) δ 11.10 (s, 1H), 8.48 (d, 1H), 7.58 (s, 1H), 7.46 (m, 2H), 7.19 (m, 2H), 7.03 (m, 2H), 6.92 (d, 1H), 6.49 (s, 1H), 4.12 (brs, 2H), 3.45 (s, 3H), 3.32 (s, 3H), 2.84 (m, 3H), 2.42 (s, 3H), 1.85 (m, 2H), 1.61 (m, 2H), 1.44 (s, 9H); m/z 591 |
| 14 | 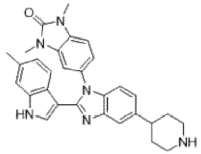 | 1,3-dimethyl-5-[2-(6-methyl-1H-indol-3-yl)-5-(4-piperidyl)benzimidaz ol-1-yl]benzimidazol-2-one | ¹H-NMR (500 MHz, DMSO-d₆) δ 11.11 (d, 1H), 8.48 (d, 1H), 7.57 (s, 1H), 7.42 (m, 2H), 7.20 (m, 2H), 7.05 (m, 2H), 6.93 (d, 1H), 6.50 (d, 1H), 3.45 (s, 3H), 3.34 (s, 3H), 3.19 (m, 3H), 2.80 (m, 3H), 2.42 (s, 3H), 1.86 (m, 2H), 1.72 (m, 2H), 1.44 (s, 9H); m/z 491 |
| 15 | 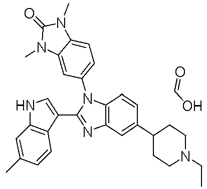 | 5-(1-ethylpiperidin-4-yl)-1',3'-dimethyl-2-(6-methyl-1H-indol-3-yl)-1',3'-dihydro-2'H-[1,5'-bibenzo[d]imidazol]-2'-one formate | ¹H-NMR (500 MHz, DMSO-d₆) δ 11.11 (d, 1H), 8.48 (d, 1H), 8.20 (s, 1H), 7.59 (s, 1H), 7.42 (m, 2H), 7.20 (m, 2H), 7.07 (m, 2H), 6.92 (d, 1H), 6.49 (d, 1H), 3.45 (s, 3H), 3.36 (s, 3H), 3.14 (m, 4H), 2.68 (m, 1H), 2.42 (s, 3H), 2.24 (m 2H), 1.88 (m, 4H), 1.11 (t, 3H); m/z 519 |
| 16 | 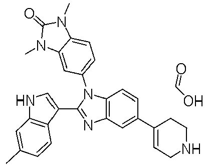 | 1',3'-dimethyl-2-(6-methyl-1H-indol-3-yl)-5-(1,2,3,6-tetrahydropyridin-4-yl)-1',3'-dihydro-2'H-[1,5'-bibenzo[d]imidazol]-2'-one formate | ¹H-NMR (500 MHz, DMSO-d₆) δ 11.16 (d, 1H), 8.50 (d, 1H), 8.26 (s, 1H), 7.82 (s, 1H), 7.45 (m, 2H), 7.31 (d, 1H), 7.21 (m, 2H), 7.05 (d, 1H), 6.98 (d, 1H), 6.50 (d, 1H), 6.21 (d, 1H), 3.67 (brs, 2H), 3.46 (s, 3H), 3.35 (s, 3H), 3.26 (m, 2H), 2.70 (brs, 2H), 2.42 (s, 3H); m/z 489 |
| 17 | 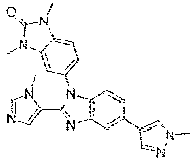 | 1,3-dimethyl-5-[2-(3-methylimidazol-4-yl)-5-(1-methylpyrazol-4-yl)benzimidazol-1-yl]benzimidazol-2-one | ¹H-NMR (500 MHz, CHLOROFORM-d) δ 7.92 (s, 1H), 7.81 (s, 1H), 7.64 (s, 1H), 7.52 (s, 1H), 7.37 (d, J = 8.5 Hz, 1H), 7.11 (s, 2H), 7.05 (d, J = 8.5 Hz, 1H), 6.94 (s, 1H), 6.45 (s, 1H), 4.16 (s, 3H), 3.96 (s, 3H), 3.51 (s, 3H), 3.40 (s, 3H); m/z 439 |
| 18 | 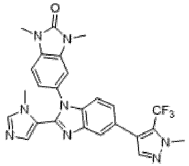 | 1,3-dimethyl-5-[2-(3-methylimidazol-4-yl)-5-[1-methyl-5-(trifluoromethyl)pyra zol-4-yl]benzimidazol-1-yl]benzimidazol-2-one | ¹H-NMR (500 MHz, Methanol-d₄) δ 7.78 (s, 1H), 7.75 (s, 1H), 7.60 (s, 1H), 7.34 (d, J = 8.2 Hz, 1H), 7.31 (s, 1H), 7.27 (d, J = 8.5 Hz, 1H), 7.21-7.18 (m, 2H), 6.58 (s, 1H), 4.07 (s, 3H), 4.04 (s, 3H), 3.49 (s, 3H), 3.39 (s, 3H); m/z N.D. |
| 19 | 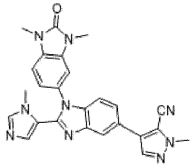 | 4-[1-(1,3-dimethyl-2-oxo-benzimidazol-5-yl)-2-(3-methylimidazol-4-yl)benzimidazol-5-yl]-2-methyl-pyrazole-3-carbonitrile | ¹H-NMR (500 MHz, Methanol-d₄) δ 8.10 (s, 1H), 7.94 (s, 1H), 7.76 (s, 1H), 7.60 (dd, J = 8.5, 1.5 Hz, 1H), 7.34 (d, J = 7.9 Hz, 1H), 7.28 (d, J = 1.5 Hz, 1H), 7.24 (d, J = 8.2 Hz, 1H), 7.20 (dd, J = 8.2, 1.8 Hz, 1H), 6.57 (s, 1H), 4.10 (s, 3H), 4.07 (s, 3H), 3.51 (s, 3H), 3.41 (s, 3H); m/z N.D. |
| 20 | 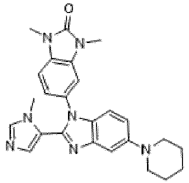 | 1,3-dimethyl-5-[2-(3-methylimidazol-4-yl)-5-(1-piperidyl)benzimidaz ol-1-yl]benzimidazol-2-one | ¹H-NMR (500 MHz, Methanol-d₄) δ 7.76 (s, 1H), 7.35 (s, 1H), 7.32 (d, J = 8.2 Hz, 1H), 7.27 (s, 1H), 7.17-7.09 (m, 3H), 6.61 (s, 1H), 3.97 (s, 3H), 3.50 (s, 3H), 3.40 (s, 3H), 3.17-3.15 (m, 4H), 1.82-1.77 (m, 4H), 1.64-1.59 (m, 3H); m/z N.D. |
| 21 | 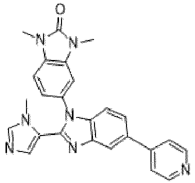 | 1,3-dimethyl-5-[2-(3-methylimidazol-4-yl)-5-(4-pyridyl)benzimidazol -1-yl]benzimidazol-2-one | ¹H-NMR (500 MHz, CHLOROFORM-d) δ 8.65 (s, 2H), 8.10 (s, 1H), 7.58-7.52 (m, 4H), 7.17 (d, J = 14.6 Hz, 1H), 7.13-7.11 (m, 2H), 6.96 (s, 1H), 6.47 (s, 1H), 4.18 (s, 3H), 3.51 (s, 3H), 3.40 (s, 3H); m/z 436 |
| 22 | 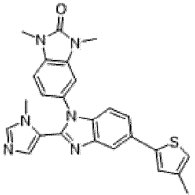 | 1,3-dimethyl-5-[2-(3-methylimidazol-4-yl)-5-(4-methyl-2-thienyl)benzimidazol -1-yl]benzimidazol-2-one | ¹H-NMR (500 MHz, CHLOROFORM-d) δ 8.03 (d, J = 0.9 Hz, 1H), 7.51 (s, 1H), 7.49 (dd, J = 8.4, 1.7 Hz, 1H), 7.15 (d, J = 0.9 Hz, 1H), 7.13-7.09 (m, 2H), 7.04 (d, J = 8.5 Hz, 1H), 6.95 (s, 1H), 6.84 (s, 1H), 6.44 (s, 1H), 4.16 (s, 3H), 3.51 (s, 3H), 3.40 (s, 3H), 2.30 (s, 3H); m/z 455 |
| 23 | 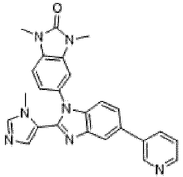 | 1,3-dimethyl-5-[2-(3-methylimidazol-4-yl)-5-(3-pyridyl)benzimidazol -1-yl]benzimidazol-2-one | ¹H-NMR (500 MHz, CHLOROFORM-d) δ 8.92 (s, 1H), 8.58 (s, 1H), 8.03 (s, 1H), 7.94 (d, J = 7.6 Hz, 1H), 7.52 (s, 1H), 7.46 (d, J = 8.2 Hz, 1H), 7.38-7.36 (m, 1H), 7.16 (d, J = 8.2 Hz, 1H), 7.13 (s, 2H), 6.96 (s, 1H), 6.47 (s, 1H), 4.17 (s, 3H), 3.51 (s, 3H), 3.40 (s, 3H); m/z N.D. |
| 24 | 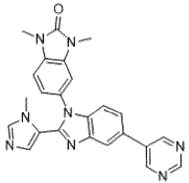 | 1,3-dimethyl-5-[2-(3-methylimidazol-4-yl)-5-pyrimidin-5-yl-benzimidazol-1-yl]benzimidazol-2-one | ¹H-NMR (500 MHz, CHLOROFORM-d) δ 9.20 (s, 1H), 9.02 (s, 2H), 8.03 (d, J = 0.9 Hz, 1H), 7.53 (s, 1H), 7.46-7.44 (m, 1H), 7.20 (d, J = 8.5 Hz, 1H), 7.16-7.12 (m, 2H), 6.96 (s, 1H), 6.47 (s, 1H), 4.19 (s, 3H), 3.52 (s, 3H), 3.41 (s, 3H); m/z N.D. |
| 25 | 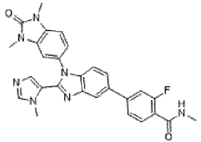 | 4-[1-(1,3-dimethyl-2-oxo-benzimidazol-5-yl)-2-(3-methylimidazol-4-yl)benzimidazol-5-yl]-2-fluoro-N-methyl-benzamide | ¹H-NMR (500 MHz, CHLOROFORM-d) δ 8.15 (t, J = 8.2 Hz, 1H), 8.04 (s, 1H), 7.54 (dd, J = 8.1, 1.4 Hz, 1H), 7.52 (s, 1H), 7.47 (dd, J = 8.4, 1.4 Hz, 1H), 7.39 (d, J = 13.4 Hz, 1H), 7.13-7.09 (m, 3H), 6.95 (s, 1H), 6.85-6.81 (m, 1H), 6.45 (s, 1H), 4.16 (s, 3H), 3.51 (s, 3H), 3.39 (s, 3H), 3.03 (d, J = 4.9 Hz, 3H); m/z N.D. |
| 26 | 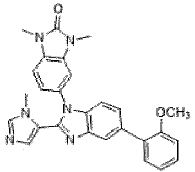 | 5-[5-(2-methoxyphenyl)-2-(3-methylimidazol-4-yl)benzimidazol-1-yl]-1,3-dimethyl-benzimidazol-2-one | ¹H-NMR (500 MHz, CHLOROFORM-d) δ 7.98 (s, 1H), 7.51 (s, 1H), 7.44 (d, J = 8.2 Hz, 1H), 7.40 (d, J = 7.3 Hz, 1H), 7.34 (t, J = 7.6 Hz, 1H), 7.11 (m, 3H), 7.06 (t, J = 7.5 Hz, 1H), 7.02 (d, J = 8.2 Hz, 1H), 6.96 (s, 1H), 6.48 (s, 1H), 4.15 (s, 3H), 3.83 (s, 3H), 3.51 (s, 3H), 3.40 (s, 3H); m/z 465 |
| 27 | 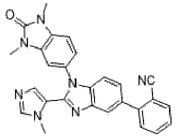 | 2-[1-(1,3-dimethyl-2-oxo-benzimidazol-5-yl)-2-(3-methylimidazol-4-yl)benzimidazol-5-yl]benzonitrile | ¹H-NMR (500 MHz, CHLOROFORM-d) δ 7.98 (s, 1H), 7.78 (d, J = 7.6 Hz, 1H), 7.68-7.65 (m, 1H), 7.59 (d, J = 7.9 Hz, 1H), 7.52 (s, 1H), 7.46-7.43 (m, 2H), 7.17 (d, J = 8.5 Hz, 1H), 7.13 (s, 2H), 6.98 (s, 1H), 6.47 (s, 1H), 4.17 (s, 3H), 3.52 (s, 3H), 3.41 (s, 3H); m/z 460 |
| 28 | 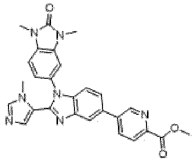 | methyl 5-[1-(1,3-dimethyl-2-oxo-benzimidazol-5-yl)-2-(3-methylimidazol-4-yl)benzimidazol-5-yl]pyridine-2-carboxylate | ¹H-NMR (500 MHz, CHLOROFORM-d) δ 9.06 (s, 1H), 8.24 (d, J = 7.9 Hz, 1H), 8.11-8.10 (m, 2H), 7.55 (s, 1H), 7.52 (d, J = 7.0 Hz, 1H), 7.20 (d, J = 8.5 Hz, 1H), 7.16-7.12 (m, 2H), 6.97 (s, 1H), 6.49 (s, 1H), 4.20 (s, 3H), 4.05 (s, 3H), 3.54 (s, 3H), 3.42 (s, 3H); m/z N.D. |
| 29 | 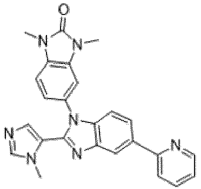 | 1,3-dimethyl-5-[2-(3-methylimidazol-4-yl)-5-(2-pyridyl)benzimidazol -1-yl]benzimidazol-2-one | ¹H-NMR (500 MHz, CHLOROFORM-d) δ 8.71 (d, J = 4.6 Hz, 1H), 8.41 (s, 1H), 8.02 (dd, J = 8.4, 1.5 Hz, 1H), 7.84-7.83 (m, 1H), 7.78 (td, J = 7.8, 1.5 Hz, 1H), 7.54 (s, 1H), 7.24-7.22 (m, 1H), 7.17 (d, J = 8.2 Hz, 1H), 7.14 (s, 2H), 6.98 (s, 1H), 6.48 (s, 1H), 4.19 (s, 3H), 3.53 (s, 3H), 3.42 (s, 3H); m/z N.D. |
| 30 | 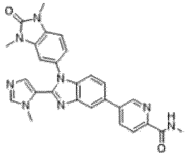 | 5-[1-(1,3-dimethyl-2-oxo-benzimidazol-5-yl)-2-(3-methylimidazol-4-yl)benzimidazol-5-yl]-N-methyl-pyridine-2-carboxamide | ¹H-NMR (500 MHz, CHLOROFORM-d) δ 8.84 (d, J = 1.8 Hz, 1H), 8.28 (d, J = 8.2 Hz, 1H), 8.10-8.07 (m, 2H), 8.04-8.01 (m, 1H), 7.54 (s, 1H), 7.50 (dd, J = 8.5, 1.5 Hz, 1H), 7.19 (d, J = 8.5 Hz, 1H), 7.16-7.09 (m, 2H), 6.97 (s, 1H), 6.47 (s, 1H), 4.19 (s, 3H), 3.53 (s, 3H), 3.42 (s, 3H), 3.07 (d, J = 5.2 Hz, 3H); m/z N.D. |
| 31 | 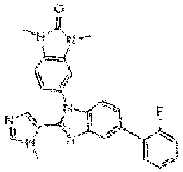 | 5-[5-(2-fluorophenyl)-2-(3-methylimidazol-4-yl)benzimidazol-1-yl]-1,3-dimethyl-benzimidazol-2-one | ¹H-NMR (500 MHz, CHLOROFORM-d) δ 8.01 (s, 1H), 7.54-7.51 (m, 2H), 7.46 (d, J = 8.2 Hz, 1H), 7.35-7.31 (m, 1H), 7.26-7.24 (m, 1H), 7.20-7.13 (m, 4H), 6.97 (s, 1H), 6.48 (s, 1H), 4.18 (s, 3H), 3.52 (s, 3H), 3.41 (s, 3H); m/z 453 |
| 32 | 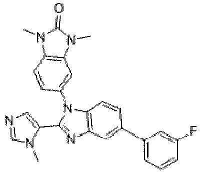 | 5-[5-(3-fluorophenyl)-2-(3-methylimidazol-4-yl)benzimidazol-1-yl]-1,3-dimethyl-benzimidazol-2-one | ¹H-NMR (500 MHz, CHLOROFORM-d) δ 8.03 (s, 1H), 7.54 (s, 1H), 7.48-7.39 (m, 3H), 7.36 (d, J = 10.1 Hz, 1H), 7.15-7.13 (m, 3H), 7.03 (t, J = 7.5 Hz, 1H), 6.96 (s, 1H), 6.48 (s, 1H), 4.18 (s, 3H), 3.52 (s, 3H), 3.41 (s, 3H); m/z 453 |
| 33 | 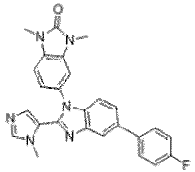 | 5-[5-(4-fluorophenyl)-2-(3-methylimidazol-4-yl)benzimidazol-1-yl]-1,3-dimethyl-benzimidazol-2-one | ¹H-NMR (500 MHz, CHLOROFORM-d) δ 7.99 (s, 1H), 7.61 (dd, J = 8.5, 5.2 Hz, 2H), 7.52 (s, 1H), 7.44 (d, J = 8.1 Hz, 1H), 7.16-7.12 (m, 5H), 6.96 (s, 1H), 6.47 (s, 1H), 4.18 (s, 3H), 3.52 (s, 3H), 3.41 (s, 3H); m/z 453 |
| 34 | 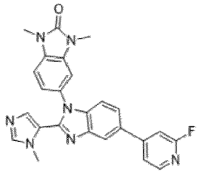 | 5-[5-(2-fluoro-4-pyridyl)-2-(3-methylimidazol-4-yl)benzimidazol-1-yl]-1,3-dimethyl-benzimidazol-2-one | ¹H-NMR (500 MHz, CHLOROFORM-d) δ 8.26 (d, J = 5.5 Hz, 1H), 8.11 (s, 1H), 7.54 (s, 1H), 7.52 (dd, J = 8.4, 1.7 Hz, 1H), 7.50-7.47 (m, 1H), 7.20-7.13 (m, 4H), 6.97 (s, 1H), 6.47 (s, 1H), 4.19 (s, 3H), 3.53 (s, 3H), 3.41 (s, 3H); m/z 454 |
| 35 | 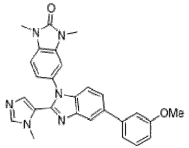 | 5-[5-(3-methoxyphenyl)-2-(3-methylimidazol-4-yl)benzimidazol-1-yl]-1,3-dimethyl-benzimidazol-2-one | ¹H-NMR (500 MHz, CHLOROFORM-d) δ 8.05 (s, 1H), 7.53-7.50 (m, 2H), 7.40-7.37 (m, 1H), 7.27 (s, 1H), 7.21 (s, 1H), 7.13 (s, 3H), 6.97 (s, 1H), 6.91 (d, J = 7.3 Hz, 1H), 6.47 (s, 1H), 4.18 (s, 3H), 3.89 (s, 3H), 3.53 (s, 3H), 3.41 (s, 3H); m/z N.D. |
| 36 | 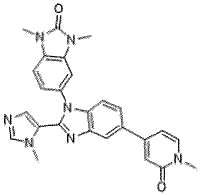 | 1,3-dimethyl-5-[2-(3-methylimidazol-4-yl)-5-(1-methyl-2-oxo-4-pyridyl)benzimidazol -1-yl]benzimidazol-2-one | ¹H-NMR (500 MHz, CHLOROFORM-d) δ 8.04 (s, 1H), 7.53 (s, 1H), 7.47 (dd, J = 8.2, 1.5 Hz, 1H), 7.36 (d, J = 7.0 Hz, 1H), 7.14-7.09 (m, 3H), 6.94 (d, J = 1.2 Hz, 1H), 6.87 (d, J = 1.5 Hz, 1H), 6.54 (dd, J = 7.2, 2.0 Hz, 1H), 6.46 (s, 1H), 4.17 (s, 3H), 3.59 (s, 3H), 3.52 (s, 3H), 3.40 (s, 3H); m/z N.D. |
| 37 | 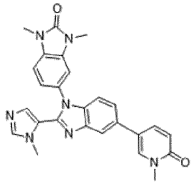 | 1,3-dimethyl-5-[2-(3-methylimidazol-4-yl)-5-(1-methyl-6-oxo-3-pyridyl)benzimidazol -1-yl]benzimidazol-2-one | ¹H-NMR (500 MHz, CHLOROFORM-d) δ 7.84 (s, 1H), 7.71-7.69 (m, 1H), 7.55-7.53 (m, 2H), 7.28 (d, J = 8.2 Hz, 1H), 7.14-7.09 (m, 3H), 6.95 (s, 1H), 6.69 (d, J = 9.2 Hz, 1H), 6.46 (s, 1H), 4.16 (s, 3H), 3.64 (s, 3H), 3.52 (s, 3H), 3.40 (s, 3H); m/z N.D. |
| 38 | 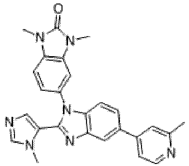 | 1,3-dimethyl-5-[2-(3-methylimidazol-4-yl)-5-(2-methyl-4-pyridyl)benzimidazol -1-yl]benzimidazol-2-one | ¹H-NMR (500 MHz, CHLOROFORM-d) δ 8.55 (s, 1H), 8.10 (s, 1H), 7.54-7.40 (m, 4H), 7.17-7.14 (m, 3H), 6.96 (s, 1H), 6.47 (s, 1H), 4.19 (s, 3H), 3.53 (s, 3H), 3.41 (s, 3H), 2.64 (s, 3H); m/z N.D. |
| 39 | 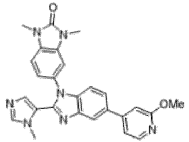 | 5-[5-(2-methoxy-4-pyridyl)-2-(3-methylimidazol-4-yl)benzimidazol-1-yl]-1,3-dimethyl-benzimidazol-2-one | ¹H-NMR (500 MHz, CHLOROFORM-d) δ 8.22 (d, J = 5.2 Hz, 1H), 8.08 (s, 1H), 7.52-7.50 (m, 2H), 7.19 (d, J = 5.2 Hz, 1H), 7.15-7.09 (m, 3H), 7.03 (s, 1H), 6.95 (s, 1H), 6.46 (s, 1H), 4.18 (s, 3H), 3.99 (s, 3H), 3.52 (s, 3H), 3.40 (s, 3H); m/z N.D. |
| 40 | 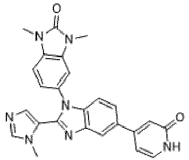 | 1,3-dimethyl-5-[2-(3-methylimidazol-4-yl)-5-(2-oxo-1H-pyridin-4-yl)benzimidazol-1-yl]benzimidazol-2-one | ¹H-NMR (500 MHz, CHLOROFORM-d) δ 13.20 (s, 1H), 8.07 (s, 1H), 7.54-7.48 (m, 3H), 7.16-7.11 (m, 3H), 6.95 (s, 1H), 6.88 (s, 1H), 6.67 (d, J = 6.7 Hz, 1H), 6.47 (s, 1H), 4.18 (s, 3H), 3.53 (s, 3H), 3.41 (s, 3H); m/z N.D. |
| 41 | 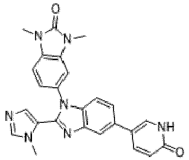 | 1,3-dimethyl-5-[2-(3-methylimidazol-4-yl)-5-(6-oxo-1H-pyridin-3-yl)benzimidazol-1-yl]benzimidazol-2-one | ¹H-NMR (500 MHz, CHLOROFORM-d) δ 13.50 (s, 1H), 7.86 (s, 2H), 7.68 (s, 1H), 7.53 (s, 1H), 7.30 (d, J = 8.5 Hz, 1H), 7.13 (s, 3H), 6.95 (s, 1H), 6.73 (d, J = 9.5 Hz, 1H), 6.47 (s, 1H), 4.17 (s, 3H), 3.52 (s, 3H), 3.41 (s, 3H); m/z N.D. |
| 42 | 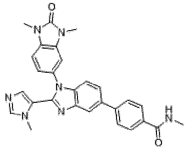 | 4-[1-(1,3-dimethyl-2-oxo-benzimidazol-5-yl)-2-(3-methylimidazol-4-yl)benzimidazol-5-yl]-N-methyl-benzamide | ¹H-NMR (500 MHz, CHLOROFORM-d) δ 8.06 (s, 1H), 7.86 (d, J = 8.1 Hz, 2H), 7.72 (d, J = 8.2 Hz, 2H), 7.53 (s, 1H), 7.51 (d, J = 8.5 Hz, 1H), 7.15-7.13 (m, 3H), 6.96 (s, 1H), 6.47 (s, 1H), 6.31 (d, J = 4.6 Hz, 1H), 4.18 (s, 3H), 3.52 (s, 3H), 3.41 (s, 3H), 3.04 (d, J = 4.9 Hz, 3H); m/z 492 |
| 43 | 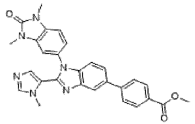 | methyl 4-[1-(1,3-dimethyl-2-oxo-benzimidazol-5-yl)-2-(3-methylimidazol-4-yl)benzimidazol-5-yl]benzoate | ¹H-NMR (500 MHz, CHLOROFORM-d) δ 8.12 (d, J = 8.2 Hz, 2H), 8.08 (s, 1H), 7.73 (d, J = 8.2 Hz, 2H), 7.54-7.52 (m, 2H), 7.16-7.13 (m, 3H), 6.96 (s, 1H), 6.46 (s, 1H), 4.18 (s, 3H), 3.94 (s, 3H), 3.52 (s, 3H), 3.40 (s, 3H); m/z N.D. |
| 44 | 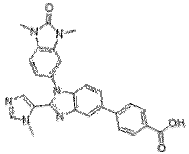 | 4-[1-(1,3-dimethyl-2-oxo-benzimidazol-5-yl)-2-(3-methylimidazol-4-yl)benzimidazol-5-yl]benzoic acid | N.D.; m/z 479 |
| 45 | 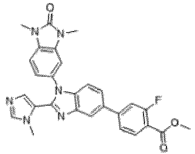 | methyl 4-[1-(1,3-dimethyl-2-oxo-benzimidazol-5-yl)-2-(3-methylimidazol-4-yl)benzimidazol-5-yl]-2-fluoro-benzoate | ¹H-NMR (400 MHz, CHLOROFORM-d) d 8.07 (s, 1H), 8.03 (t, J = 7.8 Hz, 1H), 7.54-7.49 (m, 3H), 7.44 (dd, J = 11.9, 1.4 Hz, 1H), 7.16-7.11 (m, 3H), 6.97 (s, 1H), 6.47 (s, 1H), 4.19 (s, 3H), 3.96 (s, 3H), 3.53 (s, 3H), 3.42 (s, 3H); m/z N.D. |
| 46 | 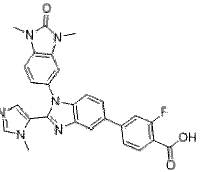 | 4-[1-(1,3-dimethyl-2-oxo-benzimidazol-5-yl)-2-(3-methylimidazol-4-yl)benzimidazol-5-yl]-2-fluoro-benzoic acid | N.D.; m/z 497 |
| 47 | 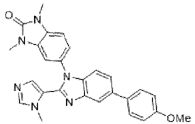 | 5-[5-(4-methoxyphenyl)-2-(3-methylimidazol-4-yl)benzimidazol-1-yl]-1,3-dimethyl-benzimidazol-2-one | ¹H-NMR (500 MHz, CHLOROFORM-d) δ 7.99 (s, 1H), 7.60 (s, 1H), 7.59 (s, 1H), 7.51 (s, 1H), 7.46 (d, J = 8.5 Hz, 1H), 7.12-7.10 (m, 3H), 7.00 (d, J = 8.5 Hz, 2H), 6.96 (s, 1H), 6.46 (s, 1H), 4.17 (s, 3H), 3.86 (s, 3H), 3.52 (s, 3H), 3.40 (s, 3H); m/z N.D. |
| 48 | 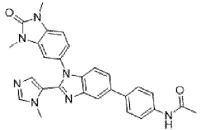 | N-[4-[1-(1,3-dimethyl-2-oxo-benzimidazol-5-yl)-2-(3-methylimidazol-4-yl)benzimidazol-5-yl]phenyl]acetamide | ¹H-NMR (500 MHz, CHLOROFORM-d) δ 8.00 (s, 1H), 7.84 (s, 1H), 7.60 (s, 4H), 7.52 (s, 1H), 7.46 (d, J = 8.2 Hz, 1H), 7.12-7.10 (m, 3H), 6.96 (s, 1H), 6.46 (s, 1H), 4.16 (s, 3H), 3.51 (s, 3H), 3.40 (s, 3H), 2.19 (s, 3H); m/z N.D. |
| 49 | 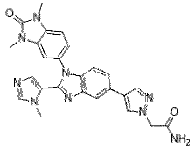 | 2-[4-[1-(1,3-dimethyl-2-oxo-benzimidazol-5-yl)-2-(3-methylimidazol-4-yl)benzimidazol-5-yl]pyrazol-1-yl]acetamide | ¹H-NMR (500 MHz, CHLOROFORM-d) δ 7.95 (s, 1H), 7.93 (s, 1H), 7.76 (s, 1H), 7.52 (s, 1H), 7.37 (d, J = 8.2 Hz, 1H), 7.14-7.10 (m, 2H), 7.07 (d, J = 8.2 Hz, 1H), 6.95 (s, 1H), 6.45 (s, 1H), 6.34 (s, 1H), 5.70 (s, 1H), 4.86 (s, 2H), 4.17 (s, 3H), 3.52 (s, 3H), 3.40 (s, 3H); m/z N.D. |
| 50 | 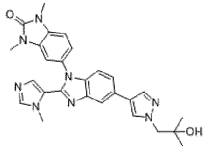 | 5-[5-[1-(2-hydroxy-2-methyl-propyl)pyrazol-4-yl]-2-(3-methylimidazol-4-yl)benzimidazol-1-yl]-1,3-dimethyl-benzimidazol-2-one | ¹H-NMR (500 MHz, CHLOROFORM-d) δ 7.91 (s, 1H), 7.86 (s, 1H), 7.71 (s, 1H), 7.49 (s, 1H), 7.37 (dd, J = 8.4, 1.4 Hz, 1H), 7.12-7.08 (m, 2H), 7.05 (d, J = 8.2 Hz, 1H), 6.94 (s, 1H), 6.43 (s, 1H), 4.14 (s, 3H), 4.10 (s, 2H), 3.50 (s, 3H), 3.39 (s, 3H), 1.21 (s, 6H); m/z N.D. |
| 51 | 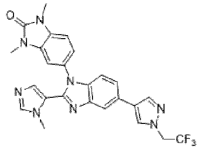 | 1,3-dimethyl-5-[2-(3-methylimidazol-4-yl)-5-[1-(2,2,2-trifluoroethyl)pyrazo l-4-yl]benzimidazol-1-yl]benzimidazol-2-one | ¹H-NMR (500 MHz, CHLOROFORM-d) δ 7.92 (s, 1H), 7.90 (s, 1H), 7.77 (s, 1H), 7.50 (s, 1H), 7.37 (dd, J = 8.4, 1.4 Hz, 1H), 7.11-7.10 (m, 2H), 7.06 (d, J = 8.2 Hz, 1H), 6.95 (s, 1H), 6.44 (s, 1H), 4.75 (q, J = 8.4 Hz, 2H), 4.15 (s, 3H), 3.50 (s, 3H), 3.39 (s, 3H); m/z N.D. |
| 52 | 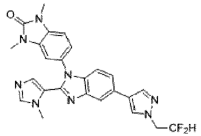 | 5-[5-[1-(2,2-difluoroethyl)pyrazol -4-yl]-2-(3-methylimidazol-4-yl)benzimidazol-1-yl]-1,3-dimethyl-benzimidazol-2-one | ¹H-NMR (500 MHz, CHLOROFORM-d) δ 7.92 (s, 1H), 7.88 (s, 1H), 7.74 (s, 1H), 7.51 (s, 1H), 7.37 (dd, J = 8.2, 1.2 Hz, 1H), 7.13-7.09 (m, 2H), 7.06 (d, J = 8.2 Hz, 1H), 6.95 (s, 1H), 6.44 (s, 1H), 6.13 (tt, J = 55.5, 4.2 Hz, 1H), 4.51 (td, J = 13.6, 4.2 Hz, 2H), 4.16 (s, 3H), 3.51 (s, 3H), 3.40 (s, 3H); m/z N.D. |
| 53 | 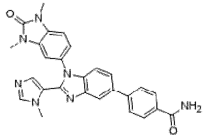 | 4-[1-(1,3-dimethyl-2-oxo-benzimidazol-5-yl)-2-(3-methylimidazol-4-yl)benzimidazol-5-yl]benzamide | ¹H-NMR (500 MHz, CHLOROFORM-d) δ 8.08 (s, 1H), 7.92 (d, J = 8.2 Hz, 2H), 7.75 (d, J = 7.9 Hz, 2H), 7.54-7.51 (m, 2H), 7.16-7.12 (m, 3H), 6.97 (s, 1H), 6.48 (s, 1H), 6.25 (s, 1H), 5.84 (s, 1H), 4.18 (s, 3H), 3.52 (s, 3H), 3.41 (s, 3H); m/z N.D. |
| 54 | 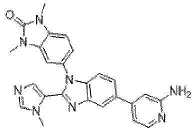 | 5-[5-(2-amino-4-pyridyl)-2-(3-methylimidazol-4-yl)benzimidazol-1-yl]-1,3-dimethyl-benzimidazol-2-one | ¹H-NMR (500 MHz, CHLOROFORM-d) δ 8.10 (d, J = 5.2 Hz, 1H), 8.06 (s, 1H), 7.52 (s, 1H), 7.48 (dd, J = 8.4, 1.4 Hz, 1H), 7.14-7.10 (m, 3H), 6.97 (d, J = 5.2 Hz, 1H), 6.95 (s, 1H), 6.81 (s, 1H), 6.46 (s, 1H), 4.74 (s, 2H), 4.17 (s, 3H), 3.51 (s, 3H), 3.40 (s, 3H); m/z N.D. |
| 55 | 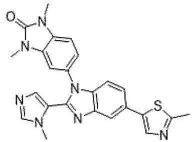 | 1,3-dimethyl-5-[2-(3-methylimidazol-4-yl)-5-(2-methylthiazol-5-yl)benzimidazol-1-yl]benzimidazol-2-one | ¹H-NMR (500 MHz, CHLOROFORM-d) δ 7.96 (s, 1H), 7.80 (s, 1H), 7.53 (s, 1H), 7.42 (dd, J = 8.5, 1.5 Hz, 1H), 7.14-7.09 (m, 2H), 7.06 (d, J = 8.5 Hz, 1H), 6.94 (d, J = 1.5 Hz, 1H), 6.45 (s, 1H), 4.17 (s, 3H), 3.52 (s, 3H), 3.40 (s, 3H), 2.74 (s, 3H); m/z N.D. |
| 56 | 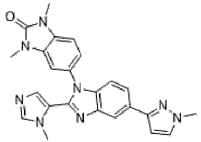 | 1,3-dimethyl-5-[2-(3-methylimidazol-4-yl)-5-(1-methylpyrazol-3-yl)benzimidazol-1-yl]benzimidazol-2-one | ¹H-NMR (500 MHz, CHLOROFORM-d) δ 8.19 (s, 1H), 7.79 (dd, J = 8.4, 1.4 Hz, 1H), 7.51 (s, 1H), 7.40 (d, J = 2.1 Hz, 1H), 7.11-7.10 (m, 2H), 7.08 (d, J = 8.5 Hz, 1H), 6.96 (s, 1H), 6.60 (d, J = 2.1 Hz, 1H), 6.45 (s, 1H), 4.16 (s, 3H), 3.96 (s, 3H), 3.51 (s, 3H), 3.40 (s, 3H); m/z N.D. |
| 57 | 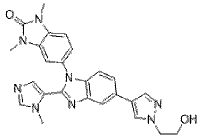 | 5-[5-[1-(2-hydroxyethyl)pyrazo l-4-yl]-2-(3-methylimidazol-4-yl)benzimidazol-1-yl]-1,3-dimethyl-benzimidazol-2-one | ¹H-NMR (500 MHz, CHLOROFORM-d) δ 7.91 (s, 1H), 7.85 (s, 1H), 7.72 (s, 1H), 7.50 (s, 1H), 7.37 (d, J = 8.5 Hz, 1H), 7.13-7.09 (m, 2H), 7.05 (d, J = 8.5 Hz, 1H), 6.94 (s, 1H), 6.44 (s, 1H), 4.30 (t, J = 4.7 Hz, 2H), 4.16 (s, 3H), 4.06 (t, J = 4.7 Hz, 2H), 3.52 (s, 3H), 3.40 (s, 3H); m/z N.D. |
| 58 | 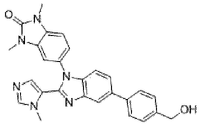 | 5-[5-[4-(hydroxymethyl)phe nyl]-2-(3-methylimidazol-4-yl)benzimidazol-1-yl]-1,3-dimethyl-benzimidazol-2-one | ¹H-NMR (500 MHz, CHLOROFORM-d) δ 8.03 (s, 1H), 7.65 (d, J = 8.2 Hz, 2H), 7.53 (s, 1H), 7.46 (d, J = 8.2 Hz, 2H), 7.12-7.10 (m, 3H), 6.95 (s, 1H), 6.46 (s, 1H), 4.75 (s, 2H), 4.17 (s, 3H), 3.52 (s, 3H), 3.40 (s, 3H); m/z N.D. |
| 59 | 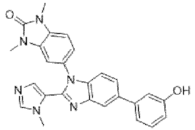 | 5-[5-(3-hydroxyphenyl)-2-(3-methylimidazol-4-yl)benzimidazol-1-yl]-1,3-dimethyl-benzimidazol-2-one | ¹H-NMR (500 MHz, CHLOROFORM-d) δ 8.17 (s, 1H), 8.02 (s, 1H), 7.56 (s, 1H), 7.47 (d, J = 8.5 Hz, 1H), 7.29-7.28 (m, 1H), 7.17-7.10 (m, 5H), 6.95 (s, 1H), 6.82 (d, J = 7.9 Hz, 1H), 6.52 (s, 1H), 4.15 (s, 3H), 3.50 (s, 3H), 3.39 (s, 3H); m/z N.D. |
| 60 | 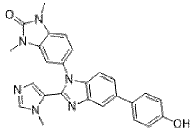 | 5-[5-(4-hydroxyphenyl)-2-(3-methylimidazol-4-yl)benzimidazol-1-yl]-1,3-dimethyl-benzimidazol-2-one | ¹H-NMR (500 MHz, CHLOROFORM-d) δ 7.98 (s, 1H), 7.56 (s, 1H), 7.51 (d, J = 8.2 Hz, 2H), 7.45 (d, J = 8.2 Hz, 1H), 7.12-7.09 (m, 3H), 6.97 (s, 1H), 6.93 (d, J = 8.2 Hz, 2H), 6.51 (s, 1H), 4.18 (s, 3H), 3.52 (s, 3H), 3.42 (s, 3H); m/z N.D. |
| 61 | 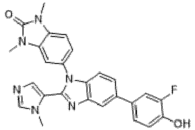 | 5-[5-(3-fluoro-4-hydroxy-phenyl)-2-(3-methylimidazol-4-yl)benzimidazol-1-yl]-1,3-dimethyl-benzimidazol-2-one | ¹H-NMR (500 MHz, CHLOROFORM-d) δ 7.96 (s, 1H), 7.57 (s, 1H), 7.43 (d, J = 7.9 Hz, 1H), 7.36 (d, J = 11.6 Hz, 1H), 7.30 (d, J = 7.6 Hz, 1H), 7.13-7.11 (m, 3H), 7.05 (t, J = 8.5 Hz, 1H), 6.96 (s, 1H), 6.50 (s, 1H), 4.18 (s, 3H), 3.53 (s, 3H), 3.42 (s, 3H); m/z N.D. |
| 62 | 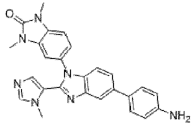 | 5-[5-(4-aminophenyl)-2-(3-methylimidazol-4-yl)benzimidazol-1-yl]-1,3-dimethyl-benzimidazol-2-one | ¹H-NMR (500 MHz, CHLOROFORM-d) δ 7.97 (s, 1H), 7.53 (s, 1H), 7.48 (d, J = 8.2 Hz, 2H), 7.45 (dd, J = 8.4, 1.4 Hz, 1H), 7.14-7.08 (m, 3H), 6.96 (s, 1H), 6.78 (d, J = 8.5 Hz, 2H), 6.47 (s, 1H), 4.17 (s, 3H), 3.52 (s, 3H), 3.40 (s, 3H); m/z N.D. |
| 63 | 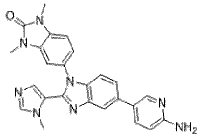 | 5-[5-(6-amino-3-pyridyl)-2-(3-methylimidazol-4-yl)benzimidazol-1-yl]-1,3-dimethyl-benzimidazol-2-one | ¹H-NMR (500 MHz, CHLOROFORM-d) δ 8.38 (s, 1H), 7.94 (s, 1H), 7.74 (d, J = 8.5 Hz, 1H), 7.52 (s, 1H), 7.39 (d, J = 8.2 Hz, 1H), 7.12-7.11 (m, 3H), 6.95 (s, 1H), 6.61 (d, J = 8.2 Hz, 1H), 6.46 (s, 1H), 4.57 (s, 2H), 4.17 (s, 3H), 3.52 (s, 3H), 3.40 (s, 3H); m/z N.D. |
| 64 | 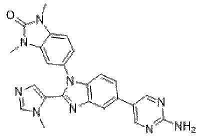 | 5-[5-(2-aminopyrimidin-5-yl)-2-(3-methylimidazol-4-yl)benzimidazol-1-yl]-1,3-dimethyl-benzimidazol-2-one | ¹H-NMR (500 MHz, CHLOROFORM-d) δ 8.60 (s, 2H), 7.92 (s, 1H), 7.54 (s, 1H), 7.35 (dd, J = 8.4, 1.4 Hz, 1H), 7.15-7.11 (m, 3H), 6.95 (s, 1H), 6.47 (s, 1H), 5.18 (s, 2H), 4.18 (s, 3H), 3.52 (s, 3H), 3.41 (s, 3H); m/z N.D. |
| 65 | 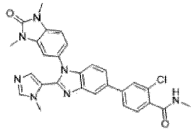 | 2-chloro-4-[1-(1,3-dimethyl-2-oxo-benzimidazol-5-yl)-2-(3-methylimidazol-4-yl)benzimidazol-5-yl]-N-methyl-benzamide | ¹H-NMR (500 MHz, CHLOROFORM-d) δ 8.00 (s, 1H), 7.75 (d, J = 7.9 Hz, 1H), 7.66 (s, 1H), 7.58 (d, J = 7.9 Hz, 1H), 7.52 (s, 1H), 7.41 (d, J = 8.2 Hz, 1H), 7.13-7.08 (m, 3H), 6.93 (s, 1H), 6.55-6.54 (m, 1H), 6.43 (s, 1H), 4.16 (s, 3H), 3.51 (s, 3H), 3.39 (s, 3H), 3.04 (d, J = 4.9 Hz, 3H); m/z N.D. |
| 66 | 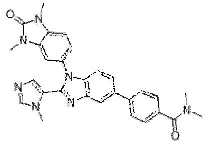 | 4-[1-(1,3-dimethyl-2-oxo-benzimidazol-5-yl)-2-(3-methylimidazol-4-yl)benzimidazol-5-yl]-N,N-dimethyl-benzamide | ¹H-NMR (500 MHz, CHLOROFORM-d) δ 8.04 (s, 1H), 7.69 (d, J = 7.9 Hz, 2H), 7.55-7.49 (m, 4H), 7.15-7.08 (m, 3H), 6.96 (s, 1H), 6.47 (s, 1H), 4.17 (s, 3H), 3.52 (s, 3H), 3.41 (s, 3H), 3.14 (s, 3H), 3.06 (s, 3H); m/z N.D. |
| 67 | 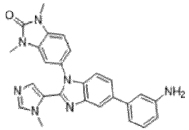 | 5-[5-(3-aminophenyl)-2-(3-methylimidazol-4-yl)benzimidazol-1-yl]-1,3-dimethyl-benzimidazol-2-one | ¹H-NMR (500 MHz, CHLOROFORM-d) δ 8.01 (s, 1H), 7.52 (s, 1H), 7.47 (d, J = 8.2 Hz, 1H), 7.25-7.22 (m, 1H), 7.11-7.05 (m, 4H), 6.98-6.95 (m, 2H), 6.67 (dd, J = 7.9, 1.5 Hz, 1H), 6.47 (s, 1H), 4.17 (s, 3H), 3.82 (s, 2H), 3.51 (s, 3H), 3.39 (s, 3H); m/z 450 |
| 68 | 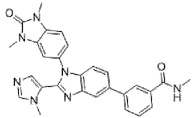 | 3-[1-(1,3-dimethyl-2-oxo-benzimidazol-5-yl)-2-(3-methylimidazol-4-yl)benzimidazol-5-yl]-N-methyl-benzamide | ¹H-NMR (500 MHz, CHLOROFORM-d) δ 8.09 (s, 1H), 8.04 (s, 1H), 7.78 (d, J = 7.6 Hz, 1H), 7.70 (d, J = 7.6 Hz, 1H), 7.51-7.48 (m, 3H), 7.13-7.12 (m, 3H), 6.96 (s, 1H), 6.46-6.43 (m, 2H), 4.17 (s, 3H), 3.51 (s, 3H), 3.40 (s, 3H), 3.03 (d, J = 4.9 Hz, 3H); m/z 492 |
| 69 | 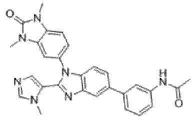 | N-[3-[1-(1,3-dimethyl-2-oxo-benzimidazol-5-yl)-2-(3-methylimidazol-4-yl)benzimidazol-5-yl]phenyl]acetamide | ¹H-NMR (500 MHz, CHLOROFORM-d) δ 8.03 (s, 1H), 7.84 (s, 1H), 7.54-7.39 (m, 6H), 7.12-7.10 (m, 3H), 6.96 (s, 1H), 6.47 (s, 1H), 4.17 (s, 3H), 3.52 (s, 3H), 3.40 (s, 3H), 2.21 (s, 3H); m/z 492 |
| 70 | 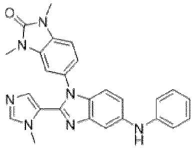 | 5-[5-anilino-2-(3-methylimidazol-4-yl)benzimidazol-1-yl]-1,3-dimethyl-benzimidazol-2-one | ¹H-NMR (500 MHz, CHLOROFORM-d) δ 7.59 (d, J = 1.8 Hz, 1H), 7.52 (s, 1H), 7.26-7.23 (m, 2H), 7.11 (s, 2H), 7.06-7.04 (m, 3H), 6.99 (d, J = 8.5 Hz, 1H), 6.95 (s, 1H), 6.88 (t, J = 7.3 Hz, 1H), 6.46 (s, 1H), 5.75 (brs, 1H), 4.14 (s, 3H), 3.52 (s, 3H), 3.41 (s, 3H); m/z 450 |
| 71 | 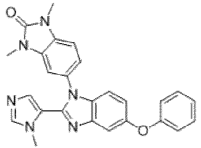 | 1,3-dimethyl-5-[2-(3-methylimidazol-4-yl)-5-phenoxy-benzimidazol-1-yl]benzimidazol-2-one | ¹H-NMR (500 MHz, CHLOROFORM-d) δ 7.51 (s, 1H), 7.46 (s, 1H), 7.34-7.31 (m, 2H), 7.12 (s, 2H), 7.08 (t, J = 7.5 Hz, 1H), 7.04-7.02 (m, 4H), 6.96 (s, 1H), 6.45 (s, 1H), 4.14 (s, 3H), 3.52 (s, 3H), 3.41 (s, 3H); m/z 451 |
| 72 | 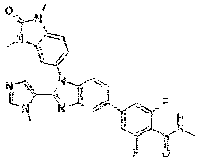 | 4-[1-(1,3-dimethyl-2-oxo-benzimidazol-5-yl)-2-(3-methylimidazol-4-yl)benzimidazol-5-yl]-2,6-difluoro-N-methyl-benzamide | ¹H-NMR (500 MHz, CHLOROFORM-d) δ 7.90 (s, 1H), 7.82 (s, 1H), 7.62-7.59 (m, 2H), 7.52-7.48 (m, 1H), 7.15-7.13 (m, 2H), 7.04 (t, J = 8.5 Hz, 1H), 6.92 (s, 1H), 6.46 (s, 1H), 6.00 (s, 1H), 4.18 (s, 3H), 3.55 (s, 3H), 3.41 (s, 3H), 3.06 (d, J = 4.6 Hz, 3H), m/z N.D. |
| 73 | 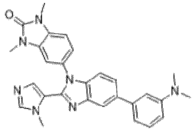 | 5-[5-[3-(dimethylamino)phe nyl]-2-(3-methylimidazol-4-yl)benzimidazol-1-yl]-1,3-dimethyl-benzimidazol-2-one | ¹H-NMR (500 MHz, CHLOROFORM-d) δ 8.07 (s, 1H), 7.53-7.51 (m, 2H), 7.34 (t, J = 8.1 Hz, 1H), 7.13-7.11 (m, 3H), 7.03-7.02 (m, 2H), 6.97 (s, 1H), 6.76 (dd, J = 8.1, 2.0 Hz, 1H), 6.47 (s, 1H), 4.18 (s, 3H), 3.52 (s, 3H), 3.41 (s, 3H), 3.03 (s, 6H); m/z 478 |
| 74 | 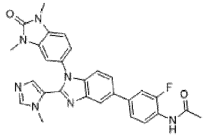 | N-[4-[1-(1,3-dimethyl-2-oxo-benzimidazol-5-yl)-2-(3-methylimidazol-4-yl)benzimidazol-5-yl]-2-fluorophenyl]acetamide | ¹H-NMR (500 MHz, CHLOROFORM-d) δ 8.36 (t, J = 8.2 Hz, 1H), 7.99 (s, 1H), 7.53-7.50 (m, 2H), 7.45-7.43 (m, 2H), 7.39 (d, J = 12.2 Hz, 1H), 7.12-7.11 (m, 3H), 6.96 (s, 1H), 6.46 (s, 1H), 4.17 (s, 3H), 3.52 (s, 3H), 3.41 (s, 3H), 2.25 (s, 3H); m/z 510 |
| 75 | 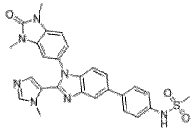 | N-[4-[1-(1,3-dimethyl-2-oxo-benzimidazol-5-yl)-2-(3-methylimidazol-4-yl)benzimidazol-5-yl]phenyl]methanesu lfonamide | ¹H-NMR (500 MHz, CHLOROFORM-d) δ 8.00 (s, 1H), 7.84 (s, 1H), 7.62 (d, J = 8.2 Hz, 2H), 7.55 (s, 1H), 7.46 (d, J = 9.2 Hz, 1H), 7.34 (d, J = 8.2 Hz, 2H), 7.14-7.11 (m, 3H), 6.97 (s, 1H), 6.49 (s, 1H), 4.16 (s, 3H), 3.51 (s, 3H), 3.41 (s, 3H), 3.03 (s, 3H); m/z 528 |
| 76 | 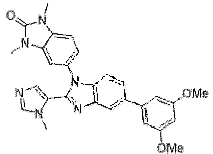 | 5-[5-(3,5-dimethoxyphenyl)-2-(3-methylimidazol-4-yl)benzimidazol-1-yl]-1,3-dimethyl-benzimidazol-2-one | ¹H-NMR (500 MHz, CHLOROFORM-d) δ 8.04 (s, 1H), 7.54 (s, 1H), 7.49 (dd, J = 8.4, 1.4 Hz, 1H), 7.12-7.09 (m, 3H), 6.96 (s, 1H), 6.81 (d, J = 2.1 Hz, 2H), 6.48-6.47 (m, 2H), 4.18 (s, 3H), 3.86 (s, 6H), 3.52 (s, 3H), 3.41 (s, 3H); m/z 495 |
| 77 | 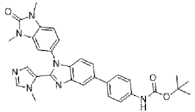 | tert-butyl N-[4-[1-(1,3-dimethyl-2-oxo-benzimidazol-5-yl)-2-(3-methylimidazol-4-yl)benzimidazol-5-yl]phenyl]carbamate | ¹H-NMR (500 MHz, CHLOROFORM-d) δ 8.00 (s, 1H), 7.59 (d, J = 8.5 Hz, 2H), 7.53 (s, 1H), 7.46-7.45 (m, 3H), 7.11-7.09 (m, 3H), 6.95 (s, 1H), 6.73 (s, 1H), 6.46 (s, 1H), 4.17 (s, 3H), 3.51 (s, 3H), 3.40 (s, 3H), 1.53 (s, 9H); m/z 550 |
| 78 | 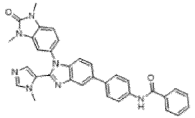 | N-[4-[1-(1,3-dimethyl-2-oxo-benzimidazol-5-yl)-2-(3-methylimidazol-4-yl)benzimidazol-5-yl]phenyl]benzamide | ¹H-NMR (500 MHz, CHLOROFORM-d) δ 8.05 (s, 1H), 7.91 (d, J = 7.3 Hz, 2H), 7.88 (s, 1H), 7.76 (d, J = 8.5 Hz, 2H), 7.70 (d, J = 8.5 Hz, 2H), 7.59-7.57 (m, 1H), 7.54-7.50 (m, 4H), 7.15-7.11 (m, 3H), 6.97 (s, 1H), 6.47 (s, 1H), 4.19 (s, 3H), 3.53 (s, 3H), 3.42 (s, 3H); m/z 554 |
| 79 | 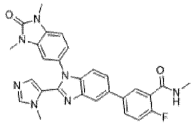 | 5-[1-(1,3-dimethyl-2-oxo-benzimidazol-5-yl)-2-(3-methylimidazol-4-yl)benzimidazol-5-yl]-2-fluoro-N-methyl-benzamide | ¹H-NMR (500 MHz, CHLOROFORM-d) δ 8.40 (dd, J = 7.6, 2.4 Hz, 1H), 8.02 (s, 1H), 7.75-7.72 (m, 1H), 7.52 (s, 1H), 7.49 (dd, J = 8.4, 1.4 Hz, 1H), 7.20 (dd, J = 11.6, 8.5 Hz, 1H), 7.13-7.12 (m, 3H), 6.96 (s, 1H), 6.82 (dd, J = 12.4, 4.7 Hz, 1H), 6.46 (s, 1H), 4.17 (s, 3H), 3.52 (s, 3H), 3.40 (s, 3H), 3.06 (d, J = 4.9 Hz, 3H); m/z N.D. |
| 80 | 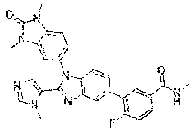 | 3-[1-(1,3-dimethyl-2-oxo-benzimidazol-5-yl)-2-(3-methylimidazol-4-yl)benzimidazol-5-yl]-4-fluoro-N-methyl-benzamide | ¹H-NMR (500 MHz, CHLOROFORM-d) δ 7.99-7.96 (m, 2H), 7.74-7.71 (m, 1H), 7.51 (s, 1H), 7.43 (d, J = 8.2 Hz, 1H), 7.20 (t, J = 9.2 Hz, 1H), 7.14-7.09 (m, 3H), 6.96 (s, 1H), 6.45 (s, 1H), 6.39-6.38 (m, 1H), 4.17 (s, 3H), 3.51 (s, 3H), 3.40 (s, 3H), 3.02 (d, J = 4.9 Hz, 3H); m/z N.D. |
| 81 | 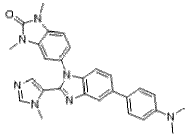 | 5-[5-[4-(dimethylamino)phe nyl]-2-(3-methylimidazol-4-yl)benzimidazol-1-yl]-1,3-dimethyl-benzimidazol-2-one | ¹H-NMR (500 MHz, CHLOROFORM-d) δ 8.00 (s, 1H), 7.58 (d, J = 8.5 Hz, 2H), 7.54 (s, 1H), 7.48 (d, J = 8.2 Hz, 1H), 7.11-7.09 (m, 3H), 6.96 (s, 1H), 6.84 (d, J = 8.5 Hz, 2H), 6.47 (s, 1H), 4.17 (s, 3H), 3.52 (s, 3H), 3.40 (s, 3H), 3.00 (s, 6H); m/z 478 |
| 82 | 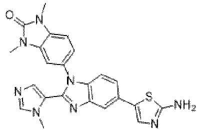 | 5-[5-(2-aminothiazol-5-yl)-2-(3-methylimidazol-4-yl)benzimidazol-1-yl]-1,3-dimethyl-benzimidazol-2-one | ¹H-NMR (500 MHz, CHLOROFORM-d) δ 7.85 (s, 1H), 7.52 (s, 1H), 7.35 (d, J = 8.5 Hz, 2H), 7.31 (s, 1H), 7.14-7.12 (m, 2H), 7.04 (d, J = 8.2 Hz, 1H), 6.94 (s, 1H), 6.45 (s, 1H), 4.99 (s, 2H), 4.17 (s, 3H), 3.52 (s, 3H), 3.41 (s, 3H); m/z N.D. |
| 83 | 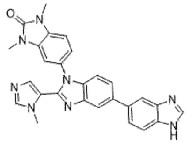 | 5-[5-(1H-benzimidazol-5-yl)-2-(3-methylimidazol-4-yl)benzimidazol-1-yl]-1,3-dimethyl-benzimidazol-2-one | ¹H-NMR (500 MHz, CHLOROFORM-d) δ 8.10 (s, 1H), 8.09 (s, 1H), 7.91 (s, 1H), 7.75 (d, J = 7.3 Hz, 1H), 7.63-7.54 (m, 3H), 7.16-7.13 (m, 3H), 6.98 (s, 1H), 6.50 (s, 1H), 4.18 (s, 3H), 3.52 (s, 3H), 3.41 (s, 3H); m/z 475 |
| 84 | 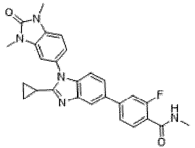 | 4-[2-cyclopropyl-1-(1,3-dimethyl-2-oxo-benzimidazol-5-yl)benzimidazol-5-yl]-2-fluoro-N-methyl-benzamide | ¹H-NMR (500 MHz, CHLOROFORM-d) δ 8.15 (t, J = 8.2 Hz, 1H), 7.92 (s, 1H), 7.51 (d, J = 8.2 Hz, 1H), 7.40 (d, J = 8.2 Hz, 1H), 7.35 (d, J = 13.7 Hz, 1H), 7.21 (d, J = 8.2 Hz, 1H), 7.15-7.13 (m, 2H), 7.08 (s, 1H), 6.81-6.77 (m, 1H), 3.51 (s, 3H), 3.46 (s, 3H), 3.04 (d, J = 4.9 Hz, 3H), 1.86-1.81 (m, 1H), 1.36-1.33 (m, 2H), 1.06-1.02 (m, 2H); m/z 470 |
| 85 | 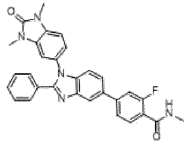 | 4-[1-(1,3-dimethyl-2-oxo-benzimidazol-5-yl)-2-phenyl-benzimidazol-5-yl]-2-fluoro-N-methyl-benzamide | ¹H-NMR (500 MHz, CHLOROFORM-d) δ 8.16 (t, J = 8.2 Hz, 1H), 8.08 (s, 1H), 7.60 (d, J = 7.6 Hz, 2H), 7.54 (d, J = 8.2 Hz, 1H), 7.48 (d, J = 8.5 Hz, 1H), 7.40-7.29 (m, 4H), 7.24 (d, J = 8.2 Hz, 1H), 7.06 (s, 2H), 6.93 (s, 1H), 6.85-6.82 (m, 1H), 3.49 (s, 3H), 3.37 (s, 3H), 3.04 (d, J = 4.9 Hz, 3H); m/z 506 |
| 86 | 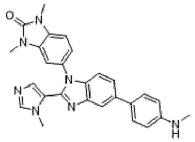 | 1,3-dimethyl-5-[5-[4-(methylamino)pheny l]-2-(3-methylimidazol-4-yl)benzimidazol-1-yl]benzimidazol-2-one | ¹H-NMR (500 MHz, CHLOROFORM-d) δ 7.98 (s, 1H), 7.53-7.51 (m, 3H), 7.46 (dd, J = 8.4, 1.4 Hz, 1H), 7.11-7.08 (m, 3H), 6.96 (s, 1H), 6.71 (d, J = 8.5 Hz, 2H), 6.47 (s, 1H), 4.16 (s, 3H), 3.51 (s, 3H), 3.40 (s, 3H), 2.89 (s, 3H); m/z 464 |
| 87 | 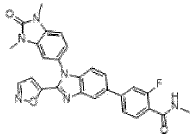 | 4-[1-(1,3-dimethyl-2-oxo-benzimidazol-5-yl)-2-isoxazol-5-yl-benzimidazol-5-yl]-2-fluoro-N-methyl-benzamide | ¹H-NMR (500 MHz, CHLOROFORM-d) δ 8.24 (d, J = 1.2 Hz, 1H), 8.18 (t, J = 8.2 Hz, 1H), 8.09 (s, 1H), 7.56 (d, J = 8.9 Hz, 1H), 7.53 (d, J = 7.9 Hz, 1H), 7.37 (d, J = 13.4 Hz, 1H), 7.22 (d, J = 8.5 Hz, 1H), 7.18-7.14 (m, 2H), 7.04 (s, 1H), 6.82-6.79 (m, 1H), 6.46 (s, 1H), 3.53 (s, 3H), 3.43 (s, 3H), 3.05 (d, J = 4.9 Hz, 3H); m/z N.D. |
| 88 | 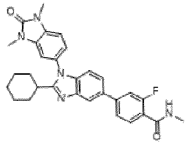 | 4-[2-cyclohexyl-1-(1,3-dimethyl-2-oxo-benzimidazol-5-yl)benzimidazol-5-yl]-2-fluoro-N-methyl-benzamide | ¹H-NMR (500 MHz, CHLOROFORM-d) δ 8.17 (t, J = 8.2 Hz, 1H), 8.03 (s, 1H), 7.53 (d, J = 8.2 Hz, 1H), 7.43 (d, J = 8.5 Hz, 1H), 7.37 (d, J = 13.4 Hz, 1H), 7.15 (d, J = 7.9 Hz, 1H), 7.10-7.08 (m, 2H), 6.96 (s, 1H), 6.79-6.76 (m, 1H), 3.53 (s, 3H), 3.46 (s, 3H), 3.06 (d, J = 4.6 Hz, 3H), 2.74-2.69 (m, 1H), 1.92-1.67 (m, 8H), 1.37-1.19 (m, 4H); m/z 512 |
| 89 | 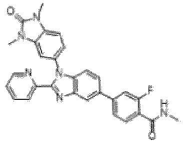 | 4-[1-(1,3-dimethyl-2-oxo-benzimidazol-5-yl)-2-(2-pyridyl)benzimidazol -5-yl]-2-fluoro-N-methyl-benzamide | ¹H-NMR (500 MHz, CHLOROFORM-d) δ 8.40 (d, J = 4.6 Hz, 1H), 8.18 (t, J = 8.4 Hz, 1H), 8.11-8.09 (m, 2H), 7.77-7.74 (m, 1H), 7.57-7.52 (m, 2H), 7.40 (d, J = 13.7 Hz, 1H), 7.27 (d, J = 5.8 Hz, 1H), 7.24 (dd, J = 7.6, 5.0 Hz, 1H), 7.06-7.02 (m, 2H), 7.00 (s, 1H), 6.84-6.80 (m, 1H), 3.49 (s, 3H), 3.39 (s, 3H), 3.06 (d, J = 4.9 Hz, 3H); m/z 507 |
| 90 | 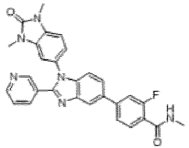 | 4-[1-(1,3-dimethyl-2-oxo-benzimidazol-5-yl)-2-(3-pyridyl)benzimidazol -5-yl]-2-fluoro-N-methyl-benzamide | ¹H-NMR (500 MHz, CHLOROFORM-d) δ 8.77 (s, 1H), 8.59 (d, J = 4.3 Hz, 1H), 8.21 (t, J = 8.2 Hz, 1H), 8.12 (s, 1H), 8.03 (d, J = 8.2 Hz, 1H), 7.56 (dd, J = 13.7, 8.2 Hz, 2H), 7.41 (d, J = 13.1 Hz, 1H), 7.32-7.27 (m, 2H), 7.11-7.07 (m, 2H), 6.95 (s, 1H), 6.82-6.78 (m, 1H), 3.50 (s, 3H), 3.39 (s, 3H), 3.07 (d, J = 4.6 Hz, 3H); m/z N.D. |
| 91 | 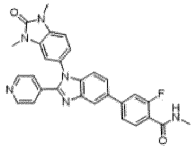 | 4-[1-(1,3-dimethyl-2-oxo-benzimidazol-5-yl)-2-(4-pyridyl)benzimidazol -5-yl]-2-fluoro-N-methyl-benzamide | ¹H-NMR (500 MHz, CHLOROFORM-d) δ 8.60 (s, 2H), 8.21 (t, J = 8.4 Hz, 1H), 8.13 (s, 1H), 7.58-7.55 (m, 2H), 7.51 (d, J = 4.0 Hz, 2H), 7.41 (dd, J = 13.6, 1.4 Hz, 1H), 7.28 (s, 1H), 7.13-7.07 (m, 2H), 6.97 (d, J = 1.2 Hz, 1H), 6.81-6.78 (m, 1H), 3.53 (s, 3H), 3.41 (s, 3H), 3.07 (d, J = 4.6 Hz, 3H); m/z 507 |
| 92 | 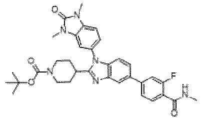 | tert-butyl 4-[1-(1,3-dimethyl-2-oxo-benzimidazol-5-yl)-5-[3-fluoro-4-(methylcarbamoyl) phenyl]benzimidazol-2-yl]piperidine-1-carboxylate | ¹H-NMR (500 MHz, CHLOROFORM-d) δ 8.17 (t, J = 8.2 Hz, 1H), 8.02 (s, 1H), 7.53 (d, J = 8.2 Hz, 1H), 7.44 (d, J = 8.5 Hz, 1H), 7.37 (d, J = 13.4 Hz, 1H), 7.15 (d, J = 7.9 Hz, 1H), 7.10-7.09 (m, 2H), 6.96 (s, 1H), 6.80-6.76 (m, 1H), 4.19 (brs, 2H), 3.53 (s, 3H), 3.46 (s, 3H), 3.06 (d, J = 4.6 Hz, 3H), 2.91-2.86 (m, 1H), 2.69 (brs, 2H), 2.01 (brs, 2H), 1.84 (brs, 2H), 1.45 (s, 9H); m/z N.D. |
| 93 | 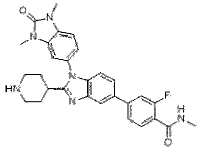 | 4-[1-(1,3-dimethyl-2-oxo-benzimidazol-5-yl)-2-(4-piperidyl)benzimidaz ol-5-yl]-2-fluoro-N-methyl-benzamide | ¹H-NMR (500 MHz, Methanol-d₄) δ 7.97 (s, 1H), 7.88-7.85 (m, 2H), 7.61-7.57 (m, 2H), 7.52 (d, J = 12.8 Hz, 1H), 7.40 (d, J = 8.2 Hz, 1H), 7.35 (s, 1H), 7.25 (d, J = 8.2 Hz, 1H), 7.19 (d, J = 8.2 Hz, 1H), 4.56 (s, 1H), 3.54 (s, 3H), 3.47 (s, 3H), 3.23 (d, J = 12.8 Hz, 2H), 3.10-3.08 (m, 1H), 2.97 (s, 3H), 2.72-2.68 (m, 2H), 2.02 (s, 4H); m/z N.D. |
| 94 | 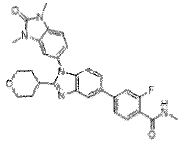 | 4-[1-(1,3-dimethyl-2-oxo-benzimidazol-5-yl)-2-tetrahydropyran-4-yl-benzimidazol-5-yl]-2-fluoro-N-methyl-benzamide | ¹H-NMR (500 MHz, CHLOROFORM-d) δ 8.17 (t, J = 8.2 Hz, 1H), 8.04 (s, 1H), 7.53 (d, J = 8.2 Hz, 1H), 7.44 (d, J = 8.7 Hz, 1H), 7.37 (d, J = 13.4 Hz, 1H), 7.16-7.08 (m, 3H), 6.97 (s, 1H), 6.80-6.77 (m, 1H), 4.03-4.02 (m, 2H), 3.53 (s, 3H), 3.46 (s, 3H), 3.36 (t, J = 11.4 Hz, 2H), 3.05 (d, J = 4.9 Hz, 3H), 3.02-2.98 (m, 1H), 2.22-2.15 (m, 2H), 1.78 (s, 2H); m/z 514 |
| 95 | 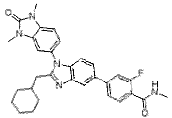 | 4-[2-(cyclohexylmethyl)-1-(1,3-dimethyl-2-oxo-benzimidazol-5-yl)benzimidazol-5-yl]-2-fluoro-N-methyl-benzamide | ¹H-NMR (500 MHz, CHLOROFORM-d) δ 8.17 (t, J = 8.4 Hz, 1H), 7.99 (s, 1H), 7.53 (d, J = 8.2 Hz, 1H), 7.42 (d, J = 8.5 Hz, 1H), 7.37 (d, J = 13.4 Hz, 1H), 7.15-7.07 (m, 3H), 6.94 (s, 1H), 6.81-6.77 (m, 1H), 3.52 (s, 3H), 3.45 (s, 3H), 3.05 (d, J = 4.6 Hz, 3H), 2.66 (d, J = 7.0 Hz, 2H), 1.95-1.90 (m, 1H), 1.72-1.61 (m, 4H), 1.23-1.18 (m, 3H), 1.14-1.05 (m, 1H), 0.95-0.88 (m, 2H); m/z 526 |
| 96 | 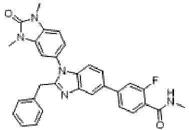 | 4-[2-benzyl-1-(1,3-dimethyl-2-oxo-benzimidazol-5-yl)benzimidazol-5-yl]-2-fluoro-N-methyl-benzamide | ¹H-NMR (500 MHz, CHLOROFORM-d) δ 8.19 (t, J = 8.2 Hz, 1H), 8.05 (s, 1H), 7.55 (d, J = 8.2 Hz, 1H), 7.45 (d, J = 8.2 Hz, 1H), 7.39 (d, J = 13.4 Hz, 1H), 7.21-7.19 (m, 3H), 7.10 (d, J = 8.2 Hz, 1H), 7.07-7.05 (m, 3H), 6.97 (dd, J = 7.9, 1.5 Hz, 1H), 6.81-6.78 (m, 1H), 6.56 (s, 1H), 4.18 (s, 2H), 3.51 (s, 3H), 3.27 (s, 3H), 3.06 (d, J = 4.6 Hz, 3H), 1.68 (s, 1H); m/z 520 |
| 97 | 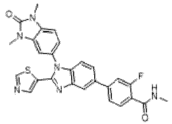 | 4-[1-(1,3-dimethyl-2-oxo-benzimidazol-5-yl)-2-thiazol-5-yl-benzimidazol-5-yl]-2-fluoro-N-methyl-benzamide | ¹H-NMR (500 MHz, CHLOROFORM-d) δ 8.78 (s, 1H), 8.20 (t, J = 8.2 Hz, 1H), 8.07 (s, 1H), 7.64 (s, 1H), 7.55 (d, J = 8.2 Hz, 1H), 7.51 (d, J = 8.5 Hz, 1H), 7.39 (d, J = 13.4 Hz, 1H), 7.21-7.19 (m, 2H), 7.17-7.15 (m, 1H), 7.02 (s, 1H), 6.81-6.77 (m, 1H), 3.55 (s, 3H), 3.44 (s, 3H), 3.06 (d, J = 4.6 Hz, 3H); m/z 513 |
| 98 | 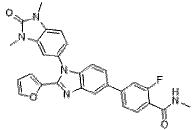 | 4-[1-(1,3-dimethyl-2-oxo-benzimidazol-5-yl)-2-(2-furyl)benzimidazol-5-yl]-2-fluoro-N-methyl-benzamide | ¹H-NMR (500 MHz, CHLOROFORM-d) δ 8.19 (t, J = 8.2 Hz, 1H), 8.07 (s, 1H), 7.56-7.55 (m, 1H), 7.51 (s, 1H), 7.48 (d, J = 8.5 Hz, 1H), 7.40 (d, J = 13.4 Hz, 1H), 7.19-7.15 (m, 2H), 7.13 (d, J = 8.2 Hz, 1H), 7.02 (s, 1H), 6.81-6.77 (m, 1H), 6.38 (d, J = 3.4 Hz, 1H), 6.18 (d, J = 3.7 Hz, 1H), 3.55 (s, 3H), 3.44 (s, 3H), 3.07 (d, J = 4.6 Hz, 3H); m/z N.D. |
| 99 | 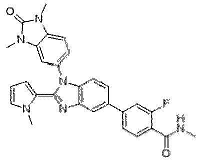 | 4-[1-(1,3-dimethyl-2-oxo-benzimidazol-5-yl)-2-(1-methylpyrrol-2-yl)benzimidazol-5-yl]-2-fluoro-N-methyl-benzamide | ¹H-NMR (500 MHz, CHLOROFORM-d) δ 8.19 (t, J = 8.2 Hz, 1H), 8.05 (s, 1H), 7.57 (d, J = 7.9 Hz, 1H), 7.46-7.40 (m, 2H), 7.13-7.12 (m, 3H), 6.96 (s, 1H), 6.81-6.76 (m, 2H), 5.96 (s, 1H), 5.74 (s, 1H), 4.15 (s, 3H), 3.52 (s, 3H), 3.41 (s, 3H), 3.07 (d, J = 4.3 Hz, 3H); m/z N.D. |
| 100 | 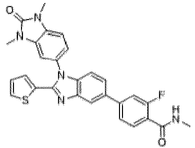 | 4-[1-(1,3-dimethyl-2-oxo-benzimidazol-5-yl)-2-(2-thienyl)benzimidazol -5-yl]-2-fluoro-N-methyl-benzamide | ¹H-NMR (500 MHz, CHLOROFORM-d) δ 8.18 (t, J = 8.2 Hz, 1H), 8.06 (s, 1H), 7.54 (d, J = 8.2 Hz, 1H), 7.46 (d, J = 8.2 Hz, 1H), 7.39-7.36 (m, 2H), 7.20-7.16 (m, 2H), 7.10 (d, J = 8.5 Hz, 1H), 7.03 (s, 1H), 6.98 (d, J = 3.7 Hz, 1H), 6.94-6.93 (m, 1H), 6.81-6.78 (m, 1H), 3.54 (s, 3H), 3.43 (s, 3H), 3.06 (d, J = 4.6 Hz, 3H); m/z 512 |
| 101 | 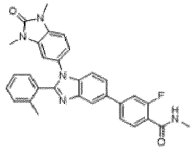 | 4-[1-(1,3-dimethyl-2-oxo-benzimidazol-5-yl)-2-(o-tolyl)benzimidazol-5-yl]-2-fluoro-N-methyl-benzamide | ¹H-NMR (500 MHz, CHLOROFORM-d) δ 8.21 (t, J = 8.2 Hz, 1H), 8.12 (s, 1H), 7.59 (d, J = 8.1 Hz, 1H), 7.56 (d, J = 8.5 Hz, 1H), 7.45-7.41 (m, 2H), 7.33-7.28 (m, 2H), 7.20 (d, J = 7.6 Hz, 1H), 7.17-7.14 (m, 1H), 7.00-6.95 (m, 2H), 6.82-6.78 (m, 2H), 3.43 (s, 3H), 3.31 (s, 3H), 3.07 (d, J = 4.6 Hz, 3H); m/z N.D. |
| 102 | 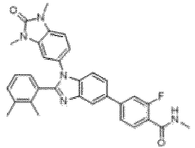 | 4-[1-(1,3-dimethyl-2-oxo-benzimidazol-5-yl)-2-(2,3-dimethylphenyl)benz imidazol-5-yl]-2-fluoro-N-methyl-benzamide | ¹H-NMR (500 MHz, CHLOROFORM-d) δ 8.21 (t, J = 8.2 Hz, 1H), 8.11 (s, 1H), 7.59 (d, J = 8.4 Hz, 1H), 7.55 (d, J = 8.5 Hz, 1H), 7.45-7.39 (m, 2H), 7.20-7.17 (m, 2H), 7.09-7.06 (m, 1H), 6.99-6.94 (m, 2H), 6.81-6.79 (m, 2H), 3.43 (s, 3H), 3.32 (s, 3H), 3.07 (d, J = 4.6 Hz, 3H), 2.23 (s, 3H), 2.09 (s, 3H); m/z N.D. |
| 103 | 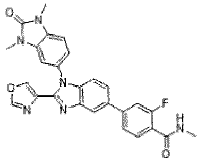 | 4-[1-(1,3-dimethyl-2-oxo-benzimidazol-5-yl)-2-oxazol-4-yl-benzimidazol-5-yl]-2-fluoro-N-methyl-benzamide | ¹H-NMR (500 MHz, CHLOROFORM-d) δ 8.20 (t, J = 8.2 Hz, 1H), 8.10 (s, 1H), 7.88 (s, 1H), 7.63 (s, 1H), 7.56 (d, J = 8.2 Hz, 1H), 7.51 (d, J = 8.5 Hz, 1H), 7.40 (d, J = 13.4 Hz, 1H), 7.20-7.15 (m, 3H), 7.02 (s, 1H), 6.81-6.77 (m, 1H), 3.53 (s, 3H), 3.44 (s, 3H), 3.07 (d, J = 4.6 Hz, 3H); m/z N.D. |
| 104 | 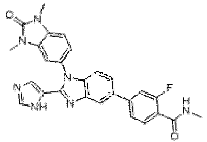 | 4-[1-(1,3-dimethyl-2-oxo-benzimidazol-5-yl)-2-(1H-imidazol-5-yl)benzimidazol-5-yl]-2-fluoro-N-methyl-benzamide | ¹H-NMR (500 MHz, CHLOROFORM-d) δ 8.19 (s, 1H), 7.97 (s, 1H), 7.72 (s, 1H), 7.54-7.49 (m, 2H), 7.38 (d, J = 14.0 Hz, 1H), 7.20-7.04 (m, 4H), 6.81-6.79 (m, 1H), 6.52 (s, 1H), 3.55 (s, 3H), 3.44 (s, 3H), 3.07 (s, 3H); m/z 496 |
| 105 | 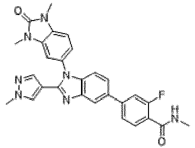 | 4-[1-(1,3-dimethyl-2-oxo-benzimidazol-5-yl)-2-(1-methylpyrazol-4-yl)benzimidazol-5-yl]-2-fluoro-N-methyl-benzamide | ¹H-NMR (500 MHz, CHLOROFORM-d) δ 8.18 (t, J = 8.4 Hz, 1H), 8.05 (s, 1H), 7.83 (s, 1H), 7.54 (d, J = 7.9 Hz, 1H), 7.47 (d, J = 8.2 Hz, 1H), 7.39 (d, J = 13.4 Hz, 1H), 7.21-7.17 (m, 3H), 7.12 (d, J = 8.2 Hz, 1H), 7.01 (s, 1H), 6.82-6.79 (m, 1H), 3.88 (s, 3H), 3.54 (s, 3H), 3.43 (s, 3H), 3.06 (d, J = 4.6 Hz, 3H); m/z N.D. |
| 106 | 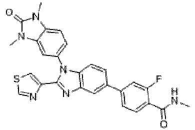 | 4-[1-(1,3-dimethyl-2-oxo-benzimidazol-5-yl)-2-thiazol-4-yl-benzimidazol-5-yl]-2-fluoro-N-methyl-benzamide | ¹H-NMR (500 MHz, CHLOROFORM-d) δ 8.73 (s, 1H), 8.20 (t, J = 8.2 Hz, 1H), 8.10 (s, 1H), 7.73 (s, 1H), 7.56 (d, J = 7.9 Hz, 1H), 7.52 (d, J = 8.2 Hz, 1H), 7.40 (d, J = 13.4 Hz, 1H), 7.22 (d, J = 8.2 Hz, 1H), 7.12-7.11 (m, 2H), 7.01 (s, 1H), 6.80-6.77 (m, 1H), 3.52 (s, 3H), 3.41 (s, 3H), 3.07 (d, J = 4.9 Hz, 3H); m/z 513 |
| 107 | 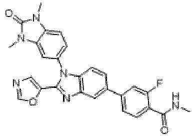 | 4-[1-(1,3-dimethyl-2-oxo-benzimidazol-5-yl)-2-oxazol-5-yl-benzimidazol-5-yl]-2-fluoro-N-methyl-benzamide | ¹H-NMR (500 MHz, CHLOROFORM-d) δ 8.21 (t, J = 8.2 Hz, 1H), 8.10 (s, 1H), 7.96 (s, 1H), 7.57-7.53 (m, 2H), 7.40 (d, J = 13.1 Hz, 1H), 7.20-7.18 (m, 3H), 7.02 (s, 1H), 6.81-6.77 (m, 1H), 6.73 (s, 1H), 3.55 (s, 3H), 3.45 (s, 3H), 3.07 (d, J = 4.6 Hz, 3H); m/z 497 |
| 108 | 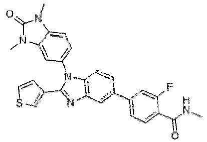 | 4-[1-(1,3-dimethyl-2-oxo-benzimidazol-5-yl)-2-(3-thienyl)benzimidazol -5-yl]-2-fluoro-N-methyl-benzamide | ¹H-NMR (500 MHz, CHLOROFORM-d) δ 8.17 (t, J = 8.1 Hz, 1H), 8.06 (s, 1H), 7.55 (d, J = 7.9 Hz, 1H), 7.47 (d, J = 7.9 Hz, 1H), 7.42-7.38 (m, 2H), 7.28 (m, 2H), 7.15 (m, 3H), 7.00 (s, 1H), 6.83-6.81 (m, 1H), 3.54 (s, 3H), 3.42 (s, 3H), 3.06 (s, 3H); m/z 512 |
| 109 | 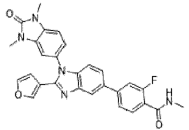 | 4-[1-(1,3-dimethyl-2-oxo-benzimidazol-5-yl)-2-(3-furyl)benzimidazol-5-yl]-2-fluoro-N-methyl-benzamide | ¹H-NMR (500 MHz, CHLOROFORM-d) δ 8.18 (t, J = 8.2 Hz, 1H), 8.05 (s, 1H), 7.54 (d, J = 8.2 Hz, 1H), 7.46 (d, J = 8.2 Hz, 1H), 7.40-7.37 (m, 2H), 7.18-7.17 (m, 3H), 7.12 (d, J = 8.2 Hz, 1H), 7.01 (s, 1H), 6.81-6.79 (m, 1H), 6.73 (s, 1H), 3.54 (s, 3H), 3.43 (s, 3H), 3.05 (d, J = 4.3 Hz, 3H); m/z 496 |
| 110 | 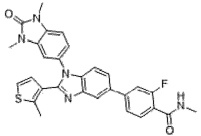 | 4-[1-(1,3-dimethyl-2-oxo-benzimidazol-5-yl)-2-(2-methyl-3-thienyl)benzimidazol -5-yl]-2-fluoro-N-methyl-benzamide | ¹H-NMR (500 MHz, CHLOROFORM-d) δ 8.20 (t, J = 8.2 Hz, 1H), 8.11 (s, 1H), 7.58 (d, J = 8.2 Hz, 1H), 7.52 (d, J = 8.4 Hz, 1H), 7.43 (d, J = 13.4 Hz, 1H), 7.33 (d, J = 8.2 Hz, 1H), 7.05 (m, 2H), 6.92 (d, J = 5.2 Hz, 1H), 6.87 (s, 1H), 6.82-6.78 (m, 1H), 6.61 (d, J = 5.2 Hz, 1H), 3.49 (s, 3H), 3.38 (s, 3H), 3.07 (d, J = 4.6 Hz, 3H), 2.67 (s, 3H); m/z 526 |
| 111 | 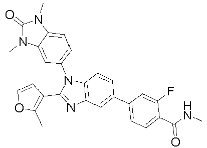 | 4-[1-(1,3-dimethyl-2-oxo-benzimidazol-5-yl)-2-(2-methyl-3-furyl)benzimidazol-5-yl]-2-fluoro-N-methyl-benzamide | ¹H-NMR (500 MHz, CHLOROFORM-d) δ 8.20 (t, J = 8.2 Hz, 1H), 8.08 (s, 1H), 7.58 (d, J = 8.2 Hz, 1H), 7.48 (d, J = 8.9 Hz, 1H), 7.42 (d, J = 13.7 Hz, 1H), 7.21 (d, J = 8.2 Hz, 1H), 7.12 (s, 1H), 6.96 (s, 1H), 6.81-6.78 (m, 1H), 5.74 (s, 1H), 3.52 (s, 3H), 3.42 (s, 3H), 3.07 (d, J = 4.6 Hz, 3H), 2.70 (s, 3H); m/z 510 |
| 112 | 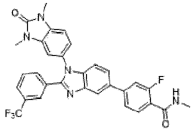 | 4-[1-(1,3-dimethyl-2-oxo-benzimidazol-5-yl)-2-[3-(trifluoromethyl)phe nyl]benzimidazol-5-yl]-2-fluoro-N-methyl-benzamide | ¹H-NMR (500 MHz, CHLOROFORM-d) δ 8.20 (t, J = 8.2 Hz, 1H), 8.11 (s, 1H), 8.04 (s, 1H), 7.68 (d, J = 7.6 Hz, 1H), 7.62 (d, J = 7.6 Hz, 1H), 7.56 (d, J = 8.2 Hz, 1H), 7.54 (d, J = 8.5 Hz, 1H), 7.42-7.39 (m, 2H), 7.27 (d, J = 8.5 Hz, 1H), 7.11-7.07 (m, 2H), 6.95 (s, 1H), 6.82-6.79 (m, 1H), 3.51 (s, 3H), 3.40 (s, 3H), 3.06 (d, J = 4.6 Hz, 3H); m/z 574 |
| 113 | 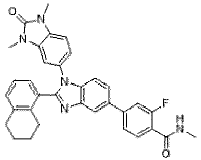 | 4-[1-(1,3-dimethyl-2-oxo-benzimidazol-5-yl)-2-tetralin-5-yl-benzimidazol-5-yl]-2-fluoro-N-methyl-benzamide | ¹H-NMR (500 MHz, CHLOROFORM-d) δ 8.20 (t, J = 8.2 Hz, 1H), 8.09 (s, 1H), 7.58 (d, J = 8.2 Hz, 1H), 7.54 (d, J = 8.5 Hz, 1H), 7.42 (d, J = 13.7 Hz, 1H), 7.38 (d, J = 8.5 Hz, 1H), 7.11-7.09 (m, 2H), 7.05 (d, J = 7.6 Hz, 1H), 7.03-6.95 (m, 2H), 6.83-6.82 (m, 1H), 6.80 (s, 1H), 3.43 (s, 3H), 3.32 (s, 3H), 3.06 (d, J = 4.6 Hz, 3H), 2.76 (t, J = 6.3 Hz, 2H), 2.62 (t, J = 6.1 Hz, 2H), 1.72-1.65 (m, 4H); m/z 560 |
| 114 | 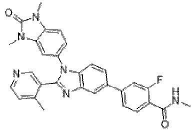 | 4-[1-(1,3-dimethyl-2-oxo-benzimidazol-5-yl)-2-(4-methyl-3-pyridyl)benzimidazol -5-yl]-2-fluoro-N-methyl-benzamide | ¹H-NMR (500 MHz, CHLOROFORM-d) δ 8.47 (s, 1H), 8.44 (d, J = 4.9 Hz, 1H), 8.18 (t, J = 8.1 Hz, 1H), 8.10 (s, 1H), 7.57 (s, 1H), 7.56 (s, 1H), 7.42-7.38 (m, 2H), 7.17 (d, J = 4.9 Hz, 1H), 6.97-6.93 (m, 2H), 6.83-6.81 (m, 2H), 3.42 (s, 3H), 3.33 (s, 3H), 3.05 (d, J = 4.3 Hz, 3H), 2.34 (s, 3H); m/z 521 |
| 115 | 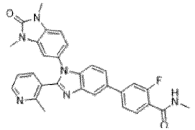 | 4-[1-(1,3-dimethyl-2-oxo-benzimidazol-5-yl)-2-(2-methyl-3-pyridyl)benzimidazol -5-yl]-2-fluoro-N-methyl-benzamide | ¹H-NMR (500 MHz, CHLOROFORM-d) δ 8.53 (d, J = 4.6 Hz, 1H), 8.19 (t, J = 8.2 Hz, 1H), 8.10 (s, 1H), 7.63 (d, J = 7.6 Hz, 1H), 7.57 (s, 1H), 7.56 (s, 1H), 7.43-7.39 (m, 2H), 7.12-7.10 (m, 1H), 6.98-6.93 (m, 2H), 6.83-6.81 (m, 1H), 6.79 (s, 1H), 3.43 (s, 3H), 3.33 (s, 3H), 3.05 (d, J = 4.9 Hz, 3H), 2.48 (s, 3H); m/z 521 |
| 116 | 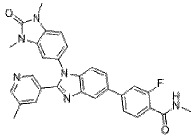 | 4-[1-(1,3-dimethyl-2-oxo-benzimidazol-5-yl)-2-(5-methyl-3-pyridyl)benzimidazol -5-yl]-2-fluoro-N-methyl-benzamide | ¹H-NMR (500 MHz, CHLOROFORM-d) δ 8.42 (s, 1H), 8.35 (s, 1H), 8.21 (t, J = 8.2 Hz, 1H), 8.11 (s, 1H), 8.08 (s, 1H), 7.58 (d, J = 9.2 Hz, 1H), 7.54 (d, J = 8.9 Hz, 1H), 7.42 (d, J = 13.1 Hz, 1H), 7.28 (d, J = 8.5 Hz, 1H), 7.09 (m, 2H), 6.95 (s, 1H), 6.81-6.77 (m, 1H), 3.51 (s, 3H), 3.40 (s, 3H), 3.07 (d, J = 4.6 Hz, 3H), 2.37 (s, 3H); m/z N.D. |
| 117 | 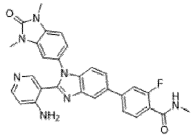 | 4-[2-(4-amino-3-pyridyl)-1-(1,3-dimethyl-2-oxo-benzimidazol-5-yl)benzimidazol-5-yl]-2-fluoro-N-methyl-benzamide | ¹H-NMR (500 MHz, CHLOROFORM-d) δ 8.24 (t, J = 8.2 Hz, 1H), 8.08 (s, 1H), 8.06 (d, J = 5.8 Hz, 1H), 7.99 (s, 1H), 7.60 (d, J = 7.3 Hz, 1H), 7.55 (d, J = 8.2 Hz, 1H), 7.44 (d, J = 13.4 Hz, 1H), 7.27 (m, 1H), 7.12 (s, 2H), 6.98 (s, 1H), 6.83-6.80 (m, 1H), 6.67 (d, J = 5.8 Hz, 1H), 6.53 (s, 2H), 3.52 (s, 3H), 3.42 (s, 3H), 3.10 (d, J = 4.6 Hz, 3H); m/z 522 |
| 118 | 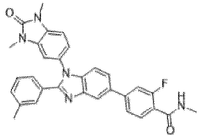 | 4-[1-(1,3-dimethyl-2-oxo-benzimidazol-5-yl)-2-(m-tolyl)benzimidazol-5-yl]-2-fluoro-N-methyl-benzamide | ¹H-NMR (500 MHz, CHLOROFORM-d) δ 8.20 (t, J = 8.2 Hz, 1H), 8.10 (s, 1H), 7.70 (s, 1H), 7.57 (d, J = 8.2 Hz, 1H), 7.50 (d, J = 8.5 Hz, 1H), 7.41 (d, J = 13.4 Hz, 1H), 7.25 (m, 1H), 7.19-7.04 (m, 5H), 6.94 (s, 1H), 6.81-6.77 (m, 1H), 3.51 (s, 3H), 3.39 (s, 3H), 3.07 (d, J = 4.6 Hz, 3H), 2.34 (s, 3H); m/z 520 |
| 119 | 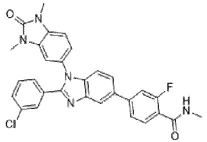 | 4-[2-(3-chlorophenyl)-1-(1,3-dimethyl-2-oxo-benzimidazol-5-yl)benzimidazol-5-yl]-2-fluoro-N-methyl-benzamide | ¹H-NMR (500 MHz, CHLOROFORM-d) δ 8.20 (t, J = 8.2 Hz, 1H), 8.10 (s, 1H), 7.79 (s, 1H), 7.57 (d, J = 7.9 Hz, 1H), 7.53 (d, J = 8.5 Hz, 1H), 7.41 (d, J = 13.4 Hz, 1H), 7.36-7.34 (m, 2H), 7.27-7.25 (m, 1H), 7.21 (t, J = 7.9 Hz, 2H), 7.11-7.07 (m, 2H), 6.94 (s, 1H), 6.81-6.78 (m, 1H), 3.52 (s, 3H), 3.41 (s, 3H), 3.07 (d, J = 4.6 Hz, 3H); m/z 540 |
| 120 | 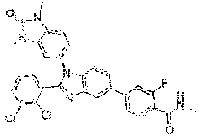 | 4-[2-(2,3-dichlorophenyl)-1-(1,3-dimethyl-2-oxo-benzimidazol-5-yl)benzimidazol-5-yl]-2-fluoro-N-methyl-benzamide | ¹H-NMR (500 MHz, CHLOROFORM-d) δ 8.17 (t, J = 8.2 Hz, 1H), 8.09 (s, 1H), 7.56-7.55 (m, 2H), 7.51 (d, J = 8.2 Hz, 1H), 7.43-7.37 (m, 3H), 7.26-7.22 (m, 1H), 7.00-6.95 (m, 2H), 6.90 (s, 1H), 6.85-6.82 (m, 1H), 3.43 (s, 3H), 3.34 (s, 3H), 3.05 (d, J = 4.6 Hz, 3H); m/z 575 |
| 121 | 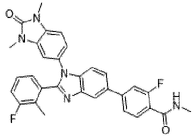 | 4-[1-(1,3-dimethyl-2-oxo-benzimidazol-5-yl)-2-(3-fluoro-2-methylphenyl)benzimidazol -5-yl]-2-fluoro-N-methyl-benzamide | ¹H-NMR (500 MHz, CHLOROFORM-d) δ 8.19 (t, J = 8.2 Hz, 1H), 8.09 (s, 1H), 7.58-7.55 (m, 2H), 7.43-7.39 (m, 2H), 7.16-7.12 (m, 2H), 7.08-7.03 (m, 1H), 6.98-6.94 (m, 2H), 6.85-6.81 (m, 1H), 6.79 (s, 1H), 3.44 (s, 3H), 3.33 (s, 3H), 3.06 (d, J = 4.9 Hz, 3H), 2.14 (d, J = 1.8 Hz, 3H); m/z 538 |
| 122 | 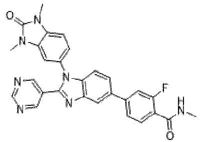 | 4-[1-(1,3-dimethyl-2-oxo-benzimidazol-5-yl)-2-pyrimidin-5-yl-benzimidazol-5-yl]-2-fluoro-N-methyl-benzamide | ¹H-NMR (500 MHz, CHLOROFORM-d) δ 9.19 (s, 1H), 8.97 (s, 2H), 8.22 (t, J = 8.2 Hz, 1H), 8.14 (s, 1H), 7.59 (s, 1H), 7.57 (s, 1H), 7.42 (d, J = 13.4 Hz, 1H), 7.29 (d, J = 8.2 Hz, 1H), 7.14-7.09 (m, 2H), 6.98 (s, 1H), 6.82-6.79 (m, 1H), 3.52 (s, 3H), 3.41 (s, 3H), 3.07 (d, J = 4.6 Hz, 3H); m/z 508 |
| 123 | 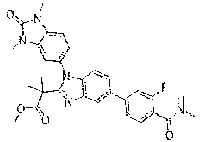 | methyl 2-[1-(1,3-dimethyl-2-oxo-benzimidazol-5-yl)-5-[3-fluoro-4-(methylcarbamoyl)p henyl]benzimidazol-2-yl]-2-methylpropanoate | ¹H-NMR (500 MHz, CHLOROFORM-d) δ 8.17 (t, J = 8.2 Hz, 1H), 8.07 (s, 1H), 7.53 (d, J = 8.2 Hz, 1H), 7.44 (d, J = 8.5 Hz, 1H), 7.36 (d, J = 13.4 Hz, 1H), 7.10-7.02 (m, 2H), 6.94 (d, J = 8.5 Hz, 1H), 6.88 (s, 1H), 6.80-6.76 (m, 1H), 3.57 (s, 3H), 3.51 (s, 3H), 3.42 (s, 3H), 3.06 (d, J = 4.6 Hz, 3H), 1.69 (s, 3H), 1.66 (s, 3H); m/z 530 |
| 124 | 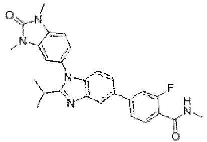 | 4-[1-(1,3-dimethyl-2-oxo-benzimidazol-5-yl)-2-isopropyl-benzimidazol-5-yl]-2-fluoro-N-methyl-benzamide | ¹H-NMR (500 MHz, CHLOROFORM-d) δ 8.18 (t, J = 8.4 Hz, 1H), 8.04 (s, 1H), 7.54 (d, J = 7.9 Hz, 1H), 7.44 (d, J = 8.2 Hz, 1H), 7.37 (d, J = 14.0 Hz, 1H), 7.16-7.09 (m, 3H), 6.97 (s, 1H), 6.80-6.76 (m, 1H), 3.53 (s, 3H), 3.46 (s, 3H), 3.12-3.08 (m, 1H), 3.06 (d, J = 4.6 Hz, 3H), 1.38 (d, J = 6.7 Hz, 6H); m/z 472 |
| 125 | 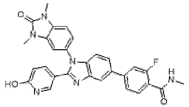 | 4-[1-(1,3-dimethyl-2-oxo-benzimidazol-5-yl)-2-(6-hydroxy-3-pyridyl)benzimidazol -5-yl]-2-fluoro-N-methyl-benzamide | ¹H-NMR (500 MHz, CHLOROFORM-d) δ 8.19 (t, J = 8.4 Hz, 1H), 8.03 (s, 1H), 7.70 (d, J = 9.8 Hz, 1H), 7.67 (s, 1H), 7.55 (d, J = 7.9 Hz, 1H), 7.49 (d, J = 7.9 Hz, 1H), 7.40 (d, J = 13.4 Hz, 1H), 7.18 (d, J = 8.2 Hz, 1H), 7.14-7.08 (m, 2H), 6.98 (s, 1H), 6.82-6.79 (m, 1H), 6.49 (d, J = 9.5 Hz, 1H), 3.52 (s, 3H), 3.44 (s, 3H), 3.06 (d, J = 4.0 Hz, 3H); m/z 523 |
| 126 | 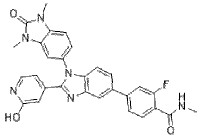 | 4-[1-(1,3-dimethyl-2-oxo-benzimidazol-5-yl)-2-(2-hydroxy-4-pyridyl)benzimidazol -5-yl]-2-fluoro-N-methyl-benzamide | ¹H-NMR (500 MHz, CHLOROFORM-d) δ 8.20 (t, J = 8.2 Hz, 1H), 8.11 (s, 1H), 7.56 (s, 1H), 7.55 (s, 1H), 7.40 (d, J = 13.4 Hz, 1H), 7.33 (d, J = 6.7 Hz, 1H), 7.23 (d, J = 8.5 Hz, 1H), 7.13-7.08 (m, 2H), 6.97 (s, 1H), 6.84 (d, J = 7.0 Hz, 1H), 6.81-6.79 (m, 1H), 6.53 (s, 1H), 3.51 (s, 3H), 3.42 (s, 3H), 3.07 (d, J = 4.6 Hz, 3H); m/z 523 |
| 127 | 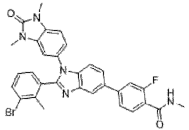 | 4-[2-(3-bromo-2-methyl-phenyl)-1-(1,3-dimethyl-2-oxo-benzimidazol-5-yl)benzimidazol-5-yl]-2-fluoro-N-methyl-benzamide | ¹H-NMR (500 MHz, CHLOROFORM-d) δ 8.21 (t, J = 8.2 Hz, 1H), 8.11 (s, 1H), 7.60-7.56 (m, 3H), 7.43 (d, J = 13.4 Hz, 1H), 7.39 (d, J = 8.5 Hz, 1H), 7.30 (d, J = 7.6 Hz, 1H), 7.05-7.02 (m, 1H), 6.98-6.93 (m, 2H), 6.82-6.80 (m, 2H), 3.44 (s, 3H), 3.34 (s, 3H), 3.07 (d, J = 4.6 Hz, 3H), 2.27 (s, 3H); m/z 598, 600 |
| 128 | 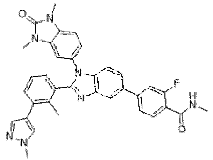 | 4-[1-(1,3-dimethyl-2-oxo-benzimidazol-5-yl)-2-[2-methyl-3-(1-methylpyrazol-4-yl)phenyl]benzimida zol-5-yl]-2-fluoro-N-methyl-benzamide | ¹H-NMR (500 MHz, CHLOROFORM-d) δ 8.17 (t, J = 8.2 Hz, 1H), 8.09 (s, 1H), 7.57-7.53 (m, 2H), 7.50 (s, 1H), 7.42 (s, 1H), 7.39 (s, 1H), 7.38 (s, 1H), 7.33 (d, J = 7.3 Hz, 1H), 7.25 (d, J = 8.2 Hz, 1H), 7.17-7.14 (m, 1H), 7.00-6.95 (m, 2H), 6.88-6.84 (m, 1H), 6.83 (s, 1H), 3.93 (s, 3H), 3.42 (s, 3H), 3.31 (s, 3H), 3.04 (d, J = 4.3 Hz, 3H), 2.22 (s, 3H); m/z 600 |
| 129 | 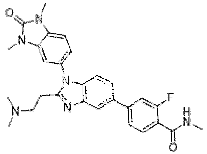 | 4-[2-[2-(dimethylamino)ethy l]-1-(1,3-dimethyl-2-oxo-benzimidazol-5-yl)benzimidazol-5-yl]-2-fluoro-N-methyl-benzamide | ¹H-NMR (500 MHz, CHLOROFORM-d) δ 8.18 (t, J = 8.2 Hz, 1H), 8.00 (s, 1H), 7.54 (d, J = 7.9 Hz, 1H), 7.45 (d, J = 8.2 Hz, 1H), 7.38 (d, J = 13.7 Hz, 1H), 7.14-7.13 (m, 3H), 7.00 (s, 1H), 6.82-6.76 (m, 1H), 3.52 (s, 3H), 3.45 (s, 3H), 3.06 (d, J = 4.6 Hz, 3H), 2.98-2.95 (m, 2H), 2.86-2.83 (m, 2H), 2.23 (s, 6H); m/z 501 |
| 130 | 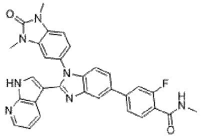 | 4-[1-(1,3-dimethyl-2-oxo-benzimidazol-5-yl)-2-(1H-pyrrolo[2,3-b]pyridin-3-yl)benzimidazol-5-yl]-2-fluoro-N-methyl-benzamide | ¹H-NMR (500 MHz, CHLOROFORM-d) δ 10.59 (s, 1H), 9.00 (d, J = 7.9 Hz, 1H), 8.35 (s, 1H), 8.19 (t, J = 8.2 Hz, 1H), 8.12 (s, 1H), 7.58 (d, J = 7.9 Hz, 1H), 7.46 (d, J = 8.2 Hz, 1H), 7.42 (d, J = 13.7 Hz, 1H), 7.29-7.27 (m, 1H), 7.23 (d, J = 7.9 Hz, 1H), 7.18 (d, J = 8.2 Hz, 1H), 7.13 (d, J = 8.2 Hz, 1H), 7.05 (s, 1H), 6.82-6.79 (m, 1H), 6.76 (s, 1H), 3.55 (s, 3H), 3.41 (s, 3H), 3.06 (d, J = 4.0 Hz, 3H); m/z 546 |
| 131 | 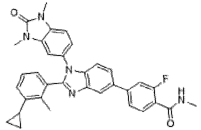 | 4-[2-(3-cyclopropyl-2-methyl-phenyl)-1-(1,3-dimethyl-2-oxo-benzimidazol-5-yl)benzimidazol-5-yl]-2-fluoro-N-methyl-benzamide | ¹H-NMR (500 MHz, CHLOROFORM-d) δ 8.21 (t, J = 8.1 Hz, 1H), 8.11 (s, 1H), 7.59 (d, J = 8.2 Hz, 1H), 7.55 (d, J = 8.2 Hz, 1H), 7.44-7.40 (m, 2H), 7.18 (d, J = 7.0 Hz, 1H), 7.10-7.05 (m, 2H), 6.99-6.94 (m, 2H), 6.82-6.80 (m, 1H), 6.78 (s, 1H), 3.43 (s, 3H), 3.31 (s, 3H), 3.07 (d, J = 4.3 Hz, 3H), 2.25 (s, 3H), 1.83-1.77 (m, 1H), 0.90 (d, J = 7.9 Hz, 2H), 0.55 (d, J = 5.2 Hz, 2H); m/z 560 |
| 132 | 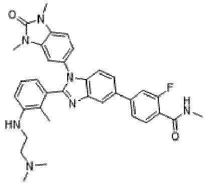 | 4-[2-[3-[2-(dimethylamino)ethy lamino]-2-methyl-phenyl]-1-(1,3-dimethyl-2-oxo-benzimidazol-5-yl)benzimidazol-5-yl]-2-fluoro-N-methyl-benzamide | ¹H-NMR (500 MHz, CHLOROFORM-d) δ 8.19 (t, J = 8.2 Hz, 1H), 8.09 (s, 1H), 7.58 (d, J = 8.2 Hz, 1H), 7.53 (d, J = 8.2 Hz, 1H), 7.43-7.38 (m, 2H), 7.09-7.06 (m, 1H), 6.99 (d, J = 8.2 Hz, 1H), 6.94 (d, J = 8.2 Hz, 1H), 6.84-6.80 (m, 2H), 6.73 (d, J = 7.6 Hz, 1H), 6.62 (d, J = 8.2 Hz, 1H), 3.47 (s, 3H), 3.42 (s, 3H), 3.31 (s, 3H), 3.15 (t, J = 5.6 Hz, 2H), 3.06 (d, J = 4.3 Hz, 3H), 2.57 (t, J = 5.5 Hz, 2H), 2.22 (s, 6H); m/z 606 |
| 133 | 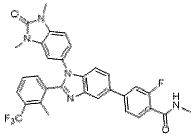 | 4-[1-(1,3-dimethyl-2-oxo-benzimidazol-5-yl)-2-[2-methyl-3-(trifluoromethyl)phe nyl]benzimidazol-5-yl]-2-fluoro-N-methyl-benzamide | ¹H-NMR (500 MHz, CHLOROFORM-d) δ 8.21 (t, J = 8.2 Hz, 1H), 8.11 (s, 1H), 7.68 (d, J = 7.5 Hz, 1H), 7.59 (s, 1H), 7.57 (s, 1H), 7.50 (d, J = 7.6 Hz, 1H), 7.44-7.40 (m, 2H), 7.29-7.27 (m, 1H), 6.97-6.93 (m, 2H), 6.82-6.80 (m, 1H), 6.78 (s, 1H), 3.43 (s, 3H), 3.33 (s, 3H), 3.07 (d, J = 4.0 Hz, 3H), 2.36 (s, 3H); m/z 588 |
| 134 | 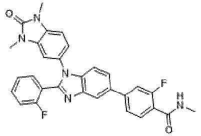 | 4-[1-(1,3-dimethyl-2-oxo-benzimidazol-5-yl)-2-(2-fluorophenyl)benzim idazol-5-yl]-2-fluoro-N-methyl-benzamide | ¹H-NMR (500 MHz, CHLOROFORM-d) δ 8.20 (t, J = 8.1 Hz, 1H), 8.12 (s, 1H), 7.68-7.66 (m, 1H), 7.58-7.55 (m, 2H), 7.43-7.38 (m, 3H), 7.22 (t, J = 7.6 Hz, 1H), 7.01-6.97 (m, 3H), 6.90 (s, 1H), 6.82-6.79 (m, 1H), 3.46 (s, 3H), 3.36 (s, 3H), 3.07 (d, J = 4.0 Hz, 3H); m/z 524 |
| 135 | 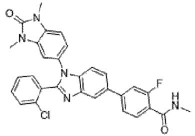 | 4-[2-(2-chlorophenyl)-1-(1,3-dimethyl-2-oxo-benzimidazol-5-yl)benzimidazol-5-yl]-2-fluoro-N-methyl-benzamide | ¹H-NMR (500 MHz, CHLOROFORM-d) δ 8.21 (t, J = 8.2 Hz, 1H), 8.13 (s, 1H), 7.59-7.57 (m, 2H), 7.52 (d, J = 7.3 Hz, 1H), 7.44-7.40 (m, 2H), 7.37-7.34 (m, 2H), 7.32-7.29 (m, 1H), 7.03 (d, J = 8.2 Hz, 1H), 6.96 (d, J = 7.9 Hz, 1H), 6.88 (s, 1H), 6.82-6.79 (m, 1H), 3.43 (s, 3H), 3.33 (s, 3H), 3.07 (d, J = 4.3 Hz, 3H); m/z 540 |
| 136 | 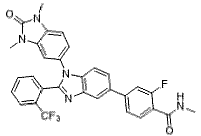 | 4-[1-(1,3-dimethyl-2-oxo-benzimidazol-5-yl)-2-[2-(trifluoromethyl)phe nyl]benzimidazol-5-yl]-2-fluoro-N-methyl-benzamide | ¹H-NMR (500 MHz, CHLOROFORM-d) δ 8.19 (t, J = 8.1 Hz, 1H), 8.12 (s, 1H), 7.74 (d, J = 7.6 Hz, 1H), 7.57 (t, J = 7.3 Hz, 2H), 7.53-7.48 (m, 2H), 7.43 (d, J = 6.7 Hz, 1H), 7.41 (s, 1H), 7.33 (d, J = 8.5 Hz, 1H), 6.99 (d, J = 8.2 Hz, 1H), 6.94 (d, J = 7.9 Hz, 1H), 6.85 (s, 1H), 6.82-6.80 (m, 1H), 3.42 (s, 3H), 3.33 (s, 3H), 3.06 (d, J = 4.3 Hz, 3H); m/z 574 |
| 137 | 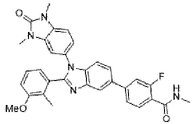 | 4-[1-(1,3-dimethyl-2-oxo-benzimidazol-5-yl)-2-(3-methoxy-2-methyl-phenyl)benzimidazol -5-yl]-2-fluoro-N-methyl-benzamide | ¹H-NMR (500 MHz, CHLOROFORM-d) δ 8.20 (t, J = 8.2 Hz, 1H), 8.11 (s, 1H), 7.58 (d, J = 7.9 Hz, 1H), 7.55 (d, J = 8.2 Hz, 1H), 7.44-7.39 (m, 2H), 7.16-7.13 (m, 1H), 7.00-6.94 (m, 3H), 6.87 (d, J = 8.2 Hz, 1H), 6.83-6.80 (m, 1H), 6.78 (s, 1H), 3.81 (s, 3H), 3.43 (s, 3H), 3.31 (s, 3H), 3.07 (s, 3H), 2.03 (s, 3H); m/z 550 |
| 138 | 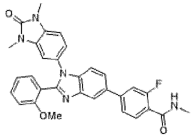 | 4-[1-(1,3-dimethyl-2-oxo-benzimidazol-5-yl)-2-(2-methoxyphenyl)benz imidazol-5-yl]-2-fluoro-N-methyl-benzamide | ¹H-NMR (500 MHz, CHLOROFORM-d) δ 8.20 (t, J = 8.1 Hz, 1H), 8.11 (s, 1H), 7.60-7.57 (m, 2H), 7.53 (d, J = 8.5 Hz, 1H), 7.43-7.38 (m, 3H), 7.03 (s, 1H), 7.02 (s, 1H), 6.96 (d, J = 8.2 Hz, 1H), 6.84 (s, 1H), 6.82-6.80 (m, 1H), 6.77 (d, J = 7.6 Hz, 1H), 3.44 (s, 6H), 3.32 (s, 3H), 3.07 (d, J = 2.7 Hz, 3H); m/z 536 |
| 139 | 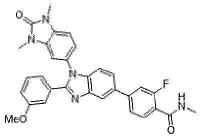 | 4-[1-(1,3-dimethyl-2-oxo-benzimidazol-5-yl)-2-(3-methoxyphenyl)benz imidazol-5-yl]-2-fluoro-N-methyl-benzamide | ¹H-NMR (500 MHz, CHLOROFORM-d) δ 8.20 (t, J = 8.1 Hz, 1H), 8.11 (s, 1H), 7.57 (d, J = 8.2 Hz, 1H), 7.51 (d, J = 8.5 Hz, 1H), 7.41 (d, J = 13.4 Hz, 1H), 7.31 (s, 1H), 7.26-7.24 (m, 1H), 7.20-7.17 (m, 1H), 7.11-7.06 (m, 3H), 6.93-6.91 (m, 2H), 6.81-6.78 (m, 1H), 3.76 (s, 3H), 3.50 (s, 3H), 3.39 (s, 3H), 3.07 (d, J = 2.7 Hz, 3H); m/z 536 |
| 140 | 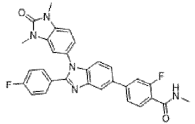 | 4-[1-(1,3-dimethyl-2-oxo-benzimidazol-5-yl)-2-(4-fluorophenyl)benzim idazol-5-yl]-2-fluoro-N-methyl-benzamide | ¹H-NMR (500 MHz, CHLOROFORM-d) δ 8.20 (t, J = 7.6 Hz, 1H), 8.09 (s, 1H), 7.63-7.61 (m, 2H), 7.57 (d, J = 8.2 Hz, 1H), 7.51 (d, J = 8.9 Hz, 1H), 7.41 (d, J = 13.4 Hz, 1H), 7.24 (m, 1H), 7.10-7.06 (m, 2H), 7.03-7.00 (m, 2H), 6.93 (s, 1H), 6.81-6.77 (m, 1H), 3.51 (s, 3H), 3.40 (s, 3H), 3.07 (d, J = 3.1 Hz, 3H); m/z 524 |
| 141 | 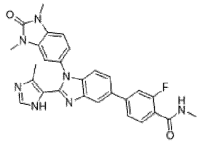 | 4-[1-(1,3-dimethyl-2-oxo-benzimidazol-5-yl)-2-(4-methyl-1H-imidazol-5-yl)benzimidazol-5-yl]-2-fluoro-N-methyl-benzamide | ¹H-NMR (500 MHz, CHLOROFORM-d) δ 8.13 (t, J = 8.1 Hz, 1H), 7.97 (s, 1H), 7.52 (d, J = 7.9 Hz, 1H), 7.46 (d, J = 8.5 Hz, 1H), 7.38-7.35 (m, 2H), 7.26-7.24 (m, 2H), 7.08 (d, J = 8.2 Hz, 1H), 7.03-7.00 (m, 2H), 6.88-6.84 (m, 1H), 3.45 (s, 3H), 3.36 (s, 3H), 3.05 (d, J = 4.0 Hz, 3H), 2.37 (s, 3H); m/z 510 |
| 142 | 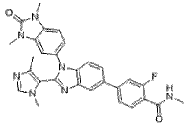 | 4-[2-(3,5-dimethylimidazol-4-yl)-1-(1,3-dimethyl-2-oxo-benzimidazol-5-yl)benzimidazol-5-yl]-2-fluoro-N-methyl-benzamide | ¹H-NMR (500 MHz, CHLOROFORM-d) δ 8.22 (t, J = 7.9 Hz, 1H), 8.12 (s, 1H), 7.59 (s, 1H), 7.57 (s, 1H), 7.48-7.42 (m, 3H), 7.05-7.02 (m, 1H), 6.97 (d, J = 8.2 Hz, 1H), 6.82 (s, 1H), 6.79-6.77 (m, 1H), 3.65 (s, 3H), 3.47 (s, 3H), 3.38 (s, 3H), 3.07 (s, 3H), 1.87 (s, 3H); m/z 524 |
| 143 | 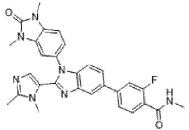 | 4-[2-(2,3-dimethylimidazol-4-yl)-1-(1,3-dimethyl-2-oxo-benzimidazol-5-yl)benzimidazol-5-yl]-2-fluoro-N-methyl-benzamide | ¹H-NMR (500 MHz, CHLOROFORM-d) δ 8.18 (t, J = 8.1 Hz, 1H), 8.05 (s, 1H), 7.56 (d, J = 8.2 Hz, 1H), 7.48 (d, J = 8.5 Hz, 1H), 7.40 (d, J = 13.4 Hz, 1H), 7.13 (d, J = 8.5 Hz, 1H), 7.10 (s, 2H), 6.95 (s, 1H), 6.81-6.78 (m, 1H), 6.38 (s, 1H), 4.09 (s, 3H), 3.51 (s, 3H), 3.41 (s, 3H), 3.06 (d, J = 3.1 Hz, 3H), 2.43 (s, 3H); m/z 524 |
| 144 | 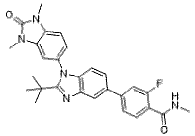 | 4-[2-tert-butyl-1-(1,3-dimethyl-2-oxo-benzimidazol-5-yl)benzimidazol-5-yl]-2-fluoro-N-methyl-benzamide | ¹H-NMR (500 MHz, CHLOROFORM-d) δ 8.20-8.15 (m, 1H), 8.04 (s, 1H), 7.53 (d, J = 8.2 Hz, 1H), 7.38-7.35 (m, 2H), 7.13-7.11 (m, 2H), 6.98 (s, 1H), 6.84 (d, J = 8.5 Hz, 1H), 6.81-6.71 (m, 1H), 3.53 (s, 3H), 3.44 (s, 3H), 3.06 (d, J = 4.0 Hz, 3.5H), 1.40 (s, 9H); m/z 486 |
| 145 | 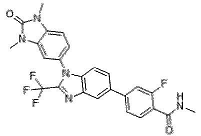 | 4-[1-(1,3-dimethyl-2-oxo-benzimidazol-5-yl)-2-(trifluoromethyl)ben zimidazol-5-yl]-2-fluoro-N-methyl-benzamide | ¹H-NMR (500 MHz, CHLOROFORM-d) δ 8.22 (t, J = 7.8 Hz, 1H), 8.15 (s, 1H), 7.63 (d, J = 8.5 Hz, 1H), 7.54 (d, J = 8.5 Hz, 1H), 7.38 (d, J = 13.4 Hz, 1H), 7.20-7.13 (m, 3H), 7.04 (s, 1H), 6.80-6.77 (m, 1H), 3.53 (s, 3H), 3.46 (s, 3H), 3.07 (d, J = 3.0Hz, 3H); m/z 498 |
| 146 | 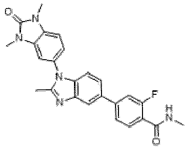 | 4-[1-(1,3-dimethyl-2-oxo-benzimidazol-5-yl)-2-methyl-benzimidazol-5-yl]-2-fluoro-N-methyl-benzamide | ¹H-NMR (500 MHz, CHLOROFORM-d) δ 8.17 (t, J = 8.2 Hz, 1H), 7.95 (s, 1H), 7.53 (dd, J = 8.2, 1.2 Hz, 1H), 7.44 (dd, J = 8.5, 1.2 Hz, 1H), 7.37 (d, J = 13.4 Hz, 1H), 7.15-7.09 (m, 3H), 6.97 (d, J = 1.5 Hz, 1H), 6.82-6.78 (m, 1H), 3.52 (s, 3H), 3.46 (s, 3H), 3.05 (d, J = 4.6 Hz, 3H), 2.52 (s, 3H); m/z 444 |
| 147 | 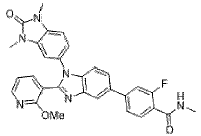 | 4-[1-(1,3-dimethyl-2-oxo-benzimidazol-5-yl)-2-(2-methoxy-3-pyridyl)benzimidazol -5-yl]-2-fluoro-N-methyl-benzamide | ¹H-NMR (500 MHz, CHLOROFORM-d) δ 8.22-8.19 (m, 2H), 8.11 (s, 1H), 7.94 (d, J = 7.3 Hz, 1H), 7.59-7.54 (m, 2H), 7.44-7.37 (m, 2H), 6.99 (s, 3H), 6.88 (s, 1H), 6.81-6.77 (m, 1H), 3.61 (s, 3H), 3.46 (s, 3H), 3.36 (s, 3H), 3.07 (d, J = 3.5 Hz, 3H); m/z 537 |
| 148 | 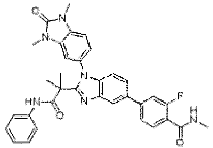 | 4-[2-(2-anilino-1,1-dimethyl-2-oxo-ethyl)-1-(1,3-dimethyl-2-oxo-benzimidazol-5-yl)benzimidazol-5-yl]-2-fluoro-N-methyl-benzamide | ¹H-NMR (500 MHz, CHLOROFORM-d) δ 8.18 (t, J = 8.1 Hz, 1H), 8.10 (s, 1H), 7.54 (d, J = 8.2 Hz, 1H), 7.51 (s, 1H), 7.47 (d, J = 8.2 Hz, 1H), 7.40-7.37 (m, 3H), 7.30-7.26 (m, 2H), 7.10 (t, J = 7.2 Hz, 1H), 7.05 (s, 2H), 6.99 (d, J = 8.2 Hz, 1H), 6.80-6.75 (m, 2H), 3.47 (s, 3H), 3.07 (s, 6H), 1.80 (s, 3H), 1.60 (s, 3H); m/z 591 |
| 149 | 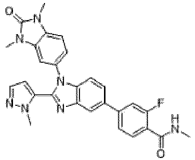 | 4-[1-(1,3-dimethyl-2-oxo-benzimidazol-5-yl)-2-(2-methylpyrazol-3-yl)benzimidazol-5-yl]-2-fluoro-N-methyl-benzamide | ¹H-NMR (500 MHz, CHLOROFORM-d) δ 8.21 (t, J = 8.2 Hz, 1H), 8.12 (s, 1H), 7.58 (d, J = 8.2 Hz, 1H), 7.54 (d, J = 8.5 Hz, 1H), 7.43 (d, J = 13.4 Hz, 1H), 7.32 (s, 1H), 7.22 (d, J = 8.9 Hz, 1H), 7.13-7.09 (m, 2H), 6.93 (s, 1H), 6.80 (s, 1H), 5.76 (s, 1H), 4.38 (s, 3H), 3.53 (s, 3H), 3.42 (s, 3H), 3.07 (s, 3H); m/z 510 |
| 150 | 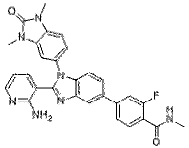 | 4-[2-(2-amino-3-pyridyl)-1-(1,3-dimethyl-2-oxo-benzimidazol-5-yl)benzimidazol-5-yl]-2-fluoro-N-methyl-benzamide | ¹H-NMR (500 MHz, CHLOROFORM-d) δ 8.21 (t, J = 8.1 Hz, 1H), 8.06-8.05 (m, 2H), 7.57 (d, J = 7.9 Hz, 1H), 7.52 (d, J = 8.5 Hz, 1H), 7.42 (d, J = 13.4 Hz, 1H), 7.24 (d, J = 8.2 Hz, 1H), 7.11 (s, 3H), 6.92 (s, 1H), 6.81-6.74 (m, 3H), 6.36-6.33 (m, 1H), 3.52 (s, 3H), 3.40 (s, 3H), 3.07 (s, 3H); m/z 522 |
| 151 | 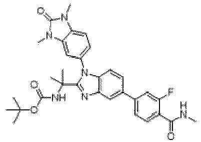 | tert-butyl N-[1-[1-(1,3-dimethyl-2-oxo-benzimidazol-5-yl)-5-[3-fluoro-4-(methylcarbamoyl)p henyl]benzimidazol-2-yl]-1-methylethyl]carbamate | ¹H-NMR (500 MHz, CHLOROFORM-d) δ 8.16 (t, J = 8.9 Hz, 1H), 8.03 (s, 1H), 7.53-7.51 (m, 1H), 7.41-7.39 (m, 1H), 7.36 (d, J = 14.0 Hz, 1H), 7.10-7.07 (m, 2H), 6.97 (s, 1H), 6.86 (d, J = 9.5 Hz, 1H), 6.79-6.76 (m, 1H), 5.04 (s, 1H), 3.52 (s, 3H), 3.43 (s, 3H), 3.06 (s, 3H), 1.76 (d, J = 19.8 Hz, 6H), 1.35 (s, 9H); m/z 587 |
| 152 | 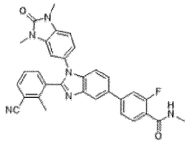 | 4-[2-(3-cyano-2-methyl-phenyl)-1-(1,3-dimethyl-2-oxo-benzimidazol-5-yl)benzimidazol-5-yl]-2-fluoro-N-methyl-benzamide | ¹H-NMR (500 MHz, CHLOROFORM-d) δ 8.22 (t, J = 8.0 Hz, 1H), 8.12 (s, 1H), 7.67 (d, J = 17.7 Hz, 1H), 7.60-7.57 (m, 3H), 7.45-7.40 (m, 2H), 7.30 (d, J = 8.5 Hz, 1H), 6.98-6.97 (m, 1H), 6.92-6.90 (m, 1H), 6.81-6.78 (m, 2H), 3.46 (s, 3H), 3.36 (s, 3H), 3.07 (d, J = 5.5 Hz, 3H), 2.46 (s, 3H); m/z 545 |
| 153 | 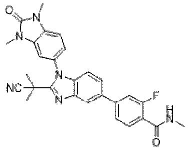 | 4-[2-(1-cyano-1-methyl-ethyl)-1-(1,3-dimethyl-2-oxo-benzimidazol-5-yl)benzimidazol-5-yl]-2-fluoro-N-methyl-benzamide | ¹H-NMR (500 MHz, CHLOROFORM-d) δ 8.17 (s, 1H), 8.05 (s, 1H), 7.53-7.48 (m, 2H), 7.36 (d, J = 13.1 Hz, 1H), 7.21 (s, 1H), 7.16 (s, 2H), 7.03 (d, J = 8.2 Hz, 1H), 6.79 (s, 1H), 3.52 (s, 3H), 3.45 (s, 3H), 3.05 (s, 3H), 1.92 (s, 3H), 1.79 (s, 3H); m/z 497 |
| 154 | 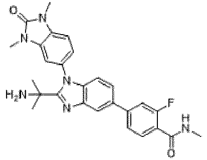 | 4-[2-(1-amino-1-methyl-ethyl)-1-(1,3-dimethyl-2-oxo-benzimidazol-5-yl)benzimidazol-5-yl]-2-fluoro-N-methyl-benzamide | ¹H-NMR (500 MHz, Methanol-d₄) δ 7.99 (s, 1H), 7.84 (t, J = 7.8 Hz, 1H), 7.60-7.50 (m, 3H), 7.40 (m, 2H), 7.29-7.28 (m, 1H), 6.95 (d, J = 7.9 Hz, 1H), 3.53 (s, 3H), 3.45 (s, 3H), 2.96 (s, 3H), 1.54 (s, 6H); m/z 487 |
| 155 | 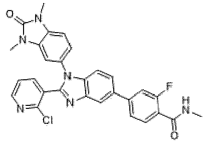 | 4-[2-(2-chloro-3-pyridyl)-1-(1,3-dimethyl-2-oxo-benzimidazol-5-yl)benzimidazol-5-yl]-2-fluoro-N-methyl-benzamide | ¹H-NMR (500 MHz, CHLOROFORM-d) δ 8.46 (s, 1H), 8.22 (t, J = 8.1 Hz, 1H), 8.14 (s, 1H), 7.89 (d, J = 8.2 Hz, 1H), 7.61-7.58 (m, 2H), 7.44-7.40 (m, 2H), 7.33-7.32 (m, 1H), 7.01-6.96 (m, 2H), 6.91 (s, 1H), 6.79 (s, 1H), 3.44 (s, 3H), 3.36 (s, 3H), 3.08 (d, J = 3.1 Hz, 3H); m/z N.D. |
| 156 | 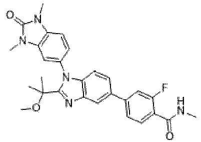 | 4-[1-(1,3-dimethyl-2-oxo-benzimidazol-5-yl)-2-(1-methoxy-1-methyl-ethyl)benzimidazol-5-yl]-2-fluoro-N-methyl-benzamide | ¹H-NMR (500 MHz, CHLOROFORM-d) δ 8.18 (t, J = 8.0 Hz, 1H), 8.07 (s, 1H), 7.54 (d, J = 8.0 Hz, 1H), 7.46 (d, J = 8.9 Hz, 1H), 7.37 (d, J = 13.7 Hz, 1H), 7.16-7.14 (m, 1H), 7.11-7.09 (m, 1H), 7.01 (m, 2H), 6.80-6.77 (m, 1H), 3.51 (s, 3H), 3.44 (s, 3H), 3.16 (s, 3H), 3.06 (d, J = 3.0 Hz, 3H), 1.65 (d, J = 21.1 Hz, 6H); m/z 502 |
| 157 | 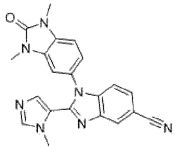 | 1',3'-dimethyl-2-(1-methyl-1H-imidazol-5-yl)-2'-oxo-2',3'-dihydro-1'H-[1,5'-bibenzo[d]imidazole] -5-carbonitrile | ¹H-NMR (500 MHz, DMSO-d₆) δ 8.33 (dd, J = 1.4, 0.7 Hz, 1H), 7.86 (s, 1H), 7.63 (ddd, J = 8.4, 1.4, 0.7 Hz, 1H), 7.48 (d, J = 1.8 Hz, 1H), 7.38 (d, J = 8.2 Hz, 1H), 7.25 - 7.23 (m, 1H), 7.23 - 7.21 (m, 1H), 6.37 (s, 1H), 4.06 (s, 3H), 3.43 (s, 3H), 3.33 (s, 3H); m/z N.D. |
| 158 | 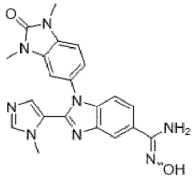 | N'-hydroxy-1',3'-dimethyl-2-(1-methyl-1H-imidazol-5-yl)-2'-oxo-2',3'-dihydro-1'H-[1,5'-bibenzo[d]imidazole] -5-carboximidamide | ¹H-NMR (500 MHz, DMSO-d₆) δ 10.43 (s, 1H), 8.89 (s, 2H), 8.10 (s, 1H), 7.82 (s, 1H), 7.80 (s, 2H), 7.64 (dd, J = 8.7, 1.3 Hz, 1H), 7.43 (d, J = 1.9 Hz, 1H), 7.36 (d, J = 8.2 Hz, 1H), 7.18 (dd, J = 8.2, 2.0 Hz, 1H), 7.06 (d, J = 8.4 Hz, 1H), 6.36 (s, 1H), 4.05 (d, J = 3.6 Hz, 3H), 3.43 (s, 3H), 3.33 (s, 3H); m/z N.D. |
| 159 | 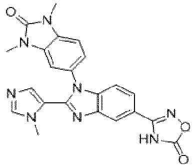 | 3-(1',3'-dimethyl-2-(1-methyl-1H-imidazol-5-yl)-2'-oxo-2',3'-dihydro-1'H-[1,5'-bibenzo[d]imidazol]-5-yl)-1,2,4-oxadiazol-5(2H)-one | ¹H-NMR (500 MHz, DMSO-d₆) δ 12.99 (s, 1H), 8.25 (d, J = 1.0 Hz, 1H), 8.14 (s, 1H), 7.76 (dd, J = 8.5, 1.4 Hz, 1H), 7.48 (d, J = 1.7 Hz, 1H), 7.39 (d, J = 8.2 Hz, 1H), 7.28 (d, J = 8.6 Hz, 1H), 7.23 (dd, J = 8.2, 1.7 Hz, 1H), 6.55 (s, 1H), 4.10 (s, 3H), 3.43 (s, 3H), 3.33 (s, 3H); m/z N.D. |
| 160 | 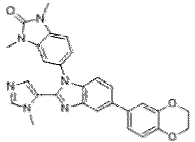 | 5-(2,3-dihydrobenzo[b][1,4] dioxin-6-yl)-1',3'-dimethyl-2-(1-methyl-1H-imidazol-5-yl)-1',3'-dihydro-2'H-[1,5'-bibenzo[d]imidazol]-2'-one | ¹H-NMR (500 MHz, CHLOROFORM-d) δ 7.98 (d, J = 1.2 Hz, 1H), 7.59 (s, 1H), 7.45 (dd, J = 8.4, 1.6 Hz, 1H), 7.19 (d, J = 2.2 Hz, 1H), 7.15 (dd, J = 8.3, 2.2 Hz, 1H), 7.13 - 7.09 (m, 3H), 6.98 - 6.93 (m, 2H), 6.48 (s, 1H), 4.31 (s, 4H), 4.19 (s, 3H), 3.52 (s, 3H), 3.41 (s, 3H); m/z N.D. |
| 161 | 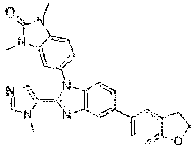 | 5-(2,3-dihydrobenzofuran-5-yl)-1',3'-dimethyl-2-(1-methyl-1H-imidazol-5-yl)-1',3'-dihydro-2'H-[1,5'-bibenzo[d]imidazol]-2'-one | ¹H-NMR (500 MHz, CHLOROFORM-d) δ 7.98 (d, J = 1.3 Hz, 1H), 7.62 (s, 1H), 7.50 (s, 1H), 7.45 (dd, 1H), 7.41 (dd, J = 8.2, 1.9 Hz, 1H), 7.13 - 7.12 (m, 2H), 7.11 (d, J = 8.5 Hz, 1H), 6.96 (s, 1H), 6.88 (d, J = 8.2 Hz, 1H), 6.49 (s, 1H), 4.63 (t, J = 8.7 Hz, 2H), 4.20 (s, 3H), 3.53 (s, 3H), 3.41 (s, 3H), 3.30 (t, J = 8.6 Hz, 2H); m/z N.D. |
| 162 | 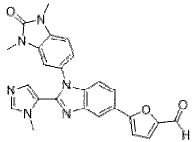 | 5-(1',3'-dimethyl-2-(1-methyl-1H-imidazol-5-yl)-2'-oxo-2',3'-dihydro-1'H-[1,5'-bibenzo[d]imidazol]-5-yl)furan-2-carbaldehyde | ¹H-NMR (500 MHz, CHLOROFORM-d) δ 9.65 (s, 1H), 8.29 (d, J = 1.0 Hz, 1H), 7.76 (dd, J = 8.5, 1.6 Hz, 1H), 7.61 (s, 1H), 7.35 (dd, J = 6.6, 2.4 Hz, 1H), 7.15 - 7.09 (m, 3H), 6.96 (d, J = 1.6 Hz, 1H), 6.87 (d, J = 3.7 Hz, 1H), 6.47 (s, 1H), 4.19 (s, 3H), 3.53 (s, 3H), 3.42 (s, 3H); m/z N.D. |
| 163 | 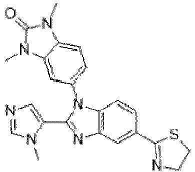 | 5-(4,5-dihydrothiazol-2-yl)-1',3'-dimethyl-2-(1-methyl-1H-imidazol-5-yl)-1',3'-dihydro-2'H-[1,5'-bibenzo[d]imidazol]-2'-one | ¹H-NMR (500 MHz, CHLOROFORM-d) δ 8.26 (d, J = 0.8 Hz, 1H), 7.86 (dd, J = 8.5, 1.4 Hz, 1H), 7.52 (s, 1H), 7.15 - 7.10 (m, 2H), 7.07 (d, J = 8.5 Hz, 1H), 6.95 (s, 1H), 6.44 (s, 1H), 4.49 (t, J = 8.2 Hz, 2H), 4.18 (s, 3H), 3.52 (s, 3H), 3.45 (t, J = 8.2 Hz, 2H), 3.41 (s, 3H); m/z N.D. |
| 164 | 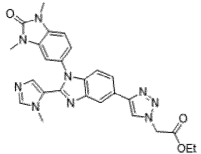 | ethyl 2-(4-(1',3'-dimethyl-2-(1-methyl-1H-imidazol-5-yl)-2'-oxo-2',3'-dihydro-1'H-[1,5'-bibenzo[d]imidazol]-5-yl)-1H-1,2,3-triazol-1-yl)acetate | ¹H-NMR (500 MHz, CHLOROFORM-d) δ 8.23 (d, J = 0.9 Hz, 1H), 7.96 (s, 1H), 7.86 (dd, J = 8.4, 1.5 Hz, 1H), 7.57 (s, 1H), 7.14 (s, 1H), 7.13 (s, 3H), 6.97 (s, 1H), 6.49 (s, 1H), 5.24 (s, 2H), 4.31 (q, J = 7.1 Hz, 2H), 4.18 (s, 3H), 3.53 (s, 3H), 3.42 (s, 3H), 1.33 (t, J = 7.1 Hz, 3H); m/z N.D. |
| 165 | 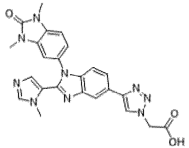 | 2-(4-(1',3'-dimethyl-2-(1-methyl-1H-imidazol-5-yl)-2'-oxo-2',3'-dihydro-1'H-[1,5'-bibenzo[d]imidazol]-5-yl)-1H-1,2,3-triazol-1-yl)acetic acid | ¹H-NMR (500 MHz, Methanol-d₄) δ 8.33 (s, 1H), 8.26 (s, 1H), 7.81 (d, J = 8.4 Hz, 2H), 7.37 (d, J = 8.2 Hz, 1H), 7.35 (s, 1H), 7.27 - 7.19 (m, 2H), 6.61 (s, 1H), 5.04 (s, 2H), 4.06 (s, 3H), 3.52 (s, 3H), 3.42 (s, 3H); m/z N.D. |
| 166 | 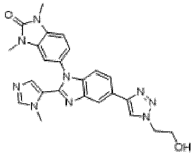 | 5-(1-(2-hydroxyethyl)-1H-1,2,3-triazol-4-yl)-1',3'-dimethyl-2-(1-methyl-1H-imidazol-5-yl)-1',3'-dihydro-2'H-[1,5'-bibenzo[d]imidazol]-2'-one | ¹H-NMR (500 MHz, Methanol-d₄) δ 8.39 (s, 1H), 8.25 (d, J = 1.0 Hz, 1H), 7.81 (dd, J = 8.5, 1.5 Hz, 2H), 7.37 (d, J = 8.2 Hz, 1H), 7.34 (d, J = 1.7 Hz, 1H), 7.26 (d, J = 8.4 Hz, 1H), 7.22 (dd, J = 8.2, 1.8 Hz, 1H), 6.61 (s, 1H), 4.57 (t, 2H), 4.06 (s, 3H), 4.01 (t, 2H), 3.52 (s, 3H), 3.42 (s, 3H); m/z N.D. |
| 167 | 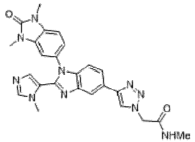 | 2-(4-(1',3'-dimethyl-2-(1-methyl-1H-imidazol-5-yl)-2'-oxo-2',3'-dihydro-1'H-[1,5'-bibenzo[d]imidazol]-5-yl)-1H-1,2,3-triazol-1-yl)-N-methylacetamide | ¹H-NMR (500 MHz, CHLOROFORM-d) δ 8.23 (d, J = 1.0 Hz, 1H), 7.94 (s, 1H), 7.82 (dd, J = 8.4, 1.5 Hz, 2H), 7.57 (s, 1H), 7.13 (dd, J = 12.6, 5.3 Hz, 2H), 6.95 (d, J = 1.7 Hz, 1H), 6.46 (d, J = 0.8 Hz, 1H), 6.14 (s, 1H), 5.12 (s, 2H), 4.18 (s, 3H), 3.54 (s, 3H), 3.41 (s, 3H), 2.85 (d, J = 4.9 Hz, 3H); m/z N.D. |
| 168 | 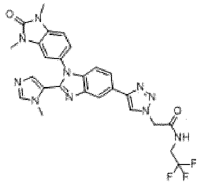 | 2-(4-(1',3'-dimethyl-2-(1-methyl-1H-imidazol-5-yl)-2'-oxo-2',3'-dihydro-1'H-[1,5'-bibenzo[d]imidazol]-5-yl)-1H-1,2,3-triazol-1-yl)-N-(2,2,2-trifluoroethyl)acetam ide | ¹H-NMR (500 MHz, CHLOROFORM-d) δ 8.20 (d, J = 0.9 Hz, 1H), 7.95 (s, 1H), 7.81 (dd, J = 8.4, 1.5 Hz, 1H), 7.60 (s, 1H), 7.14 (dd, J = 8.2, 1.5 Hz, 2H), 7.09 (dd, J = 8.2, 1.9 Hz, 1H), 6.94 (d, J = 1.7 Hz, 1H), 6.44 (d, J = 0.8 Hz, 1H), 5.21 (s, 2H), 4.17 (s, 3H), 3.99 - 3.91 (m, 2H), 3.55 (s, 3H), 3.41 (s, 3H); m/z N.D. |
| 169 | 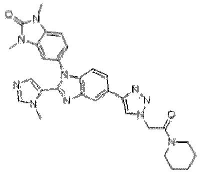 | 1',3'-dimethyl-2-(1-methyl-1H-imidazol-5-yl)-5-(1-(2-oxo-2-(piperidin-1-yl)ethyl)-1H-1,2,3-triazol-4-yl)-1',3'-dihydro-2'H-[1,5'-bibenzo[d]imidazol]-2'-one | ¹H-NMR (500 MHz, Methanol-d₄) δ 8.33 (s, 1H), 8.26 (d, J = 0.9 Hz, 1H), 7.82 (dd, J = 8.5, 1.6 Hz, 1H), 7.80 (s, 1H), 7.37 (d, J = 8.2 Hz, 1H), 7.35 (d, J = 1.9 Hz, 1H), 7.26 (d, J = 8.5 Hz, 1H), 7.22 (dd, J = 8.2, 2.0 Hz, 1H), 6.60 (s, 1H), 5.53 (s, 2H), 4.06 (s, 3H), 3.63 - 3.58 (m, 4H), 3.52 (s, 3H), 3.42 (s, 3H), 1.73 (s, 4H), 1.61 (s, 2H); m/z N.D. |
| 170 | 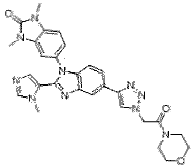 | 1',3'-dimethyl-2-(1-methyl-1H-imidazol-5-yl)-5-(1-(2-morpholino-2-oxoethyl)-1H-1,2,3-triazol-4-yl)-1',3'-dihydro-2'H-[1,5'-bibenzo[d]imidazol]-2'-one | ¹H-NMR (500 MHz, Methanol-d₄) δ 8.34 (s, 1H), 8.26 (d, J = 0.9 Hz, 1H), 8.11 (s, 1H), 7.82 (dd, J = 8.5, 1.5 Hz, 1H), 7.37 (d, J = 8.3 Hz, 1H), 7.35 (d, J = 1.8 Hz, 1H), 7.27 (d, J = 8.4 Hz, 1H), 7.23 (dd, J = 8.2, 1.9 Hz, 1H), 6.61 (s, 1H), 5.56 (s, 2H), 4.06 (s, 3H), 3.80 - 3.76 (m, 2H), 3.74 - 3.69 (m, 2H), 3.68 - 3.61 (m, 4H), 3.52 (s, 3H), 3.42 (s, 3H); m/z N.D. |
| 171 | 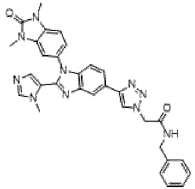 | N-benzyl-2-(4-(1',3'-dimethyl-2-(1-methyl-1H-imidazol-5-yl)-2'-oxo-2',3'-dihydro-1'H-[1,5'-bibenzo[d]imidazol]-5-yl)-1H-1,2,3-triazol-1-yl)acetamide | ¹H-NMR (500 MHz, CHLOROFORM-d) δ 8.21 (s, 1H), 7.97 (d, J = 1.5 Hz, 1H), 7.83 - 7.75 (m, 1H), 7.51 (s, 1H), 7.31 - 7.21 (m, 5H), 7.12 (s, 3H), 6.96 (s, 1H), 6.76 (d, J = 62.4 Hz, 1H), 6.46 (s, 1H), 5.14 (s, 2H), 4.46 (dd, J = 5.4, 3.0 Hz, 2H), 4.15 (s, 3H), 3.51 (s, 3H), 3.40 (s, 3H); m/z N.D. |
| 172 | 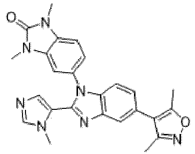 | 5-(3,5-dimethylisoxazol-4-yl)-1',3'-dimethyl-2-(1-methyl-1H-imidazol-5-yl)-1',3'-dihydro-2'H-[1,5'-bibenzo[d]imidazol]-2'-one | ¹H-NMR (500 MHz, CHLOROFORM-d) δ 8.88 (s, 1H), 7.78 (s, 1H), 7.24 - 7.19 (m, 3H), 7.12 (d, J = 7.9 Hz, 1H), 6.98 (s, 1H), 6.73 (s, 1H), 4.41 (s, 3H), 3.57 (s, 3H), 3.45 (s, 3H), 2.75 (s, 3H), 2.44 (d, J = 15.3 Hz, 3H); m/z N.D. |
| 173 | 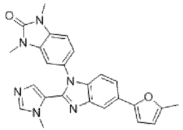 | 1',3'-dimethyl-2-(1-methyl-1H-imidazol-5-yl)-5-(5-methylfuran-2-yl)-1',3'-dihydro-2'H-[1,5'-bibenzo[d]imidazol]-2'-one | ¹H-NMR (500 MHz, CHLOROFORM-d) δ 8.09 (d, J = 1.0 Hz, 1H), 7.56 (dd, J = 8.5, 1.5 Hz, 1H), 7.54 (s, 1H), 7.12 (d, J = 4.5 Hz, 2H), 7.04 (d, J = 8.5 Hz, 1H), 6.95 (d, J = 6.3 Hz, 1H), 6.55 (d, J = 3.1 Hz, 1H), 6.46 (d, J = 7.5 Hz, 1H), 6.08 (dd, J = 3.1, 1.0 Hz, 1H), 4.18 (s, 3H), 3.52 (s, 3H), 3.41 (s, 3H), 2.40 (s, 3H); m/z N.D. |
| 174 | 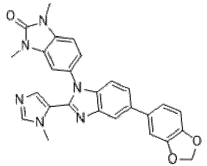 | 5-(benzo[d][1,3]dioxol -5-yl)-1',3'-dimethyl-2-(1-methyl-1H-imidazol-5-yl)-1',3'-dihydro-2'H-[1,5'-bibenzo[d]imidazol]-2'-one | ¹H-NMR (500 MHz, CHLOROFORM-d) δ 7.96 (d, J = 1.3 Hz, 1H), 7.59 (s, 1H), 7.42 (dt, J = 14.2, 7.1 Hz, 1H), 7.13 (ddd, J = 12.9, 8.2, 5.3 Hz, 5H), 6.96 (s, 1H), 6.91 (d, J = 8.0 Hz, 1H), 6.48 (s, 1H), 6.02 (s, 2H), 4.19 (s, 3H), 3.53 (s, J = 2.8 Hz, 3H), 3.42 (s, 3H); m/z N.D. |
| 175 | 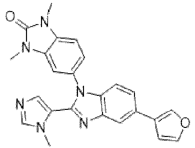 | 5-(furan-3-yl)-1',3'-dimethyl-2-(1-methyl-1H-imidazol-5-yl)-1',3'-dihydro-2'H-[1,5'-bibenzo[d]imidazol]-2'-one | ¹H-NMR (500 MHz, CHLOROFORM-d) δ 7.94 (d, J = 1.1 Hz, 1H), 7.79 - 7.75 (m, 1H), 7.55 (s, 1H), 7.51 (t, J = 1.7 Hz, 1H), 7.40 (dd, J = 8.4, 1.6 Hz, 1H), 7.14 - 7.11 (m, 2H), 7.07 (d, J = 8.4 Hz, 1H), 6.95 (s, 1H), 6.78 (dd, J = 1.8, 0.9 Hz, 1H), 6.46 (s, 1H), 4.18 (s, 3H), 3.52 (s, 3H), 3.41 (s, 3H); m/z N.D. |
| 176 | 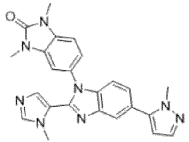 | 1',3'-dimethyl-2-(1-methyl-1H-imidazol-5-yl)-5-(1-methyl-1H-pyrazol-5-yl)-1',3'-dihydro-2'H-[1,5'-bibenzo[d]imidazol]-2'-one | ¹H-NMR (500 MHz, Methanol-d₄) δ 7.89 (d, J = 1.0 Hz, 1H), 7.80 (s, 1H), 7.53 (d, J = 2.0 Hz, 1H), 7.42 (dd, J = 8.4, 1.6 Hz, 1H), 7.38 (d, J = 8.2 Hz, 1H), 7.35 (d, J = 1.9 Hz, 1H), 7.30 (d, J = 8.4 Hz, 1H), 7.23 (dd, J = 8.2, 2.0 Hz, 1H), 6.59 (s, 1H), 6.42 (d, J = 2.0 Hz, 1H), 4.07 (s, 3H), 3.92 (s, 3H), 3.52 (s, 3H), 3.42 (s, 3H); m/z N.D. |

### Experimental Example 1. CBP Inhibitory Activity

CBP inhibitory activity was measured using a time resolved fluorescence resonance energy transfer (TR-FRET) experiment to verify the activity of CBP bromodomain.

The assay was performed in a final volume of 14 µl, optimized based on a CREBBP time resolved fluorescence resonance energy transfer (TR-FRET) assay kit (BPS Bioscience, cat. 32619) to maximize an assay window, and performed in a final volume of 14 µl, while maximizing the assay window. A TR-FRET fluorescence acceptor and a Tb donor were diluted in an assay buffer containing 2-[4-(2-hydroxyethyl)piperazin-1-yl]ethanesulfonic acid (hereafter, referred to as an assay buffer, BPS Bioscience, cat. 33012), and 3 µl of each solution was transferred to all wells of a white 384-low-volume well plate (Corning, cat. 4513). 2 µl of 4X Ac-H4 peptide (final concentration 85 nM) was transferred to high control wells and test wells. Each compound was prepared at 10 concentration points by serially diluting 3-fold from the highest concentration in a dimethyl sulfoxide solvent. The compound at each concentration was then diluted in the assay buffer to prepare a diluted compound solution that was 4-fold more concentrated than the final concentration. 2 µl of each compound or dimethyl sulfoxide prepared in this way was distributed into an assay plate so that the final test concentration was a minimum of 0.5 µM to a maximum of 10 µM (final dimethyl sulfoxide concentration 1%). 6 µL of 1X assay buffer was added to low control wells and 2 µl was added to high control and test wells. Then, 2 µl of 4X CBP BRD protein (final concentration 7.5 nM, BPS bioscience, cat 31128) was added to initiate a PPI reaction. The plate was reacted at room temperature for 2 hours before measuring the signal. The TR-FRET signal was measured using an EnVision Multilabel plate reader (PerkinElmer; Mirror LNACE/DELFIA Dual/Bias).

For data analysis, the values of each well were obtained using a TR-FRET ratio ((Signal 665 nm/Signal 615 nm)X20000. Data analysis was performed using the TR-FRET ratio ((Signal 665 nm/Signal 615 nm)X20000. Data were reported as percent inhibition compared to control wells based on the following equation: %Activity = ((FRETcompound - FRETLow av)/(FRETHigh av - FRETLow av))X100. IC50 analysis was determined by standard 4 parameter curve fitting according to an algorithm using GraphPad Prism 7.0 (GraphPad Software Inc., La Jolla, CA, USA). All data are expressed as mean ± standard error of the mean (SEM).

### Experimental Example 2. BRD4 Inhibitory Activity

BRD4 inhibitory activity was measured using a time resolved fluorescence resonance energy transfer (TR-FRET) experiment to verify the activity of BRD4 bromodomain.

The assay was performed in a final volume of 14 µl, optimized based on a CREBBP time resolved fluorescence resonance energy transfer (TR-FRET) assay kit (BPS Bioscience, cat. 32613) to maximize an assay window, and performed in a final volume of 14 µl, while maximizing the assay window. A TR-FRET fluorescence acceptor and a Tb donor were diluted in an assay buffer containing 2-[4-(2-hydroxyethyl)piperazin-1-yl]ethanesulfonic acid (hereafter, referred to as an assay buffer, BPS Bioscience, cat. 33012), and 3 µl of each solution was transferred to all wells of a white 384-low-volume well plate (Corning, cat. 4513). 2 µl of 4X Ac-H4 peptide (final concentration 15 nM) was transferred to high control wells and test wells. Each compound was prepared at 10 concentration points by serially diluting 3-fold from the highest concentration in a dimethyl sulfoxide solvent. The compound at each concentration was then diluted in the assay buffer to prepare a diluted compound solution that was 4-fold more concentrated than the final concentration. 2 µl of each compound or dimethyl sulfoxide prepared in this way was distributed into an assay plate so that the final test concentration was a minimum of 0.5 µM to a maximum of 10 µM (final dimethyl sulfoxide concentration 1%). 6 µL of 1X assay buffer was added to low control wells and 2 µl was added to high control and test wells. Then, 2 µl of 4X BRD4 (BD1) protein (final concentration 7.5 nM, BPS bioscience, cat 31040) was added to initiate a PPI reaction. The plate was reacted at room temperature for 2 hours before measuring the signal. The TR-FRET signal was measured using an EnVision Multilabel plate reader (PerkinElmer; Mirror LNACE/DELFIA Dual/Bias). For data analysis, the values of each well were obtained using a TR-FRET ratio ((Signal 665 nm/Signal 615 nm) X20000. Data analysis was performed using the TR-FRET ratio ((Signal 665 nm/Signal 615 nm) X20000. Data were reported as percent inhibition compared to control wells based on the following equation: %Activity = ((FRETcompound - FRETLow av)/(FRETHigh av - FRETLow av)) X100. IC50 analysis was determined by standard 4 parameter curve fitting according to an algorithm using GraphPad Prism 7.0 (GraphPad Software Inc., La Jolla, CA, USA). All data are expressed as mean ± standard error of the mean (SEM).

The results according to Experimental Examples 1 and 2 are shown in Tables 2 and 3 below.

In Tables 2 and 3 below, a case where the activity value is 0 to 500 nM is indicated as "+++", a case with greater than 500 and 5 µM or less is indicated as "++", and a case with greater than 5 µM is indicated as "+."

**[Table 2]**

| **Example** | **CBP BRD IC₅₀** | **Example** | **CBP BRD IC₅₀** |
|---|---|---|---|
| 1 | + | 89 | + |
| 2 | + | 90 | ++ |
| 3 | + | 91 | + |
| 4 | + | 92 | + |
| 5 | + | 93 | + |
| 6 | + | 94 | + |
| 7 | ++ | 95 | + |
| 8 | + | 96 | + |
| 9 | ++ | 97 | ++ |
| 10 | ++ | 98 | + |
| 11 | ++ | 99 | +++ |
| 12 | + | 100 | + |
| 13 | + | 101 | ++ |
| 14 | ++ | 102 | +++ |
| 15 | ++ | 103 | + |
| 16 | ++ | 104 | + |
| 17 | ++ | 105 | ++ |
| 18 | ++ | 106 | + |
| 19 | + | 107 | + |
| 20 | ++ | 108 | ++ |
| 21 | +++ | 109 | ++ |
| 22 | ++ | 110 | +++ |
| 23 | ++ | 111 | ++ |
| 24 | ++ | 112 | ++ |
| 25 | +++ | 113 | +++ |
| 26 | ++ | 114 | ++ |
| 27 | ++ | 115 | ++ |
| 28 | +++ | 116 | ++ |
| 29 | ++ | 117 | ++ |
| 30 | ++ | 118 | ++ |
| 31 | ++ | 119 | ++ |
| 32 | ++ | 120 | ++ |
| 33 | ++ | 121 | +++ |
| 34 | ++ | 122 | ++ |
| 35 | +++ | 123 | ++ |
| 36 | ++ | 124 | ++ |
| 37 | ++ | 125 | + |
| 38 | +++ | 126 | ++ |
| 39 | +++ | 127 | +++ |
| 40 | +++ | 128 | ++ |
| 41 | +++ | 129 | + |
| 42 | +++ | 130 | +++ |
| 43 | +++ | 131 | +++ |
| 44 | +++ | 132 | ++ |
| 45 | ++ | 133 | ++ |
| 46 | +++ | 134 | ++ |
| 47 | +++ | 135 | ++ |
| 48 | +++ | 136 | ++ |
| 49 | ++ | 137 | +++ |
| 50 | ++ | 138 | +++ |
| 51 | ++ | 139 | ++ |
| 52 | ++ | 140 | + |
| 53 | +++ | 141 | ++ |
| 54 | +++ | 142 | ++ |
| 55 | +++ | 143 | ++ |
| 56 | +++ | 144 | +++ |
| 57 | ++ | 145 | ++ |
| 58 | +++ | 146 | ++ |
| 59 | +++ | 147 | +++ |
| 60 | +++ | 148 | + |
| 61 | +++ | 149 | ++ |
| 62 | +++ | 150 | ++ |
| 63 | +++ | 151 | + |
| 64 | +++ | 152 | ++ |
| 65 | +++ | 153 | +++ |
| 66 | +++ | 154 | +++ |
| 67 | +++ | 155 | ++ |
| 68 | +++ | 156 | ++ |
| 69 | +++ | 157 | + |
| 70 | ++ | 158 | + |
| 71 | ++ | 159 | +++ |
| 72 | ++ | 160 | +++ |
| 73 | +++ | 161 | ++ |
| 74 | +++ | 162 | ++ |
| 75 | +++ | 163 | +++ |
| 76 | +++ | 164 | ++ |
| 77 | ++ | 165 | ++ |
| 78 | +++ | 166 | ++ |
| 79 | ++ | 167 | ++ |
| 80 | +++ | 168 | ++ |
| 81 | +++ | 169 | ++ |
| 82 | ++ | 170 | ++ |
| 83 | +++ | 171 | ++ |
| 84 | ++ | 172 | ++ |
| 85 | ++ | 173 | ++ |
| 86 | ++ | 174 | +++ |
| 87 | + | 175 | ++ |
| 88 | + | 176 | ++ |

**[Table 3]**

| **Example** | BRD4 (BD1) IC₅₀ | **Example** | BRD4 (BD1) IC₅₀ |
|---|---|---|---|
| 1 | + | 89 | + |
| 2 | + | 90 | + |
| 3 | N.D | 91 | + |
| 4 | N.D | 92 | + |
| 5 | N.D | 93 | + |
| 6 | N.D | 94 | + |
| 7 | + | 95 | + |
| 8 | N.D | 96 | + |
| 9 | + | 97 | + |
| 10 | + | 98 | + |
| 11 | + | 99 | + |
| 12 | N.D | 100 | + |
| 13 | N.D | 101 | + |
| 14 | + | 102 | + |
| 15 | + | 103 | + |
| 16 | + | 104 | + |
| 17 | ++ | 105 | + |
| 18 | + | 106 | + |
| 19 | + | 107 | + |
| 20 | + | 108 | + |
| 21 | + | 109 | + |
| 22 | + | 110 | + |
| 23 | + | 111 | + |
| 24 | + | 112 | + |
| 25 | ++ | 113 | + |
| 26 | + | 114 | + |
| 27 | + | 115 | + |
| 28 | + | 116 | + |
| 29 | + | 117 | + |
| 30 | + | 118 | + |
| 31 | + | 119 | + |
| 32 | + | 120 | + |
| 33 | + | 121 | + |
| 34 | + | 122 | + |
| 35 | + | 123 | + |
| 36 | + | 124 | + |
| 37 | ++ | 125 | ++ |
| 38 | ++ | 126 | + |
| 39 | ++ | 127 | + |
| 40 | + | 128 | + |
| 41 | ++ | 129 | + |
| 42 | ++ | 130 | ++ |
| 43 | + | 131 | + |
| 44 | + | 132 | + |
| 45 | + | 133 | + |
| 46 | + | 134 | + |
| 47 | + | 135 | + |
| 48 | + | 136 | + |
| 49 | + | 137 | + |
| 50 | + | 138 | + |
| 51 | + | 139 | + |
| 52 | + | 140 | + |
| 53 | + | 141 | ++ |
| 54 | ++ | 142 | + |
| 55 | + | 143 | + |
| 56 | + | 144 | + |
| 57 | + | 145 | + |
| 58 | + | 146 | + |
| 59 | + | 147 | + |
| 60 | + | 148 | + |
| 61 | ++ | 149 | + |
| 62 | ++ | 150 | + |
| 63 | + | 151 | + |
| 64 | + | 152 | + |
| 65 | ++ | 153 | + |
| 66 | + | 154 | + |
| 67 | ++ | 155 | + |
| 68 | ++ | 156 | + |
| 69 | + | 157 | + |
| 70 | + | 158 | + |
| 71 | + | 159 | + |
| 72 | ++ | 160 | + |
| 73 | + | 161 | + |
| 74 | ++ | 162 | + |
| 75 | ++ | 163 | + |
| 76 | + | 164 | + |
| 77 | + | 165 | + |
| 78 | ++ | 166 | + |
| 79 | ++ | 167 | + |
| 80 | + | 168 | + |
| 81 | + | 169 | + |
| 82 | + | 170 | + |
| 83 | + | 171 | + |
| 84 | + | 172 | + |
| 85 | + | 173 | + |
| 86 | + | 174 | + |
| 87 | + | 175 | + |
| 88 | + | 176 | + |

## Claims

1. A compound represented by the following Formula I: 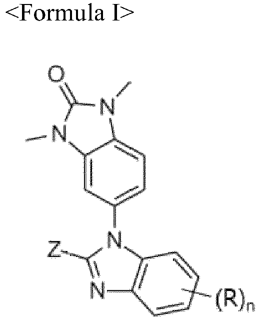 where
n is an integer from 0 to 3, where if n is an integer of 2 or more, Rs are the same or different, and Rs are each independently NR^{a}R^{b}, SO₂NHR^{c}, OR^{d}, CONR^{e}R^{f}, halogen, cyano, amidinyl, imine, C₁₋₈ alkyl, C₃₋₁₀ cycloalkyl, C₆₋₁₀ aryl, 5- to 13-membered heteroaryl, 3- to 14-membered heterocycloalkyl, or 4- to 9-membered heterocycloalkenyl, and R^{a} to R^{f} are each independently selected from the group consisting of hydrogen, C₁₋₄ alkyl, C₃₋₁₀ cycloalkyl and C₆₋₁₀ aryl, and
Z is C₁₋₈ alkyl, C₃₋₁₀ cycloalkyl, C₁₋₅ haloalkyl, C₆₋₁₀ aryl, 5- to 13-membered heteroaryl or 3- to 14-membered heterocycloalkyl,
where R, R^{a} to R^{f} and Z are each substituted or unsubstituted with Substituent Group I:
Substituent Group I: substituted or unsubstituted C₁₋₁₀ alkyl, substituted or unsubstituted C₂₋₁₀ alkenyl, substituted or unsubstituted C₃₋₂₀ carbocycle, substituted or unsubstituted C₃₋₂₀ cycloalkyl, substituted or unsubstituted C₆₋₃₀ aryl, substituted or unsubstituted 3- to 30-membered heterocycle, substituted or unsubstituted C₁₋₁₀ alkoxy, substituted or unsubstituted C₂₋₁₀ alkenyloxy, substituted or unsubstituted C₃₋₁₀ cycloalkyloxy, substituted or unsubstituted C₆₋₃₀ aryloxy, substituted or unsubstituted 3-to 30-membered heterocycleoxy, substituted or unsubstituted C₁₋₁₀ alkylketone, substituted or unsubstituted C₂₋₁₀ alkenylketone, substituted or unsubstituted C₃₋₁₀ cycloalkylketone, substituted or unsubstituted C₆₋₃₀ arylketone, substituted or unsubstituted 3- to 30-membered heterocycleketone, substituted or unsubstituted C₁₋₁₀ alkylthio, substituted or unsubstituted C₂₋₁₀ alkenylthio, substituted or unsubstituted C₃₋₁₀ cycloalkylthio, substituted or unsubstituted C₆₋₃₀ arylthio, substituted or unsubstituted 3- to 30-membered heterocyclethio, aldehyde, carboxy, halogen, substituted or unsubstituted C₁₋₁₀ haloalkyl, hydroxy, substituted or unsubstituted amino, imine, cyano, nitro, amide, thiol, sulfone and phosphoric acid,
where a C₁₋₁₀ alkyl moiety is interposed by a heteroatom group selected from - O-, -S-, -SO-, -SO₂-, -NR^{g}- (R^{g} is hydrogen or substituted or unsubstituted C₁₋₁₀ alkyl), - N=, =N-, -POR^{g}- and -PO₄R^{g}-, and a heterocycle moiety comprises at least one of S, N, P and O as a ring-forming atom.

2. The compound according to claim 1, wherein R, R^{a} to R^{f} and Z are each substituted or unsubstituted with Substituent Group I-1:
Substituent Group I-1: substituted or unsubstituted C₁₋₈ alkyl, substituted or unsubstituted C₃₋₈ cycloalkyl, hydroxy, substituted or unsubstituted C₁₋₃ alkoxy, substituted or unsubstituted C₁₋₄ haloalkyl, substituted or unsubstituted C₆₋₈ aryl, substituted or unsubstituted 3- to 8-membered heteroaryl, substituted or unsubstituted cyano, substituted or unsubstituted amino, substituted or unsubstituted amide, aldehyde, halogen, CO₂R^{p1}, NHCOR^{p2}, NHCO₂R^{p3} or NHSO₂R^{p4}, where R^{p1} to R^{p4} are each independently hydrogen, substituted or unsubstituted methyl, substituted or unsubstituted ethyl, substituted or unsubstituted butyl or substituted or unsubstituted phenyl.

3. The compound according to claim 1, wherein the compound of Formula I is represented by the following Formula I-1: 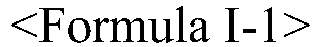 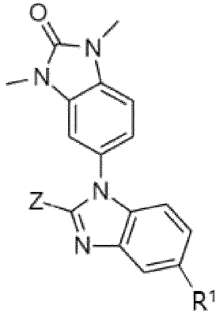 where
R¹ is NR^{a}R^{b}, SO₂NHR^{c}, OR^{d}, CONR^{e}R^{f}, halogen, cyano or imine, and is substituted or unsubstituted with Substituent Group I, and
R^{a} to R^{f} and Z are the same as defined in claim 1.

4. The compound according to claim 3, wherein
R¹ is NR^{a}R^{b}, SO₂NHR^{c}, OR^{d}, CONR^{e}R^{f}, bromo, cyano or amidinyl,
where R^{a} to R^{f} are each independently hydrogen, substituted or unsubstituted methyl, substituted or unsubstituted ethyl, substituted or unsubstituted propyl, substituted or unsubstituted cyclopropyl, substituted or unsubstituted cyclobutyl or substituted or unsubstituted phenyl, and
R¹ is substituted or unsubstituted with Substituent Group I-1-1:
Substituent Group I-1-1: substituted or unsubstituted C₁₋₃ alkyl, substituted or unsubstituted C₃₋₅ cycloalkyl, and hydroxy.

5. The compound according to claim 1, wherein the compound of Formula I is represented by the following Formula I-2: 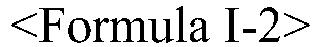 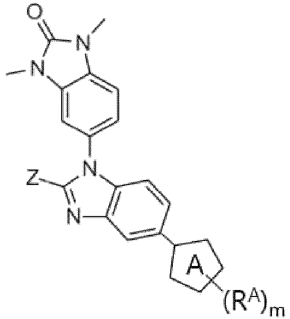 where 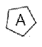 is C₅ cycloalkyl, 5-membered heteroaryl or 5-membered heterocycloalkyl,
m is an integer from 0 to 3, where if m is an integer of 2 or more, R^{A}s are the same or different, and R^{A} is substituted or unsubstituted C₁₋₆ alkyl, substituted or unsubstituted C₁₋₄ haloalkyl, substituted or unsubstituted cyano, substituted or unsubstituted amide, substituted or unsubstituted amino, or aldehyde, and
Z is the same as defined in claim 1.

6. The compound according to claim 5, wherein 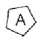 is furanyl, isoxazolyl, oxadiazolonyl, pyrazolyl, thiophenyl, triazolyl, thiazolyl or thiazolinyl.

7. The compound according to claim 1, wherein the compound of Formula I is represented by the following Formula I-3: 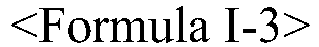 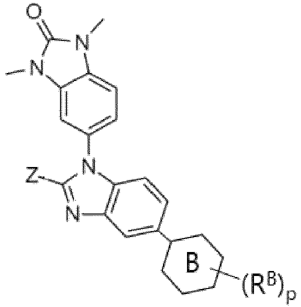 where 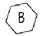 is C₅ cycloalkyl, C₆ aryl, 6-membered heteroaryl, 6-membered heterocycloalkyl or 6-membered heterocycloalkenyl,
p is an integer from 0 to 3, where if p is an integer of 2 or more, R^{B}s are the same or different, and R^{B} is substituted or unsubstituted C₁₋₅ alkyl, substituted or unsubstituted C₁₋₃ alkoxy, substituted or unsubstituted amide, substituted or unsubstituted amino, halogen, cyano, hydroxy, aldehyde, CO₂R^{h}, NHCORⁱ, NHCO₂R^{j} or NHSO₂R^{k}, where R^{h} to R^{k} are each independently hydrogen, substituted or unsubstituted methyl, substituted or unsubstituted butyl or substituted or unsubstituted phenyl, and
Z is the same as defined in claim 1.

8. The compound according to claim 7, wherein 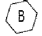 is phenyl, pyridonyl, pyridinyl, pyrimidinyl, piperidinyl or tetrahydropyridinyl.

9. The compound according to claim 1, wherein the compound of Formula I is represented by the following Formula I-4: 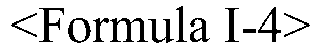 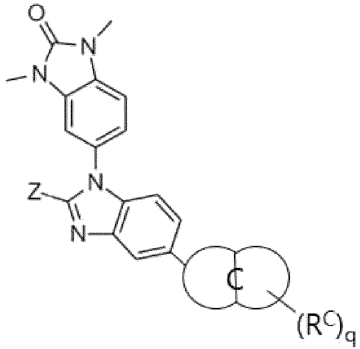 where
q is an integer from 0 to 3, where if q is an integer of 2 or more, R^{C}s are the same or different, 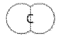 is C₉ cycloalkyl, C₁₀ cycloalkyl, C₉ aryl, C₁₀ aryl, 9-membered heteroaryl, 10-membered heteroaryl, 9-membered heterocycloalkyl or 10-membered heterocycloalkyl,
R^{C} is substituted or unsubstituted C₁₋₈ alkyl, substituted or unsubstituted C₃₋₇ cycloalkyl, halogen or cyano, and
Z is the same as defined in claim 1.

10. The compound according to claim 9, wherein 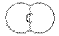 is benzimidazolyl, benzodioxolyl, benzodioxanyl or dihydrobenzofuranyl, and q is 0.

11. The compound according to claim 1, wherein
Z is methyl, ethyl, propyl, butyl, cyclopropyl, cyclohexyl, trifluoromethyl, phenyl, furanyl, imidazolyl, isoxazolyl, oxazolyl, pyrazolyl, pyrrolyl, thiazolyl, thiophenyl, pyridinyl, pyrimidinyl, azaindolyl, indolyl, piperidinyl, tetrahydropyranyl, lactamyl or tetralinyl, and
Z is substituted or unsubstituted with Substituent Group I-1-2:
Substituent Group I-1-2: substituted or unsubstituted C₁₋₃ alkyl, substituted or unsubstituted C₃₋₈ cycloalkyl, substituted or unsubstituted C₁₋₃ haloalkyl, halogen, hydroxy, substituted or unsubstituted C₁₋₃ alkoxy, substituted or unsubstituted C₆₋₈ aryl, substituted or unsubstituted 3- to 8-membered heteroaryl, substituted or unsubstituted cyano, substituted or unsubstituted amino, substituted or unsubstituted amide, CO₂R^{l}, or NHCO₂R^{m}, where R^{l} and R^{m} are each independently hydrogen, substituted or unsubstituted methyl, substituted or unsubstituted ethyl, substituted or unsubstituted butyl, or substituted or unsubstituted phenyl.

12. The compound according to claim 1, being any one selected from the following group:
1-(1,3-dimethyl-2-oxo-benzimidazol-5-yl)-N-methyl-2-(3-methylimidazol-4-yl)benzimidazole-5-sulfonamide;
1-(1,3-dimethyl-2-oxo-benzimidazol-5-yl)-N-methyl-2-(6-methyl-1H-indol-3-yl)benzimidazole-5-sulfonamide;
1-(1,3-dimethyl-2-oxo-benzimidazol-5-yl)-N-methyl-2-(3-phenylimidazol-4-yl)benzimidazole-5-sulfonamide;
2-[3-(3,4-difluorophenyl)imidazol-4-yl]-1-(1,3-dimethyl-2-oxo-benzimidazol-5-yl)-N-methyl-benzimidazole-5-sulfonamide;
1-(1,3-dimethyl-2-oxo-benzimidazol-5-yl)-N-methyl-2-(6-oxo-2-piperidyl)benzimidazole-5-sulfonamide;
2-(6-bromo-1H-indol-3-yl)-1-(1,3-dimethyl-2-oxo-benzimidazol-5-yl)-N-methyl-benzimidazole-5-sulfonamide;
1,3-dimethyl-5-[2-(3-methylimidazol-4-yl)-5-phenyl-benzimidazol-1-yl]benzimidazol-2-one;
5-[5-bromo-2-(6-methyl-1H-indol-3-yl)benzimidazol-1-yl]-1,3-dimethyl-benzimidazol-2-one;
1,3-dimethyl-5-[2-(6-methyl-1H-indol-3-yl)-5-(1-methylpyrazol-4-yl)benzimidazol-1-yl]benzimidazol-2-one;
1-(1,3-dimethyl-2-oxo-benzimidazol-5-yl)-N-methyl-2-(6-methyl-1H-indol-3-yl)benzimidazole-5-carboxamide;
1-(1,3-dimethyl-2-oxo-benzimidazol-5-yl)-N-methyl-2-(3-methylimidazol-4-yl)benzimidazole-5-carboxamide;
1-(1,3-dimethyl-2-oxo-benzimidazol-5-yl)-N-methyl-2-(6-oxo-2-piperidyl)benzimidazole-5-carboxamide;
tert-butyl 4-[1-(1,3-dimethyl-2-oxo-benzimidazol-5-yl)-2-(6-methyl-1H-indol-3-yl)benzimidazol-5-yl]piperidine-1-carboxylate;
1,3-dimethyl-5-[2-(6-methyl-1H-indol-3-yl)-5-(4-piperidyl)benzimidazol-1-yl]benzimidazol-2-one;
5-(1-ethylpiperidin-4-yl)-1',3'-dimethyl-2-(6-methyl-1H-indol-3-yl)-1',3'-dihydro-2'H-[1,5'-bibenzo[d]imidazol]-2'-one formate;
1',3'-dimethyl-2-(6-methyl-1H-indol-3-yl)-5-(1,2,3,6-tetrahydropyridin-4-yl)-1',3'-dihydro-2'H-[1,5'-bibenzo[d]imidazol]-2'-one formate;
1,3-dimethyl-5-[2-(3-methylimidazol-4-yl)-5-(1-methylpyrazol-4-yl)benzimidazol-1-yl]benzimidazol-2-one;
1,3-dimethyl-5-[2-(3-methylimidazol-4-yl)-5-[1-methyl-5-(trifluoromethyl)pyrazol-4-yl]benzimidazol-1-yl]benzimidazol-2-one;
4-[1-(1,3-dimethyl-2-oxo-benzimidazol-5-yl)-2-(3-methylimidazol-4-yl)benzimidazol-5-yl]-2-methyl-pyrazole-3-carbonitrile;
1,3-dimethyl-5-[2-(3-methylimidazol-4-yl)-5-(1-piperidyl)benzimidazol-1-yl]benzimidazol-2-one;
1,3-dimethyl-5-[2-(3-methylimidazol-4-yl)-5-(4-pyridyl)benzimidazol-1-yl]benzimidazol-2-one;
1,3-dimethyl-5-[2-(3-methylimidazol-4-yl)-5-(4-methyl-2-thienyl)benzimidazol-1-yl]benzimidazol-2-one;
1,3-dimethyl-5-[2-(3-methylimidazol-4-yl)-5-(3-pyridyl)benzimidazol-1-yl]benzimidazol-2-one;
1,3-dimethyl-5-[2-(3-methylimidazol-4-yl)-5-pyrimidin-5-yl-benzimidazol-1-yl]benzimidazol-2-one;
4-[1-(1,3-dimethyl-2-oxo-benzimidazol-5-yl)-2-(3-methylimidazol-4-yl)benzimidazol-5-yl]-2-fluoro-N-methyl-benzamide;
5-[5-(2-methoxyphenyl)-2-(3-methylimidazol-4-yl)benzimidazol-1-yl]-1,3-dimethyl-benzimidazol-2-one;
2-[1-(1,3-dimethyl-2-oxo-benzimidazol-5-yl)-2-(3-methylimidazol-4-yl)benzimidazol-5-yl]benzonitrile;
methyl 5-[1-(1,3-dimethyl-2-oxo-benzimidazol-5-yl)-2-(3-methylimidazol-4-yl)benzimidazol-5-yl]pyridine-2-carboxylate;
1,3-dimethyl-5-[2-(3-methylimidazol-4-yl)-5-(2-pyridyl)benzimidazol-1-yl]benzimidazol-2-one;
5-[1-(1,3-dimethyl-2-oxo-benzimidazol-5-yl)-2-(3-methylimidazol-4-yl)benzimidazol-5-yl]-N-methyl-pyridine-2-carboxamide;
5-[5-(2-fluorophenyl)-2-(3-methylimidazol-4-yl)benzimidazol-1-yl]-1,3-dimethyl-benzimidazol-2-one;
5-[5-(3-fluorophenyl)-2-(3-methylimidazol-4-yl)benzimidazol-1-yl]-1,3-dimethyl-benzimidazol-2-one;
5-[5-(4-fluorophenyl)-2-(3-methylimidazol-4-yl)benzimidazol-1-yl]-1,3-dimethyl-benzimidazol-2-one;
5-[5-(2-fluoro-4-pyridyl)-2-(3-methylimidazol-4-yl)benzimidazol-1-yl]-1,3-dimethyl-benzimidazol-2-one;
5-[5-(3-methoxyphenyl)-2-(3-methylimidazol-4-yl)benzimidazol-1-yl]-1,3-dimethyl-benzimidazol-2-one;
1,3-dimethyl-5-[2-(3-methylimidazol-4-yl)-5-(1-methyl-2-oxo-4-pyridyl)benzimidazol-1-yl]benzimidazol-2-one;
1,3-dimethyl-5-[2-(3-methylimidazol-4-yl)-5-(1-methyl-6-oxo-3-pyridyl)benzimidazol-1-yl]benzimidazol-2-one;
1,3-dimethyl-5-[2-(3-methylimidazol-4-yl)-5-(2-methyl-4-pyridyl)benzimidazol-1-yl]benzimidazol-2-one;
5-[5-(2-methoxy-4-pyridyl)-2-(3-methylimidazol-4-yl)benzimidazol-1-yl]-1,3-dimethyl-benzimidazol-2-one;
1,3-dimethyl-5-[2-(3-methylimidazol-4-yl)-5-(2-oxo-1H-pyridin-4-yl)benzimidazol-1-yl]benzimidazol-2-one;
1,3-dimethyl-5-[2-(3-methylimidazol-4-yl)-5-(6-oxo-1H-pyridin-3-yl)benzimidazol-1-yl]benzimidazol-2-one;
4-[1-(1,3-dimethyl-2-oxo-benzimidazol-5-yl)-2-(3-methylimidazol-4-yl)benzimidazol-5-yl]-N-methyl-benzamide;
methyl 4-[1-(1,3-dimethyl-2-oxo-benzimidazol-5-yl)-2-(3-methylimidazol-4-yl)benzimidazol-5-yl]benzoate;
4-[1-(1,3-dimethyl-2-oxo-benzimidazol-5-yl)-2-(3-methylimidazol-4-yl)benzimidazol-5-yl]benzoic acid;
methyl 4-[1-(1,3-dimethyl-2-oxo-benzimidazol-5-yl)-2-(3-methylimidazol-4-yl)benzimidazol-5-yl]-2-fluoro-benzoate;
4-[1-(1,3-dimethyl-2-oxo-benzimidazol-5-yl)-2-(3-methylimidazol-4-yl)benzimidazol-5-yl]-2-fluoro-benzoic acid;
5-[5-(4-methoxyphenyl)-2-(3-methylimidazol-4-yl)benzimidazol-1-yl]-1,3-dimethyl-benzimidazol-2-one;
N-[4-[1-(1,3-dimethyl-2-oxo-benzimidazol-5-yl)-2-(3-methylimidazol-4-yl)benzimidazol-5-yl]phenyl]acetamide;
2-[4-[1-(1,3-dimethyl-2-oxo-benzimidazol-5-yl)-2-(3-methylimidazol-4-yl)benzimidazol-5-yl]pyrazol-1-yl]acetamide;
5-[5-[1-(2-hydroxy-2-methyl-propyl)pyrazol-4-yl]-2-(3-methylimidazol-4-yl)benzimidazol-1-yl]-1,3-dimethyl-benzimidazol-2-one;
1,3-dimethyl-5-[2-(3-methylimidazol-4-yl)-5-[1-(2,2,2-trifluoroethyl)pyrazol-4-yl]benzimidazol-1-yl]benzimidazol-2-one;
5-[5-[1-(2,2-difluoroethyl)pyrazol-4-yl]-2-(3-methylimidazol-4-yl)benzimidazol-1-yl]-1,3-dimethyl-benzimidazol-2-one;
4-[1-(1,3-dimethyl-2-oxo-benzimidazol-5-yl)-2-(3-methylimidazol-4-yl)benzimidazol-5-yl]benzamide;
5-[5-(2-amino-4-pyridyl)-2-(3-methylimidazol-4-yl)benzimidazol-1-yl]-1,3-dimethyl-benzimidazol-2-one;
1,3-dimethyl-5-[2-(3-methylimidazol-4-yl)-5-(2-methylthiazol-5-yl)benzimidazol-1-yl]benzimidazol-2-one;
1,3-dimethyl-5-[2-(3-methylimidazol-4-yl)-5-(1-methylpyrazol-3-yl)benzimidazol-1-yl]benzimidazol-2-one;
5-[5-[1-(2-hydroxyethyl)pyrazol-4-yl]-2-(3-methylimidazol-4-yl)benzimidazol-1-yl]-1,3-dimethyl-benzimidazol-2-one;
5-[5-[4-(hydroxymethyl)phenyl]-2-(3-methylimidazol-4-yl)benzimidazol-1-yl]-1,3-dimethyl-benzimidazol-2-one;
5-[5-(3-hydroxyphenyl)-2-(3-methylimidazol-4-yl)benzimidazol-1-yl]-1,3-dimethyl-benzimidazol-2-one;
5-[5-(4-hydroxyphenyl)-2-(3-methylimidazol-4-yl)benzimidazol-1-yl]-1,3-dimethyl-benzimidazol-2-one;
5-[5-(3-fluoro-4-hydroxy-phenyl)-2-(3-methylimidazol-4-yl)benzimidazol-1-yl]-1,3-dimethyl-benzimidazol-2-one;
5-[5-(4-aminophenyl)-2-(3-methylimidazol-4-yl)benzimidazol-1-yl]-1,3-dimethyl-benzimidazol-2-one;
5-[5-(6-amino-3-pyridyl)-2-(3-methylimidazol-4-yl)benzimidazol-1-yl]-1,3-dimethyl-benzimidazol-2-one;
5-[5-(2-aminopyrimidin-5-yl)-2-(3-methylimidazol-4-yl)benzimidazol-1-yl]-1,3-dimethyl-benzimidazol-2-one;
2-chloro-4-[1-(1,3-dimethyl-2-oxo-benzimidazol-5-yl)-2-(3-methylimidazol-4-yl)benzimidazol-5-yl]-N-methyl-benzamide;
4-[1-(1,3-dimethyl-2-oxo-benzimidazol-5-yl)-2-(3-methylimidazol-4-yl)benzimidazol-5-yl]-N,N-dimethyl-benzamide;
5-[5-(3-aminophenyl)-2-(3-methylimidazol-4-yl)benzimidazol-1-yl]-1,3-dimethyl-benzimidazol-2-one;
3-[1-(1,3-dimethyl-2-oxo-benzimidazol-5-yl)-2-(3-methylimidazol-4-yl)benzimidazol-5-yl]-N-methyl-benzamide;
N-[3-[1-(1,3-dimethyl-2-oxo-benzimidazol-5-yl)-2-(3-methylimidazol-4-yl)benzimidazol-5-yl]phenyl]acetamide;
5-[5-anilino-2-(3-methylimidazol-4-yl)benzimidazol-1-yl]-1,3-dimethyl-benzimidazol-2-one;
1,3-dimethyl-5-[2-(3-methylimidazol-4-yl)-5-phenoxy-benzimidazol-1-yl]benzimidazol-2-one;
4-[1-(1,3-dimethyl-2-oxo-benzimidazol-5-yl)-2-(3-methylimidazol-4-yl)benzimidazol-5-yl]-2,6-difluoro-N-methyl-benzamide;
5-[5-[3-(dimethylamino)phenyl]-2-(3-methylimidazol-4-yl)benzimidazol-1-yl]-1,3-dimethyl-benzimidazol-2-one;
N-[4-[1-(1,3-dimethyl-2-oxo-benzimidazol-5-yl)-2-(3-methylimidazol-4-yl)benzimidazol-5-yl]-2-fluoro-phenyl]acetamide;
N-[4-[1-(1,3-dimethyl-2-oxo-benzimidazol-5-yl)-2-(3-methylimidazol-4-yl)benzimidazol-5-yl]phenyl]methanesulfonamide;
5-[5-(3,5-dimethoxyphenyl)-2-(3-methylimidazol-4-yl)benzimidazol-1-yl]-1,3-dimethyl-benzimidazol-2-one;
tert-butyl N-[4-[1-(1,3-dimethyl-2-oxo-benzimidazol-5-yl)-2-(3-methylimidazol-4-yl)benzimidazol-5-yl]phenyl]carbamate;
N-[4-[1-(1,3-dimethyl-2-oxo-benzimidazol-5-yl)-2-(3-methylimidazol-4-yl)benzimidazol-5-yl]phenyl]benzamide;
5-[1-(1,3-dimethyl-2-oxo-benzimidazol-5-yl)-2-(3-methylimidazol-4-yl)benzimidazol-5-yl]-2-fluoro-N-methyl-benzamide;
3-[1-(1,3-dimethyl-2-oxo-benzimidazol-5-yl)-2-(3-methylimidazol-4-yl)benzimidazol-5-yl]-4-fluoro-N-methyl-benzamide;
5-[5-[4-(dimethylamino)phenyl]-2-(3-methylimidazol-4-yl)benzimidazol-1-yl]-1,3-dimethyl-benzimidazol-2-one;
5-[5-(2-aminothiazol-5-yl)-2-(3-methylimidazol-4-yl)benzimidazol-1-yl]-1,3-dimethyl-benzimidazol-2-one;
5-[5-(1H-benzimidazol-5-yl)-2-(3-methylimidazol-4-yl)benzimidazol-1-yl]-1,3-dimethyl-benzimidazol-2-one;
4-[2-cyclopropyl-1-(1,3-dimethyl-2-oxo-benzimidazol-5-yl)benzimidazol-5-yl]-2-fluoro-N-methyl-benzamide;
4-[1-(1,3-dimethyl-2-oxo-benzimidazol-5-yl)-2-phenyl-benzimidazol-5-yl]-2-fluoro-N-methyl-benzamide;
1,3-dimethyl-5-[5-[4-(methylamino)phenyl]-2-(3-methylimidazol-4-yl)benzimidazol-1-yl]benzimidazol-2-one;
4-[1-(1,3-dimethyl-2-oxo-benzimidazol-5-yl)-2-isoxazol-5-yl-benzimidazol-5-yl]-2-fluoro-N-methyl-benzamide;
4-[2-cyclohexyl-1-(1,3-dimethyl-2-oxo-benzimidazol-5-yl)benzimidazol-5-yl]-2-fluoro-N-methyl-benzamide;
4-[1-(1,3-dimethyl-2-oxo-benzimidazol-5-yl)-2-(2-pyridyl)benzimidazol-5-yl]-2-fluoro-N-methyl-benzamide;
4-[1-(1,3-dimethyl-2-oxo-benzimidazol-5-yl)-2-(3-pyridyl)benzimidazol-5-yl]-2-fluoro-N-methyl-benzamide;
4-[1-(1,3-dimethyl-2-oxo-benzimidazol-5-yl)-2-(4-pyridyl)benzimidazol-5-yl]-2-fluoro-N-methyl-benzamide;
tert-butyl 4-[1-(1,3-dimethyl-2-oxo-benzimidazol-5-yl)-5-[3-fluoro-4-(methylcarbamoyl)phenyl]benzimidazol-2-yl]piperidine-1-carboxylate;
4-[1-(1,3-dimethyl-2-oxo-benzimidazol-5-yl)-2-(4-piperidyl)benzimidazol-5-yl]-2-fluoro-N-methyl-benzamide;
4-[1-(1,3-dimethyl-2-oxo-benzimidazol-5-yl)-2-tetrahydropyran-4-yl-benzimidazol-5-yl]-2-fluoro-N-methyl-benzamide;
4-[2-(cyclohexylmethyl)-1-(1,3-dimethyl-2-oxo-benzimidazol-5-yl)benzimidazol-5-yl]-2-fluoro-N-methyl-benzamide;
4-[2-benzyl-1-(1,3-dimethyl-2-oxo-benzimidazol-5-yl)benzimidazol-5-yl]-2-fluoro-N-methyl-benzamide;
4-[1-(1,3-dimethyl-2-oxo-benzimidazol-5-yl)-2-thiazol-5-yl-benzimidazol-5-yl]-2-fluoro-N-methyl-benzamide;
4-[1-(1,3-dimethyl-2-oxo-benzimidazol-5-yl)-2-(2-furyl)benzimidazol-5-yl]-2-fluoro-N-methyl-benzamide;
4-[1-(1,3-dimethyl-2-oxo-benzimidazol-5-yl)-2-(1-methylpyrrol-2-yl)benzimidazol-5-yl]-2-fluoro-N-methyl-benzamide;
4-[1-(1,3-dimethyl-2-oxo-benzimidazol-5-yl)-2-(2-thienyl)benzimidazol-5-yl]-2-fluoro-N-methyl-benzamide;
4-[1-(1,3-dimethyl-2-oxo-benzimidazol-5-yl)-2-(o-tolyl)benzimidazol-5-yl]-2-fluoro-N-methyl-benzamide;
4-[1-(1,3-dimethyl-2-oxo-benzimidazol-5-yl)-2-(2,3-dimethylphenyl)benzimidazol-5-yl]-2-fluoro-N-methyl-benzamide;
4-[1-(1,3-dimethyl-2-oxo-benzimidazol-5-yl)-2-oxazol-4-yl-benzimidazol-5-yl]-2-fluoro-N-methyl-benzamide;
4-[1-(1,3-dimethyl-2-oxo-benzimidazol-5-yl)-2-(1H-imidazol-5-yl)benzimidazol-5-yl]-2-fluoro-N-methyl-benzamide;
4-[1-(1,3-dimethyl-2-oxo-benzimidazol-5-yl)-2-(1-methylpyrazol-4-yl)benzimidazol-5-yl]-2-fluoro-N-methyl-benzamide;
4-[1-(1,3-dimethyl-2-oxo-benzimidazol-5-yl)-2-thiazol-4-yl-benzimidazol-5-yl]-2-fluoro-N-methyl-benzamide;
4-[1-(1,3-dimethyl-2-oxo-benzimidazol-5-yl)-2-oxazol-5-yl-benzimidazol-5-yl]-2-fluoro-N-methyl-benzamide;
4-[1-(1,3-dimethyl-2-oxo-benzimidazol-5-yl)-2-(3-thienyl)benzimidazol-5-yl]-2-fluoro-N-methyl-benzamide;
4-[1-(1,3-dimethyl-2-oxo-benzimidazol-5-yl)-2-(3-furyl)benzimidazol-5-yl]-2-fluoro-N-methyl-benzamide;
4-[1-(1,3-dimethyl-2-oxo-benzimidazol-5-yl)-2-(2-methyl-3-thienyl)benzimidazol-5-yl]-2-fluoro-N-methyl-benzamide;
4-[1-(1,3-dimethyl-2-oxo-benzimidazol-5-yl)-2-(2-methyl-3-furyl)benzimidazol-5-yl]-2-fluoro-N-methyl-benzamide;
4-[1-(1,3-dimethyl-2-oxo-benzimidazol-5-yl)-2-[3-(trifluoromethyl)phenyl]benzimidazol-5-yl]-2-fluoro-N-methyl-benzamide;
4-[1-(1,3-dimethyl-2-oxo-benzimidazol-5-yl)-2-tetralin-5-yl-benzimidazol-5-yl]-2-fluoro-N-methyl-benzamide;
4-[1-(1,3-dimethyl-2-oxo-benzimidazol-5-yl)-2-(4-methyl-3-pyridyl)benzimidazol-5-yl]-2-fluoro-N-methyl-benzamide;
4-[1-(1,3-dimethyl-2-oxo-benzimidazol-5-yl)-2-(2-methyl-3-pyridyl)benzimidazol-5-yl]-2-fluoro-N-methyl-benzamide;
4-[1-(1,3-dimethyl-2-oxo-benzimidazol-5-yl)-2-(5-methyl-3-pyridyl)benzimidazol-5-yl]-2-fluoro-N-methyl-benzamide;
4-[2-(4-amino-3-pyridyl)-1-(1,3-dimethyl-2-oxo-benzimidazol-5-yl)benzimidazol-5-yl]-2-fluoro-N-methyl-benzamide;
4-[1-(1,3-dimethyl-2-oxo-benzimidazol-5-yl)-2-(m-tolyl)benzimidazol-5-yl]-2-fluoro-N-methyl-benzamide;
4-[2-(3-chlorophenyl)-1-(1,3-dimethyl-2-oxo-benzimidazol-5-yl)benzimidazol-5-yl]-2-fluoro-N-methyl-benzamide;
4-[2-(2,3-dichlorophenyl)-1-(1,3-dimethyl-2-oxo-benzimidazol-5-yl)benzimidazol-5-yl]-2-fluoro-N-methyl-benzamide;
4-[1-(1,3-dimethyl-2-oxo-benzimidazol-5-yl)-2-(3-fluoro-2-methylphenyl)benzimidazol-5-yl]-2-fluoro-N-methyl-benzamide;
4-[1-(1,3-dimethyl-2-oxo-benzimidazol-5-yl)-2-pyrimidin-5-yl-benzimidazol-5-yl]-2-fluoro-N-methyl-benzamide;
methyl 2-[1-(1,3-dimethyl-2-oxo-benzimidazol-5-yl)-5-[3-fluoro-4-(methylcarbamoyl)phenyl]benzimidazol-2-yl]-2-methyl-propanoate;
4-[1-(1,3-dimethyl-2-oxo-benzimidazol-5-yl)-2-isopropyl-benzimidazol-5-yl]-2-fluoro-N-methyl-benzamide;
4-[1-(1,3-dimethyl-2-oxo-benzimidazol-5-yl)-2-(6-hydroxy-3-pyridyl)benzimidazol-5-yl]-2-fluoro-N-methyl-benzamide;
4-[1-(1,3-dimethyl-2-oxo-benzimidazol-5-yl)-2-(2-hydroxy-4-pyridyl)benzimidazol-5-yl]-2-fluoro-N-methyl-benzamide;
4-[2-(3-bromo-2-methyl-phenyl)-1-(1,3-dimethyl-2-oxo-benzimidazol-5-yl)benzimidazol-5-yl]-2-fluoro-N-methyl-benzamide;
4-[1-(1,3-dimethyl-2-oxo-benzimidazol-5-yl)-2-[2-methyl-3-(1-methylpyrazol-4-yl)phenyl]benzimidazol-5-yl]-2-fluoro-N-methyl-benzamide;
4-[2-[2-(dimethylamino)ethyl]-1-(1,3-dimethyl-2-oxo-benzimidazol-5-yl)benzimidazol-5-yl]-2-fluoro-N-methyl-benzamide;
4-[1-(1,3-dimethyl-2-oxo-benzimidazol-5-yl)-2-(1H-pyrrolo[2,3-b]pyridin-3-yl)benzimidazol-5-yl]-2-fluoro-N-methyl-benzamide;
4-[2-(3-cyclopropyl-2-methyl-phenyl)-1-(1,3-dimethyl-2-oxo-benzimidazol-5-yl)benzimidazol-5-yl]-2-fluoro-N-methyl-benzamide;
4-[2-[3-[2-(dimethylamino)ethylamino]-2-methyl-phenyl]-1-(1,3-dimethyl-2-oxo-benzimidazol-5-yl)benzimidazol-5-yl]-2-fluoro-N-methyl-benzamide;
4-[1-(1,3-dimethyl-2-oxo-benzimidazol-5-yl)-2-[2-methyl-3-(trifluoromethyl)phenyl]benzimidazol-5-yl]-2-fluoro-N-methyl-benzamide;
4-[1-(1,3-dimethyl-2-oxo-benzimidazol-5-yl)-2-(2-fluorophenyl)benzimidazol-5-yl]-2-fluoro-N-methyl-benzamide;
4-[2-(2-chlorophenyl)-1-(1,3-dimethyl-2-oxo-benzimidazol-5-yl)benzimidazol-5-yl]-2-fluoro-N-methyl-benzamide;
4-[1-(1,3-dimethyl-2-oxo-benzimidazol-5-yl)-2-[2-(trifluoromethyl)phenyl]benzimidazol-5-yl]-2-fluoro-N-methyl-benzamide;
4-[1-(1,3-dimethyl-2-oxo-benzimidazol-5-yl)-2-(3-methoxy-2-methylphenyl)benzimidazol-5-yl]-2-fluoro-N-methyl-benzamide;
4-[1-(1,3-dimethyl-2-oxo-benzimidazol-5-yl)-2-(2-methoxyphenyl)benzimidazol-5-yl]-2-fluoro-N-methyl-benzamide;
4-[1-(1,3-dimethyl-2-oxo-benzimidazol-5-yl)-2-(3-methoxyphenyl)benzimidazol-5-yl]-2-fluoro-N-methyl-benzamide;
4-[1-(1,3-dimethyl-2-oxo-benzimidazol-5-yl)-2-(4-fluorophenyl)benzimidazol-5-yl]-2-fluoro-N-methyl-benzamide;
4-[1-(1,3-dimethyl-2-oxo-benzimidazol-5-yl)-2-(4-methyl-1H-imidazol-5-yl)benzimidazol-5-yl]-2-fluoro-N-methyl-benzamide;
4-[2-(3,5-dimethylimidazol-4-yl)-1-(1,3-dimethyl-2-oxo-benzimidazol-5-yl)benzimidazol-5-yl]-2-fluoro-N-methyl-benzamide;
4-[2-(2,3-dimethylimidazol-4-yl)-1-(1,3-dimethyl-2-oxo-benzimidazol-5-yl)benzimidazol-5-yl]-2-fluoro-N-methyl-benzamide;
4-[2-tert-butyl-1-(1,3-dimethyl-2-oxo-benzimidazol-5-yl)benzimidazol-5-yl]-2-fluoro-N-methyl-benzamide;
4-[1-(1,3-dimethyl-2-oxo-benzimidazol-5-yl)-2-(trifluoromethyl)benzimidazol-5-yl]-2-fluoro-N-methyl-benzamide;
4-[1-(1,3-dimethyl-2-oxo-benzimidazol-5-yl)-2-methyl-benzimidazol-5-yl]-2-fluoro-N-methyl-benzamide;
4-[1-(1,3-dimethyl-2-oxo-benzimidazol-5-yl)-2-(2-methoxy-3-pyridyl)benzimidazol-5-yl]-2-fluoro-N-methyl-benzamide;
4-[2-(2-anilino-1,1-dimethyl-2-oxo-ethyl)-1-(1,3-dimethyl-2-oxo-benzimidazol-5-yl)benzimidazol-5-yl]-2-fluoro-N-methyl-benzamide;
4-[1-(1,3-dimethyl-2-oxo-benzimidazol-5-yl)-2-(2-methylpyrazol-3-yl)benzimidazol-5-yl]-2-fluoro-N-methyl-benzamide;
4-[2-(2-amino-3-pyridyl)-1-(1,3-dimethyl-2-oxo-benzimidazol-5-yl)benzimidazol-5-yl]-2-fluoro-N-methyl-benzamide;
tert-butyl N-[1-[1-(1,3-dimethyl-2-oxo-benzimidazol-5-yl)-5-[3-fluoro-4-(methylcarbamoyl)phenyl]benzimidazol-2-yl]-1-methyl-ethyl]carbamate;
4-[2-(3-cyano-2-methyl-phenyl)-1-(1,3-dimethyl-2-oxo-benzimidazol-5-yl)benzimidazol-5-yl]-2-fluoro-N-methyl-benzamide;
4-[2-(1-cyano-1-methyl-ethyl)-1-(1,3-dimethyl-2-oxo-benzimidazol-5-yl)benzimidazol-5-yl]-2-fluoro-N-methyl-benzamide;
4-[2-(1-amino-1-methyl-ethyl)-1-(1,3-dimethyl-2-oxo-benzimidazol-5-yl)benzimidazol-5-yl]-2-fluoro-N-methyl-benzamide;
4-[2-(2-chloro-3-pyridyl)-1-(1,3-dimethyl-2-oxo-benzimidazol-5-yl)benzimidazol-5-yl]-2-fluoro-N-methyl-benzamide;
4-[1-(1,3-dimethyl-2-oxo-benzimidazol-5-yl)-2-(1-methoxy-1-methylethyl)benzimidazol-5-yl]-2-fluoro-N-methyl-benzamide;
1',3'-dimethyl-2-(1-methyl-1H-imidazol-5-yl)-2'-oxo-2',3'-dihydro-1'H-[1,5'-bibenzo[d]imidazole]-5-carbonitrile;
N'-hydroxy-1',3'-dimethyl-2-(1-methyl-1H-imidazol-5-yl)-2'-oxo-2',3'-dihydro-1'H-[1,5'-bibenzo[d]imidazole]-5-carboximidamide;
3-(1',3'-dimethyl-2-(1-methyl-1H-imidazol-5-yl)-2'-oxo-2',3'-dihydro-1'H-[1,5'-bibenzo[d]imidazol]-5-yl)-1,2,4-oxadiazol-5(2H)-one;
5-(2,3-dihydrobenzo[b][1,4]dioxin-6-yl)-1',3'-dimethyl-2-(1-methyl-1H-imidazol-5-yl)-1',3'-dihydro-2'H-[1,5'-bibenzo[d]imidazol]-2'-one;
5-(2,3-dihydrobenzofuran-5-yl)-1',3'-dimethyl-2-(1-methyl-1H-imidazol-5-yl)-1',3'-dihydro-2'H-[1,5'-bibenzo[d]imidazol]-2'-one;
5-(1',3'-dimethyl-2-(1-methyl-1H-imidazol-5-yl)-2'-oxo-2',3'-dihydro-1'H-[1,5'-bibenzo[d]imidazol]-5-yl)furan-2-carbaldehyde;
5-(4,5-dihydrothiazol-2-yl)-1',3'-dimethyl-2-(1-methyl-1H-imidazol-5-yl)-1',3'-dihydro-2'H-[1,5'-bibenzo[d]imidazol]-2'-one;
ethyl 2-(4-(1',3'-dimethyl-2-(1-methyl-1H-imidazol-5-yl)-2'-oxo-2',3'-dihydro-1'H-[1,5'-bibenzo[d]imidazol]-5-yl)-1H-1,2,3-triazol-1-yl)acetate;
2-(4-(1',3'-dimethyl-2-(1-methyl-1H-imidazol-5-yl)-2'-oxo-2',3'-dihydro-1'H-[1,5'-bibenzo[d]imidazol]-5-yl)-1H-1,2,3-triazol-1-yl)acetic acid;
5-(1-(2-hydroxyethyl)-1H-1,2,3-triazol-4-yl)-1',3'-dimethyl-2-(1-methyl-1H-imidazol-5-yl)-1',3'-dihydro-2'H-[1,5'-bibenzo[d]imidazol]-2'-one;
2-(4-(1',3'-dimethyl-2-(1-methyl-1H-imidazol-5-yl)-2'-oxo-2',3'-dihydro-1'H-[1,5'-bibenzo[d]imidazol]-5-yl)-1H-1,2,3-triazol-1-yl)-N-methylacetamide;
2-(4-(1',3'-dimethyl-2-(1-methyl-1H-imidazol-5-yl)-2'-oxo-2',3'-dihydro-1'H-[1,5'-bibenzo[d]imidazol]-5-yl)-1H-1,2,3-triazol-1-yl)-N-(2,2,2-trifluoroethyl)acetamide;
1',3'-dimethyl-2-(1-methyl-1H-imidazol-5-yl)-5-(1-(2-oxo-2-(piperidin-1-yl)ethyl)-1H-1,2,3-triazol-4-yl)-1',3'-dihydro-2'H-[1,5'-bibenzo[d]imidazol]-2'-one;
1',3'-dimethyl-2-(1-methyl-1H-imidazol-5-yl)-5-(1-(2-morpholino-2-oxoethyl)-1H-1,2,3-triazol-4-yl)-1',3'-dihydro-2'H-[1,5'-bibenzo[d]imidazol]-2'-one;
N-benzyl-2-(4-(1',3'-dimethyl-2-(1-methyl-1H-imidazol-5-yl)-2'-oxo-2',3'-dihydro-1'H-[1,5'-bibenzo[d]imidazol]-5-yl)-1H-1,2,3-triazol-1-yl)acetamide;
5-(3,5-dimethylisoxazol-4-yl)-1',3'-dimethyl-2-(1-methyl-1H-imidazol-5-yl)-1',3'-dihydro-2'H-[1,5'-bibenzo[d]imidazol]-2'-one;
1',3'-dimethyl-2-(1-methyl-1H-imidazol-5-yl)-5-(5-methylfuran-2-yl)-1',3'-dihydro-2'H-[1,5'-bibenzo[d]imidazol]-2'-one;
5-(benzo[d][1,3]dioxol-5-yl)-1',3'-dimethyl-2-(1-methyl-1H-imidazol-5-yl)-1',3'-dihydro-2'H-[1,5'-bibenzo[d]imidazol]-2'-one;
5-(furan-3-yl)-1',3'-dimethyl-2-(1-methyl-1H-imidazol-5-yl)-1',3'-dihydro-2'H-[1,5'-bibenzo[d]imidazol]-2'-one; and
1',3'-dimethyl-2-(1-methyl-1H-imidazol-5-yl)-5-(1-methyl-1H-pyrazol-5-yl)-1',3'-dihydro-2'H-[1,5'-bibenzo[d]imidazol]-2'-one.

13. The compound according to claim 1, used as a CBP/p300 inhibitor.

14. An isomer of the compound according to claim 1, a pharmaceutically acceptable salt thereof, a hydrate thereof or a solvate thereof.

15. A pharmaceutical composition for preventing or treating cancer, inflammatory disorders or autoimmune diseases, the composition comprising at least one among the compound according to claim 1, an isomer thereof, a pharmaceutically acceptable salt thereof, a hydrate thereof and a solvate thereof, as an active ingredient.

16. The pharmaceutical composition according to claim 15, wherein the cancer is cancer dependent on oncogene MYC, tumor harboring a loss of function mutation of CBP or p300, or cancer with activation of CBP and/or p300 function.

17. The pharmaceutical composition according to claim 15, wherein the cancer is one or more selected from the group consisting of blood cancer, prostate cancer, breast cancer, lung cancer, pancreatic cancer, colorectal cancer, and melanoma..

18. The pharmaceutical composition according to claim 15, wherein the cancer is one or more selected from the group consisting of acoustic neuroma, acute leukemia, acute lymphoblastic leukemia, acute myeloid leukemia, acute T-cell leukemia, basal cell carcinoma, cholangiocarcinoma, bladder cancer, brain cancer, breast cancer, bronchial carcinoma, cervical cancer, chondrosarcoma, chordoma, choriocarcinoma, chronic leukemia, chronic lymphocytic leukemia, chronic myeloid leukemia, chronic myeloid leukemia, colon cancer, colon cancer, craniopharyngioma, cystadenocarcinoma, diffuse large B-cell lymphoma, dysproliferative changes, embryonal carcinoma, endometrial cancer, endotheliosarcoma, ependymoma, epithelial carcinoma, erythroleukemia, esophageal cancer, estrogen receptor-positive breast cancer, essential thrombocythemia, Ewing's tumor, fibrosarcoma, follicular lymphoma, germ cell testicular cancer, glioma, glioblastoma, pedunculated tumor, heavy chain disease head and neck cancer, hemangioblastoma, hepatoma, hepatocellular carcinoma, hormone-insensitive prostate cancer, leiomyosarcoma, leukemia, liposarcoma, lung cancer, lymphoendotheliosarcoma, lymphangiosarcoma, lymphocytic leukemia, lymphoma, lymphoid malignancy of T-cell or B-cell origin, medullary carcinoma, medulloblastoma, melanoma, meningioma, mesothelioma, multiple myeloma, myeloid leukemia, myeloma, myxosarcoma, neuroblastoma, NUT midline carcinoma (NMC), non-small cell lung cancer, oligodendroglioma, oral cancer, osteosarcoma, ovarian cancer, pancreatic cancer, papillary adenocarcinoma, papillary carcinoma, pinealoma, polycythemia vera, prostate cancer, rectal cancer, renal cell carcinoma, retinoblastoma, rhabdomyosarcoma, sarcoma, sebaceous carcinoma, seminoma, skin cancer, small cell lung carcinoma, solid tumors (carcinomas and sarcomas), small cell lung cancer, stomach cancer, squamous cell carcinoma, synovioma, sweat gland carcinoma, thyroid cancer, Waldenstrom macroglobulinemia, testicular tumor, uterine cancer and Wilms tumor.

19. The pharmaceutical composition according to claim 15, wherein the inflammatory disorder or autoimmune disease is one or more selected from the group consisting of Addison's disease, acute gout, ankylosing spondylitis, asthma, atherosclerosis, Behcet's disease, bullous skin disease, chronic obstructive pulmonary disease, Crohn's disease, dermatitis, eczema, giant cell arteritis, fibrosis, glomerulonephritis, hepatic vascular occlusion, hepatitis, hypophysitis, immunodeficiency syndrome, inflammatory bowel disease, Kawasaki disease, lupus nephritis, multiple sclerosis, myocarditis, myositis, nephritis, organ transplant rejection, osteoarthritis, pancreatitis, pericarditis, polyarteritis nodosa pneumonia, primary biliary cirrhosis, psoriasis, psoriatic arthritis, rheumatoid arthritis, scleritis, sclerosing cholangitis, sepsis, systemic lupus erythematosus, Takayasu's arteritis, toxic shock, thyroiditis, type 1 diabetes, ulcerative colitis, uveitis, vitiligo, vasculitis, and Wegener's granulomatosis.
